(19) Europäisches Patentamt
European Patent Office
Office européen des brevets

(11) EP 2 078 085 B1

(12) **EUROPÄISCHE PATENTSCHRIFT**

(45) Veröffentlichungstag und Bekanntmachung des Hinweises auf die Patenterteilung:
**20.08.2014 Patentblatt 2014/34**

(21) Anmeldenummer: **07821449.1**

(22) Anmeldetag: **17.10.2007**

(51) Int Cl.:
***C12N 15/60*** (2006.01) ***C12N 9/88*** (2006.01)
***C12P 13/08*** (2006.01) ***C12P 13/22*** (2006.01)
***A23K 1/16*** (2006.01)

(86) Internationale Anmeldenummer:
**PCT/EP2007/061084**

(87) Internationale Veröffentlichungsnummer:
**WO 2008/049769 (02.05.2008 Gazette 2008/18)**

(54) **ALLELE DES PRPD1-GENS AUS CORYNEFORMEN BAKTERIEN**

ALLELES OF THE PRPD1-GENE FROM CORYNEFORM BACTERIA

ALLÈLES DU GÈNE PRPD1 ISSU DE BACTÉRIES CORYNÉFORMES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.10.2006 DE 102006050489**

(43) Veröffentlichungstag der Anmeldung:
**15.07.2009 Patentblatt 2009/29**

(73) Patentinhaber: **Evonik Degussa GmbH**
**45128 Essen (DE)**

(72) Erfinder:
- **BATHE, Brigitte**
  **33154 Salzkotten (DE)**
- **KREUTZER, Caroline**
  **33813 Oerlinghausen (DE)**
- **THIERBACH, Georg**
  **33613 Bielefeld (DE)**

(56) Entgegenhaltungen:
**EP-A- 0 533 039** **EP-A- 0 615 693**
**EP-A- 1 029 919** **WO-A-02/086137**
**US-A- 4 778 808**

- **CLAES W A ET AL: "Identification of two prpDBC gene clusters in Corynebacterium glutamicum and their involvement in propionate degradation via the 2-methylcitrate cycle" JOURNAL OF BACTERIOLOGY, Bd. 184, Nr. 10, Mai 2002 (2002-05), Seiten 2728-2739, XP002449885 ISSN: 0021-9193 in der Anmeldung erwähnt**
- **DATABASE EMBL [Online] 3. Mai 2002 (2002-05-03), "Corynebacterium glutamicum prpDBC2 gene cluster, complete sequence." XP002466855 gefunden im EBI accession no. EMBL:AF434799 Database accession no. AF434799**
- **DATABASE UniProt [Online] 19. September 2002 (2002-09-19), "2-methylcitrate dehydratase 2 (EC 4.2.1.79)." XP002466856 gefunden im EBI accession no. UNIPROT:Q8NSL3 Database accession no. Q8NSL3**
- **DATABASE EMBL [Online] 3. Mai 2002 (2002-05-03), "Corynebacterium glutamicum prpDBC1 gene cluster, complete sequence." XP002466857 gefunden im EBI accession no. EMBL:AF434798 Database accession no. AF434798 in der Anmeldung erwähnt**
- **DATABASE UniProt [Online] 19. September 2002 (2002-09-19), "2-methylcitrate dehydratase 1 (EC 4.2.1.79)." XP002466858 gefunden im EBI accession no. UNIPROT:Q8NSH9 Database accession no. Q8NSH9**
- **WENDISCH V F ET AL: "Emerging Corynebacterium glutamicum systems biology." JOURNAL OF BIOTECHNOLOGY, Bd. 124, Nr. 1, 25. Juni 2006 (2006-06-25), Seiten 74-92, XP005480500 ISSN: 0168-1656**
- **KOFFAS M ET AL: "Strain improvement by metabolic engineering: lysine production as a case study for systems biology." CURRENT OPINION IN BIOTECHNOLOGY, Bd. 16, Nr. 3, Juni 2005 (2005-06), Seiten 361-366, XP002466854 ISSN: 0958-1669**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 078 085 B1

## Beschreibung

Gegenstand der Erfindung sind Mutanten und Allele des prpD1-Gens coryneformer Bakterien kodierend für Varianten der 2-Methylcitrat-Dehydratase (EC Nr. 4.2.1.79) und Verfahren zur Herstellung von L-Lysin unter Verwendung von Bakterien, die diese Allele enthalten.

Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserungvon L-Aminosäure produzierenden Stämmen von Corynebacterium eingesetzt, indem man einzelne Aminosäure-Biosynthesegene amplifiziert und die Auswirkung auf die Aminosäure-Produktion untersucht.

Das Chromosom von Corynebacterium glutamicum wurde vor einiger Zeit vollständig sequenziert (Kalinowski et al., Journal of Biotechnology 104, 5-25 (2003)). Das Chromosom von Corynebacterium efficiens wurde ebenfalls bereits sequenziert (Nishio et al., Genome Res. 13 (7), 1572-1579 (2003)).

Entsprechende Sequenzangaben können den öffentlichen Datenbanken entnommen werden. Geeignete Datenbanken sind beispielsweise die Datenbank der European Molecular Biology Laboratories (EMBL, Heidelberg, Deutschland bzw. Cambridge, UK), die Datenbank des National Center for Biotechnology Information (NCBI, Bethesda, MD, USA), die des Swiss Institute of Bioinformatics (Swissprot, Genève, Switzerland), die Protein Information Resource Database (PIR, Washington, DC, USA) und die DNA Data Bank of Japan (DDBJ, 1111 Yata, Mishima, 411-8540, Japan).

Zusammenfassende Darstellungen zur Genetik, zum Stoffwechsel und zur technischen Bedeutung von Corynebacterium findet man in den Aufsätzen von Ikeda, von Pfefferle et al. und von Mueller und Huebner im Buch "Microbial Production of L-Amino Acids" (Advances in Biochemical Engineering 79, (2003), Springer Verlag, Berlin, Deutschland, Herausgeber: T. Scheper), in der Spezialausgabe "A New Era in Corynebacterium glutamicum Biotechnology" des Journal of Biotechnology (Band 104 (1-3), 2003, Herausgeber: A. Pühler und T. Tauch) und im "Handbook of Corynebacterium glutamicum" (Herausgeber: L. Eggeling und M. Bott, CRC Press, Taylor & Francis Group, Boca Raton, FL, USA, 2005).

Die Nukleotidsequenz des für die 2-Methylcitrat-Dehydratase von Corynebacterium glutamicum kodierenden prpD1-Gens ist unter anderem in der Datenbank des National Center for Biotechnology Information (NCBI) der National Library of Medicin (Bethesda, MD, USA) unter der Zugangsnummer AF434798 allgemein verfügbar. Sie kann weiterhin der Patentanmeldung EP 1 108 790 als Sequenz Nr. 770 entnommen werden.

Claes et al. (Journal of Bacteriology 184(10), 2728-2739 (2002)) berichten über genetische, mikrobiologische und biochemische Untersuchungen an den Genen prpD1, prpB1 und prpC1 von Corynebacterium glutamicum ATCC 13032.

Der besseren Übersichtlichkeit halber ist die Nukleotidsequenz des für die 2-Methylcitrat-Dehydratase aus Corynebacterium glutamicum kodierenden prpD1-Gens ("Wildtyp-Gen") gemäß den Angaben der NCBI-Datenbank in SEQ ID NO:1 und die sich daraus ergebende Aminosäuresequenz der kodierten 2-Methylcitrat-Dehydratase in SEQ ID NO:2 und 4 dargestellt. In SEQ ID NO:3 sind stromaufwärts (upstream) und stromabwärts (downstream) gelegene Nukleotidsequenzen zusätzlich angegeben.

Aufgabe der Erfindung

Die Erfinder haben sich zur Aufgabe gestellt neue Maßnahmen zur verbesserten Herstellung von Aminosäuren, insbesondere L-Lysin, L-Tryptophan, L-Valin, L-Isoleucin und L-Homoserin bereitzustellen.

Beschreibung der Erfindung

Gegenstand der Erfindung sind erzeugte bzw. isolierte Mutanten coryneformer Bakterien, die bevorzugt Aminosäuren ausscheiden, und welche ein Gen bzw. Allel enthalten, das für ein Polypeptid mit 2-Methylcitrat-Dehydratase Aktivität kodiert, dadurch gekennzeichnet,
dass das Polypeptid an der Position 272 der Aminosäuresequenz von SEQ ID NO:2 entsprechenden Position L-Leucin enthält oder dass das Polypeptid die Aminosäuresequenz von SEQ ID NO: 2, umfasst, wobei an Position 272 L-Leucin enthalten ist.

Bei den coryneformen Bakterien wird die Gattung Corynebacterium bevorzugt. Bei der Gattung Corynebacterium werden folgenden Arten bevorzugt:

Corynebacterium efficiens (Typ-Stamm DSM44549),

Corynebacterium glutamicum (Typ-Stamm ATCC13032),

Corynebacterium thermoaminogenes (beispielsweise der Stamm FERM BP-1539), und

Corynebacterium ammoniagenes (Typ-Stamm ATCC6871),

wobei die Art Corynebacterium glutamicum ganz besonders bevorzugt wird.

Einige Vertreter der Art Corynebacterium glutamicum sind im Stand der Technik auch unter anderen Artbezeichnungen bekannt. Hierzu gehören beispielsweise:

Corynebacterium acetoacidophilum ATCC127270,

Corynebacterium lilium DSM20137,

Corynebacterium melassecola ATCC17965,

Brevibacterium flavum ATCC14067,

Brevibacterium lactofermentum ATCC127269,

Brevibacterium divaricatum ATCC14020, und

Microbacterium ammoniaphilum ATCC15354.

Der Begriff "Micrococcus glutamicus" für Corynebacterium glutamicum war ebenfalls gebräuchlich.

Die für die Massnahmen der Erfindung eingesetzten Stämme coryneformer Bakterien besitzen bevorzugt bereits die Fähigkeit die gewünschte Aminosäure in der Zelle anzureichern und/oder in das sie umgebende Nährmedium auszuscheiden und zu akkumulieren. Hierfür wird im Folgenden auch der Ausdruck "produzieren" verwendet. Insbesondere besitzen die eingesetzten Stämme coryneformer Bakterien die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l der gewünschten Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Bekannte Vertreter L-Lysin produzierender bzw. ausscheidender Stämme coryneformer Bakterien sind beispielsweise:

Corynebacterium glutamicum DM58-1/pDM6 (= DSM4697) beschrieben in EP 0 358 940,

Corynebacterium glutamicum MH20-22B (= DSM16835) beschrieben in Menkel et al. (Applied and Environmental Microbiology 55(3), 684-688 (1989)),

Corynebacterium glutamicum AHP-3 (= Ferm BP-7382) beschrieben in EP 1 108 790,

Corynebacterium glutamicum NRRL B-11474 beschrieben in US 4,275,157, und

Corynebacterium thermoaminogenes AJ12521 (= FERM BP-3304) beschrieben in US 5,250,423.

Bekannte Vertreter L- Tryptophan produzierender bzw. ausscheidender Stämme coryneformer Bakterien sind beispielsweise:

Corynebacterium glutamicum K76 (=Ferm BP-1847) beschrieben in US 5,563,052,

Corynebacterium glutamicum BPS13 (=Ferm BP-1777) beschrieben in US 5,605,818, und

Corynebacterium glutamicum Ferm BP-3055 beschrieben in US 5,235,940.

Bekannte Vertreter L-Valin produzierender bzw. ausscheidender Stämme coryneformer Bakterien sind beispielsweise:

Brevibacterium lactofermentum FERM BP-1763 beschrieben in US 5,188,948,

Brevibacterium lactofermentum FERM BP-3007 beschrieben in US 5,521,074,

Corynebacterium glutamicum FERM BP-3006 beschrieben in US 5,521,074, und

Corynebacterium glutamicum FERM BP-1764 beschrieben in US 5,188,948.

Bekannte Vertreter L-Isoleucin produzierender bzw. ausscheidender Stämme coryneformer Bakterien sind beispielsweise:

Brevibacterium flavum FERM BP-760 beschrieben in US 4,656,135,

Brevibacterium flavum FERM BP-2215 beschrieben in US 5,294,547, und

Corynebacterium glutamicum FERM BP-758 beschrieben in US 4,656,135.

Bekannte Vertreter L-Homoserin produzierender bzw. ausscheidender Stämme coryneformer Bakterien sind beispielsweise:

Micrococcus glutamicus ATCC 14296 beschrieben in US 3,189,526 und

Micrococcus glutamicus ATCC 14297 beschrieben in US 3,189,526.

Angaben zur taxonomischen Einordnung von Stämmen dieser Gruppe von Bakterien findet man unter anderem bei Seiler (Journal of General Microbiology 129, 1433-1477 (1983)), Kinoshita (1985, Glutamic Acid Bacteria, p 115-142. In: Demain and Solomon (ed), Biology of Industrial Microorganisms. The Benjamin/Cummins Publishing Co., London, UK), Kämpfer und Kroppenstedt (Canadian Journal of Microbiology 42, 989-1005 (1996)), Liebl et al (International Journal of Systematic Bacteriology 41, 255-260 (1991)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden.

Dem vom prpD1-Gen kodierte Polypeptid mit 2-Methylcitrat-Dehydratase Aktivität (PrpD1) wird entsprechend der Nomenklatur der IUPAC (International Union of Pure and Applied Chemistry) die EC-Nummer 4.2.1.79 zugeordnet.

Ein Gen ist chemisch ein Polynukleotid. Ein anderer Begriff hierfür ist Nukleinsäure.

Unter proteinogenen Aminosäuren versteht man die Aminosäuren, die in natürlichen Proteinen, das heißt in Proteinen von Mikroorganismen, Pflanzen, Tieren und Menschen vorkommen. Im Zusammenhang mit der vorliegenden Erfindung wird unter proteinogenen Aminosäuren die Gruppe der L-Aminosäuren, bestehend aus L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin und gegebenenfalls L-Selenocystein verstanden. Zu den L-Aminosäuren gehört ebenfalls das L-Homoserin.

Die erfindungsgemäßen Mutanten scheiden bevorzugt die genannten proteinogenen Aminosäuren, insbesondere L-Lysin, L-Tryptophan, L-Valin, L-Isoleucin oder L-Homoserin aus. Der Begriff Aminosäuren umfasst auch deren Salze wie beispielsweise das Lysin-Monohydrochlorid oder Lysin-Sulfat im Falle der Aminosäure L-Lysin.

Gegenstand der Erfindung sind weiterhin Mutanten coryneformer Bakterien, die ein prpD1-Allel enthalten, das für ein Polypeptid mit 2-Methylcitrat-Dehydratase Enzymaktivität kodiert, das die Aminosäuresequenz von SEQ ID NO: 2 umfasst, wobei an Position 272 L-Leucin enthalten ist.

Gegenstand der Erfindung sind weiterhin Mutanten coryneformer Bakterien, die ein prpD1-Allel enthalten, das für ein Polypeptid mit 2-Methylcitrat-Dehydratase Enzymaktivität kodiert, das an der Position 272 der Aminosäuresequenz von SEQ ID NO:2 entsprechenden Position L-Leucin enthält, wobei das Gen eine Nukleotidsequenz umfasst, die mit der Nukleotidsequenz eines Polynukleotids identisch ist, das durch eine Polymerasekettenreaktion (PCR) unter Verwendung eines Primerpaars erhältlich ist, deren Nukleotidsequenzen jeweils mindestens 15 aufeinanderfolgende Nukleotide umfassen, die aus der Nukleotidsequenz zwischen Position 1 und 750 von SEQ ID NO:3 oder SEQ ID NO:7 und aus der komplementären Nukleotidsequenz zwischen Position 2245 und 3000 von SEQ ID NO:3 oder SEQ ID NO:7 ausgewählt werden. Ein Beispiel für ein derartiges geeignetes Primerpaar ist in SEQ ID NO:9 und SEQ ID NO:10 dargestellt. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Gegenstand der Erfindung sind weiterhin Mutanten coryneformer Bakterien, die ein prpD1-Allel enthalten, das für ein Polypeptid mit 2-Methylcitrat-Dehydratase Enzymaktivität kodiert, das eine Aminosäuresequenz mit einer Länge entsprechend 498 L-Aminosäuren umfasst, wobei an Position 272 L-Leucin, enthalten ist.

Gegenstand der Erfindung sind weiterhin Mutanten coryneformer Bakterien, die ein prpD1-Allel enthalten, das für ein Polypeptid mit 2-Methylcitrat-Dehydratase Enzymaktivität kodiert, das an Position 252 bis 291 der Aminosäuresequenz die Aminosäuresequenz entsprechend Position 252 bis 291 von SEQ ID NO:6 bzw. 8 enthält. Bevorzugt enthält die Aminosäuresequenz des kodierten Polypeptids eine Aminosäuresequenz entsprechend Position 202 bis 341 von SEQ ID NO:6 bzw. 8 oder Position 152 bis 391 von SEQ ID NO:6 bzw. 8 oder Position 102 bis 441 von SEQ ID NO:6 bzw. 8 oder Position 52 bis 491 von SEQ ID NO:6 bzw. 8 oder Position 2 bis 495 von SEQ ID NO:6 bzw. 8 oder Position 2 bis 496 von SEQ ID NO:6 bzw. 8 oder Position 2 bis 497 von SEQ ID NO:6 bzw. 8. Ganz besonders bevorzugt umfasst die Länge des kodierten Polypeptids 498 Aminosäuren.

Gegenstand der Erfindung sind weiterhin Mutanten coryneformer Bakterien, die ein prpD1-Allel enthalten, das für ein Polypeptid mit 2-Methylcitrat-Dehydratase Enzymaktivität kodiert, welches an Position 272 bzw. an der entsprechenden Position der Aminosäuresequenz L-Leucin enthält und dessen Aminosäuresequenz außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Aminosäuresequenz von SEQ ID NO:6 bzw. SEQ ID NO:8.

Gegenstand der Erfindung sind weiterhin Mutanten coryneformer Bakterien, die ein prpD1-Allel enthalten, das für ein Polypeptid mit 2-Methylcitrat-Dehydratase Enzymaktivität kodiert, welches an Position 272 bzw. an der entsprechenden Position der Aminosäuresequenz L-Leucin enthält und dessen Nukleotidsequenz außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Nukleotidsequenz von SEQ ID NO:5.

Es ist bekannt, dass konservative Aminosäureaustausche die Enzymaktivität nur unwesentlich verändern. Dementsprechend kann das in den erfindungsgemäßen Mutanten enthaltene prpD1-Allel, das für ein Polypeptid mit 2-Methylcitrat-Dehydratase Enzymaktivität kodiert, zusätzlich zu der in SEQ ID NO:6 bzw. SEQ ID NO:8 gezeigten Aminosäuresequenz einen (1) oder mehrere konservative Aminosäureaustausch(e) enthalten. Bevorzugt enthält das Polypeptid höchstens zwei (2), höchstens drei (3), höchstens vier (4) oder höchstens fünf (5) konservative Aminosäureaustausche. Durch derartige konservative Aminosäureaustausche wird die enzymatische Aktivität im Wesentlichen nicht berührt.

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Bei der Arbeit an der vorliegenden Erfindung wurde durch Vergleich der Aminosäuresequenz mit dem Clustal-Programm (Thompson et al., Nucleic Acids Research 22, 4637-4680 (1994)) festgestellt, dass die Aminosäuresequenzen der 2-Methylcitrat-Dehydratase verschiedener Bakterien wie bespielsweise Mycobacterium tuberculosis, Mycobacterium bovis, Corynebacterium efficiens und Corynebacterium glutamicum ein Sequenzmotiv bestehend aus der Abfolge Arg-Glu-Leu-Asp-Phe-His-Asp-Thr-Phe-Leu-Ala-Ala-Asp/Glu-Tyr-Ser-His-Pro, ein Sequenzmotiv bestehend aus der Abfolge Gly-Ile-Cys-Leu-His-Glu-His-Lys-Ile-Asp-His-Val-Ala-His-Leu-Gly-Pro und auch ein Sequenzmotiv bestehend aus der Abfolge Pro-Ala-Pro-Ile-Trp-Glu-Gly-Glu-Asp-Gly-Val-Ile-Ala-Trp-Leu-Leu enthalten. Die Bezeichnung "Asp/Glu" bedeutet, dass an der entsprechenden Position "Asp oder Glu" vorliegen kann.

Dementsprechend sind solche Mutanten coryneformer Bakterien bevorzugt, die ein prpD1-Allel enthalten, das für ein Polypeptid mit 2-Methylcitrat-Dehydratase Enzymaktivität kodiert, welches mindestens eine Aminosäuresequenz ausgewählt aus der Gruppe Arg-Glu-Leu-Asp-Phe-His-Asp-Thr-Phe-Leu-Ala-Ala-Asp/Glu-Tyr-Ser-His-Pro, Gly-Ile-Cys-Leu-His-Glu-His-Lys-Ile-Asp-His-Val-Ala-His-Leu-Gly-Pro und Pro-Ala-Pro-Ile-Trp-Glu-Gly-Glu-Asp-Gly-Val-Ile-Ala-Trp-Leu-Leu umfasst und an Position 272 bzw. der entsprechenden oder vergleichbaren Position der Aminosäuresequenz eine der proteinogenen Aminosäuren ausgenommen L-Prolin, bevorzugt L-Leucin, enthält.

Das Aminosäuresequenzmotiv Arg-Glu-Leu-Asp-Phe-His-Asp-Thr-Phe-Leu-Ala-Ala-Asp/Glu-Tyr-Ser-His-Pro ist beispielsweise in der SEQ ID NO:2 bzw. 4 oder 6 bzw. 8 von Position 102 bis 118 enthalten. Das Aminosäuresequenzmotiv Gly-Ile-Cys-Leu-His-Glu-His-Lys-Ile-Asp-His-Val-Ala-His-Leu-Gly-Pro ist beispielsweise in der SEQ ID NO:2 bzw. 4 oder 6 bzw. 8 von Position 156 bis 172 enthalten. Das Aminosäuresequenzmotiv Pro-Ala-Pro-Ile-Trp-Glu-Gly-Glu-Asp-Gly-Val-Ile-Ala-Trp-Leu-Leu ist beispielsweise in der SEQ ID NO: 2 bzw. 4 oder 6 bzw. 8 von Position 240 bis 255 enthalten.

Gegenstand der Erfindung sind schließlich Mutanten coryneformer Bakterien, die ein prpD1-Allel enthalten, das für ein Polypeptid mit 2-Methylcitrat-Dehydratase Enzymaktivität kodiert, welches die Aminosäuresequenz von SEQ ID NO:6 bzw. SEQ ID NO:8 umfasst.

Gegenstand der Erfindung sind schließlich auch Mutanten coryneformer Bakterien, die ein prpD1-Allel entsprechend SEQ ID NO:5 oder SEQ ID NO:7 enthalten.

Es ist bekannt, dass durch wirtseigene Enzyme, sogenannte Aminopeptidasen, das endständige Methionin bei der Proteinsynthese entfernt wird.

Unter dem Begriff "einer zu Position 272 der Aminosäuresequenz entsprechenden Position" oder "einer zu Position 272 der Aminosäuresequenz vergleichbaren Position" versteht man die Tatsache, dass durch Insertion oder Deletion eines für eine Aminosäure kodierenden Kodons im N-terminalen Bereich (bezogen auf die Position 272 von SEQ ID NO:6 bzw. 8) des kodierten Polypeptids die Positionsangabe und Längenangabe im Falle einer Insertion formal um eine Einheit erhöht oder im Falle einer Deletion um eine Einheit verringert wird. Beispielsweise rückt durch Deletion des für die Aminosäure L-Glutaminsäure kodierenden GAG-Kodons an Position 19 von SEQ ID NO:6 bzw. 8 das L-Leucin von Position 272 an Position 271. Die Längenangabe wäre dann: 497 Aminosäuren. In gleicher Weise wird durch Insertion oder Deletion eines für eine Aminosäure kodierenden Kodons im C-terminalen Bereich (bezogen auf die Position 272) des kodierten Polypeptids die Längenangabe im Falle einer Insertion formal um eine Einheit erhöht oder im Falle einer Deletion um eine Einheit verringert. Derartige vergleichbare Positionen lassen sich durch Vergleich der Aminosäuresequenzen in Form eines "alignments" beispielsweise mit Hilfe des Clustal-Programmes oder des MAFFT-Programmesleicht identifizieren.

Durch derartige Insertionen und Deletionen wird die enzymatische Aktivität im Wesentlichen nicht berührt. "Im wesentlichen nicht berührt" bedeutet, dass sich die enzymatische Aktivität der genannten Varianten um maximal 10%, maximal 7,5%, maximal 5%, maximal 2,5% oder maximal 1% von der Aktivität des Polypeptids mit der Aminosäuresequenz von SEQ ID NO:6 bzw. 8 unterscheidet.

Eine Methode zur Bestimmung der enzymatischen Aktivität der 2-Methylcitrat-Dehydratase ist bei Horswill and Escalante-Semerena (Biochemistry 40(15), 4703-4713 (2001)) beschrieben.

Gegenstand der Erfindung sind dementsprechend auch prpD1-Allele, die für Polypeptid Varianten von SEQ ID NO:6 bzw. 8 kodieren, die eine oder mehrere Insertion(en) oder Deletion(en) enthalten. Bevorzugt enthält das Polypeptid maximal 5, maximal 4, maximal 3 oder maximal 2 Insertionen oder Deletionen von Aminosäuren.

Die Sequenzmotive Arg-Glu-Leu-Asp-Phe-His-Asp-Thr-Phe-Leu-Ala-Ala-Asp/Glu-Tyr-Ser-His-Pro, Gly-Ile-Cys-Leu-His-Glu-His-Lys-Ile-Asp-His-Val-Ala-His-Leu-Gly-Pro und Pro-Ala-Pro-Ile-Trp-Glu-Gly-Glu-Asp-Gly-Val-Ile-Ala-Trp-Leu-Leu werden durch derartige Insertionen/Deletionen vorzugsweise nicht zerrissen.

Zur Herstellung der erfindungsgemäßen Mutanten können klassische in-vivo Mutageneseverfahren mit Zellpopulationen coryneformer Bakterien unter Verwendung mutagener Stoffe wie beispielsweise N-Methyl-N'-Nitro-N-Nitrosoguanidin (MNNG), Ethylmethansulfonat (EMS), 5-Bromuracil oder ultraviolettes Licht verwendet werden. Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al (Folia Microbiologica 33, 337-343 (1988)) beschrieben. Typische Mutagenesen unter Verwendung von MNNG umfassen Konzentrationen von 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90% oder ca. 50% bis 99% oder ca. 50% bis 99,9% oder mehr reduziert.

Aus der mutagenisierten Zellpopulation werden Mutanten bzw. Zellen entnommen und vermehrt. Vorzugsweise wird in einem weiteren Schritt deren Fähigkeit untersucht, in einer Satzkultur (batch) unter Verwendung eines geeigneten Nährmediums Aminosäuren, bevorzugt L-Lysin, L-Tryptophan, L-Valin, L-Isoleucin oder L-Homoserin, auszuscheiden. Geeignete Nährmedien und Testbedingungen sind unter anderem in der US 6,221,636, in der US 5,840,551, in der US 5,770,409, in der US 5,605,818, in der US 5,275,940 und in der US 4,224,409 beschrieben. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428 (2005)) beschrieben, können zahlreiche Mutanten in kurzer Zeit untersucht werden. Im Allgemeinen werden maximal 3.000, maximal 10.000, maximal 30.000 oder auch maximal 60.000 Mutanten gegebenenfalls auch mehr untersucht. Auf diese Weise werden Mutanten identifiziert, die im Vergleich zum Elternstamm bzw. nicht mutagenisierten Ausgangsstamm vermehrt Aminosäuren in das Nährmedium oder in das Zellinnere ausscheiden. Dazu gehören beispielsweise solche Mutanten, deren Aminosäuresekretion um mindestens 0,5% erhöht ist.

Anschließend wird aus den Mutanten DNA bereitgestellt, bzw. isoliert und mit Hilfe der Polymerase-Kettenreaktion unter Verwendung von Primerpaaren, die die Amplifizierung des prpD1-Gens bzw. des erfindungsgemäßen prpD1-Allels oder der erfindungsgemäßen Mutation an Position 272 erlauben, das entsprechende Polynukleotid synthetisiert. Vorzugsweise wird die DNA aus solchen Mutanten isoliert, die in erhöhter Weise Aminosäuren ausscheiden.

Hierzu können beliebige Primerpaare aus der stromaufwärts und stromabwärts der erfindungsgemäßen Mutation gelegenen Nukleotidsequenz und der dazu komplementären Nukleotidsequenz ausgewählt werden. Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 1563 von SEQ ID NO:3 oder SEQ ID NO:7. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz zwischen Position 3000 und 1567 von SEQ ID NO:3 oder SEQ ID NO:7. Ist die Amplifikation der Kodierregion gewünscht, so wird das Primerpaar vorzugsweise aus der Nukleotidsequenz zwischen Position 1 und 750 von SEQ ID NO:3 oder SEQ ID NO:7 und aus der komplementären Nukleotidsequenz zwischen Position 3000 und 2245 von SEQ ID NO:3 oder SEQ ID NO:7 ausgewählt. Ein geeignetes Primerpaar ist beispielsweise das unter SEQ ID NO:9 und SEQ ID NO:10 wiedergegebene Primerpaar prpD1_XL_A1 und prpD1_XL_E1. Ein unter Verwendung dieses Primerpaars hergestelltes PCR-Produkt ist in SEQ ID NO:13 dargestellt.

Ist die Amplifikation eines Teils der Kodierregion, wie beispielsweise in SEQ ID NO:17 und 19 dargestellt, erwünscht, so wird das Primerpaar vorzugsweise aus der Nukleotidsequenz zwischen Position 751 und 1563 von SEQ ID NO:3 oder SEQ ID NO:7 und aus der komplementären Nukleotidsequenz zwischen ' Position 2244 und 1567 von SEQ ID NO:3 oder SEQ ID NO:7 ausgewählt.

Der Primer kann überdies mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Für die Herstellung von Polynukleotiden durch Amplifikation ausgewählter Sequenzen, wie dem erfindungsgemäßen prpD1-Allel, aus vorgelegter, beispielsweise chromosomaler DNA ("template DNA") durch Amplifikation mittels PCR, werden im Allgemeinen thermostabile DNA-Polymerasen eingesetzt. Beispiele derartiger DNA-Polymerasen sind die Taq-Polymerase aus Thermus aquaticus, die unter anderem von der Firma Qiagen (Hilden, Deutschland) vertrieben wird, die Vent-Polymerase aus Thermococcus litoralis, die unter anderem von der Firma New England Biolabs (Frankfurt, Deutschland) vertrieben wird oder die Pfu-Polymerase aus Pyrococcus furiosus, die unter anderem von der Firma Stratagene (La Jolla, USA) vertrieben wird. Bevorzugt werden Polymerasen mit "proof-reading"-Aktivität. "proof-reading"-Aktivität bedeutet, dass diese Polymerasen in der Lage sind, fehlerhaft eingebaute Nukleotide zu erkennen und den Fehler durch erneute Polymerisation zu beheben (Lottspeich und Zorbas, Bioanalytik, Spektrum Akademischer Verlag, Heidelberg, Deutschland (1998)). Beispiele für Polymerasen mit "proof-reading"-Aktivität sind die Vent-Polymerase und die Pfu-Polymerase.

Die Bedingungen im Reaktionsansatz werden nach Angaben des Herstellers eingestellt. Die Polymerasen werden vom Hersteller im Allgemeinen zusammen mit dem gebräuchlichen Puffer geliefert, welcher üblicherweise Konzentrationen von 10 - 100 mM Tris/HCl und 6 - 55 mM KCl bei pH 7,5 - 9,3 aufweist. Magnesiumchlorid wird in einer Konzentration von 0,5 - 10 mM zugesetzt, falls es nicht in dem vom Hersteller gelieferten Puffer enthalten ist. Dem Reaktionsansatz werden weiterhin Desoxynucleosidtriphosphate in einer Konzentration von 0,1-16,6 mM zugesetzt. Die Primer werden im Reaktionsansatz mit einer Endkonzentration von 0,1 - 3 $\mu$M vorgelegt und die Template-DNA im optimalen Fall mit $10^2$ bis $10^5$ Kopien. Es können auch $10^6$ bis $10^7$ Kopien eingesetzt werden. Die entsprechende Polymerase wird dem Reaktionsansatz in einer Menge von 2-5 Units zugegeben. Ein typischer Reaktionsansatz hat ein Volumen von 20 - 100 $\mu$l.

Als weitere Zusätze können der Reaktion Rinderserumalbumin, Tween-20, Gelatin, Glycerin, Formamid oder DMSO beigesetzt werden (Dieffenbach und Dveksler, PCR Primer - A Laboratory Manual, Cold Spring Harbor Laboratory Press, USA 1995).

Ein typischer PCR-Verlauf besteht aus drei unterschiedlichen, sich aufeinanderfolgend wiederholenden Temperaturstufen. Vorab wird die Reaktion mit einer Temperaturerhöhung auf 92°C - 98°C für 4 bis 10 Minuten gestartet, um die vorgelegte DNA zu denaturieren. Dann folgen sich wiederholend zuerst ein Schritt zur Denaturierung der vorgelegten DNA von 10 - 60 Sekunden bei ungefähr 92-98°C, dann ein Schritt zum Binden der Primer an die vorgelegte DNA von 10 - 60 Sekunden bei einer bestimmten, von den Primern abhängigen Temperatur ("Annealing-Temperatur"), die erfahrungsgemäß bei 50°C bis 60°C liegt und sich für jedes Primerpaar einzeln berechnen läßt. Genaue Informationen dazu findet der Fachmann bei Rychlik et al. (Nucleic Acids Research 18 (21): 6409-6412). Anschließend folgt ein Synthese-Schritt zur Verlängerung der vorgelegten Primer ("Extension") bei dem jeweils für die Polymerase angegebenen Aktivitätsoptimum, üblicherweise je nach Polymerase im Bereich von 73°C bis 67°C bevorzugt 72°C bis 68°C. Die Dauer dieses Extensionschrittes hängt von der Leistungsfähigkeit der Polymerase und Länge des zu amplifizierenden PCR-Produktes ab. In einer typischen PCR dauert dieser Schritt 0,5 - 8 Minuten, bevorzugt 2 - 4 Minuten. Diese drei Schritte werden 30 bis 35 mal, gegebenenfalls bis zu 50 mal wiederholt. Ein abschließender "Extension"-Schritt von 4 - 10 Minuten beendet die Reaktion. Die auf diese Weise hergestellten Polynukleotide werden auch als Amplifikate bezeichnet; der Begriff Nukleinsäurefragment ist ebenfalls gebräuchlich.

Ein unter Verwendung des Primerpaars prpD1_XL_A1 / prpD1_XL_E1 (siehe SEQ ID NO:9 und SEQ ID NO:10) hergestelltes PCR-Produkt ist in SEQ ID NO:13 dargestellt.

Weitere Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994).

Die Nukleotidsequenz wird anschließend beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067 (1990)) angegebenen Modifikationen bestimmt und das von dieser Nukleotidsequenz kodierte Polypeptid insbesondere bezüglich der Aminosäuresequenz analysiert. Dazu wird die Nukleotidsequenz in ein Programm zur Übersetzung von DNA-Sequenz in eine Aminosäuresequenz eingegeben: Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Auf diese Weise werden Mutanten identifiziert, deren prpD1-Allele für Polypeptide mit 2-Methylcitrat-Dehydratase Enzymaktivität kodieren, welche an Position 272 der Aminosäuresequenz, bzw. der entsprechenden oder vergleichbaren Position, eine der proteinogenen Aminosäuren ausgenommen L-Prolin enthalten. Bevorzugt wird der Austausch gegen L-Leucin.

Gegebenenfalls wird das gesamte Chromosom der Mutante bestimmt. Hierbei kann die von Margulies et al. (Nature, 437(7057): 376-2720 (2005)) und Velicer et al. (Proceedings of the National Academy of Sciences, U.S.A., 103(21), 8107-8112 (2006)) beschriebene Methode, die unter dem Stichwort "pyrosequencing" in der Fachwelt bekannt ist und eine rasche Sequenzierung kompletter Genome ermöglicht, eingesetzt werden.

Gegenstand der Erfindung ist dementsprechend eine Mutante eines coryneformen Bakteriums, dadurch gekennzeichnet, dass diese durch folgende Schritte erhältlich ist:

a) Behandlung eines coryneformen Bakteriums, das die Fähigkeit besitzt Aminosäuren auszuscheiden, mit einem mutagenen Agenz,

b) Isolierung und Vermehrung der in a) erzeugten Mutante,

c) vorzugsweise Bestimmung der Fähigkeit der Mutante in einem Medium mindestens 0,5% mehr Aminosäure auszuscheiden oder im Zellinneren anzureichern als das in

a) eingesetzte coryneforme Bakterium,

d) Bereitstellung von Nukleinsäure aus der in b) erhaltenen Mutante,

e) Herstellung eines Nukleinsäuremoleküls (bzw. Amplifikates oder Nukleinsäurefragments) unter Verwendung der Polymerasekettenreaktion, der Nukleinsäure aus d), und eines Primerpaares bestehend aus einem ersten Primer umfassend mindestens 15 aufeinanderfolgenden Nukleotide ausgewählt aus der Nukleotidsequenz zwischen Position 1 und 1563, bevorzugt 1 bis 750 von SEQ ID NO:3 oder SEQ ID NO:7 und einem zweitem Primer umfassend mindestens 15 aufeinanderfolgende Nukleotide ausgewählt aus der komplementären Nukleotidsequenz zwischen Position 3000 und 1567, bevorzugt 3000 und 2245 von SEQ ID NO:3 oder 7,

f) Bestimmung der Nukleotidsequenz des in e) erhaltenen Nukleinsäuremoleküls, und Bestimmung der kodierten Aminosäuresequenz,

g) gegebenfalls Vergleich der in f) bestimmten Aminosäuresesequenz mit SEQ ID NO:6 bzw. 8, und

h) Identifizierung einer Mutante, die ein Polynukleotid enthält, das für ein Polypeptid kodiert, welches an Position 272 oder an vergleichbarer Position L-Leucin, enthält.

Die auf diese Weise erzeugten Mutanten enthalten typischerweise eine (1) Kopie des beschriebenen prpD1-Allels.

In der SEQ ID NO:5 ist beispielhaft die Kodierregion des prpD1-Allels einer erfindungsgemäßen Mutante wiedergegeben. Die Kodierregion des Wildtypgens ist als SEQ ID NO:1 wiedergegeben. SEQ ID NO:1 enthält an Position 814 die Nukleobase Cytosin, an Position 815 die Nukleobase Cytosin und an Position 816 die Nukleobase Cytosin. SEQ ID NO:1 enthält somit von Position 814 bis 816 das für die Aminosäure L-Prolin kodierende CCC Kodon. SEQ ID NO:5 enthält an Position 815 die Nukleobase Thymin. Durch diese Cytosin Thymin Transition entsteht an Position 814 bis 816 das für die Aminosäure L-Leucin kodierende CTC Kodon.

Darüber hinaus können die in SEQ ID NO: 5 bzw. 7 dargestellten Nukleotidsequenzen weitere Basenaustausche enthalten, die sich durch die Mutagenesebehandlung ergeben haben, sich jedoch nicht in einer veränderten Aminosäuresequenz äußern. Derartige Mutationen werden in der Fachwelt auch als stille oder neutrale Mutationen bezeichnet. Diese stillen Mutationen können ebenfalls in dem für die Mutagenesebehandlung eingesetzten coryneformen Bakterium bereits enthalten sein.

Die für die Mutagenese verwendeten coryneformen Bakterien besitzen bevorzugt bereits die Fähigkeit, die gewünschte Aminosäure in das sie umgebende Nährmedium bzw. Fermentationsbrühe auszuscheiden und/oder im Zellinneren anzureichern.

L-Lysin produzierende coryneforme Bakterien besitzen typischerweise eine "feed back" resistente bzw. desensibilisierte Aspartatkinase. Unter "feed back" resistenten Aspartatkinasen versteht man Aspartatkinasen (LysC), die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene bzw. Allele werden auch als lysC$^{FBR}$-Allele bezeichnet. Im Stand der Technik sind zahlreiche lysC$^{FBR}$-Allele beschrieben, die für Aspartatkinase-Varianten kodieren, welche Aminosäureaustausche im Vergleich zum Wildtypprotein besitzen. Die Kodierregion des Wildtyp lysC-Gens von Corynebacterium glutamicum entsprechend der Zugangsnummer AX756575 der NCBI Datenbank ist in SEQ ID NO:11 und das von diesem Gen kodierte Polypeptid in SEQ ID NO:12 dargestellt.

Die für die Massnahmen der Erfindung eingesetzten L-Lysin produzierenden coryneformen Bakterien verfügen bevorzugt über ein lysC-Allel, das für eine Aspartatkinasevariante kodiert, welche die Aminosäuresequenz von SEQ ID NO:12 besitzt, wobei diese eine oder mehrere der Aminosäureaustausche ausgewählt aus der Gruppe:

LysC A279T (Austausch von L-Alanin an Position 279 des kodierten Aspartatkinaseproteins gemäß SEQ ID NO: 12 gegen L-Threonin; siehe US 5,688,671 und Zugangsnummern E06825, E06826, E08178 und I74588 bis I74597),

LysC A279V (Austausch von L-Alanin an Position 279 des kodierten Aspartatkinaseproteins gemäß SEQ ID NO: 12 gegen L-Valin, siehe JP 6-261766 und Zugangsnummer E08179),

LysC L297Q (Austausch von L-Leucin an Position 297 des kodierten Aspartatkinaseproteins gemäß SEQ ID NO: 12 gegen L-Glutamin; siehe DE 102006026328,

LysC S301F (Austausch von L-Serin an Position 301 des kodierten Aspartatkinaseproteins gemäß SEQ ID NO: 12 gegen L-Phenylalanin; siehe US 6,844,176 und Zugangsnummer E08180),

LysC S301Y (Austausch von L-Serin an Position 301 des kodierten Aspartatkinaseproteins gemäß SEQ ID NO: 12 gegen L-Tyrosin, siehe Kalinowski et al. (Molecular and General Genetics 224, 317-324 (1990)) und Zugangsnummer X57226),

LysC T308I (Austausch von L-Threonin an Position 308 des kodierten Aspartatkinaseproteins gemäß SEQ ID NO: 12 gegen L-Isoleucin; siehe JP 6-261766 und Zugangsnummer E08181)

LysC T311I (Austausch von L-Threonin an Position 311 des kodierten Aspartatkinaseproteins gemäß SEQ ID NO: 12 gegen L-Isoleucin; siehe WO 00/632728 und US 6,893,848),

LysC S317A (Austausch von L-Serin an Position 317 des kodierten Aspartatkinaseproteins gemäß SEQ ID NO: 12 gegen L-Alanin; siehe US 5,688,671 und Zugangsnummer I74589),

LysC R320G (Austausch von L-Arginin an Position 320 des kodierten Aspartatkinaseproteins gemäß SEQ ID NO: 12 gegen Glycin; siehe Jetten et al. (Applied Microbiology and Biotechnology 43, 76-82 (995)) und Zugangsnummer L27125),

LysC G345D (Austausch von Glycin an Position 345 des kodierten Aspartatkinaseproteins gemäß SEQ ID NO: 12 gegen L-Asparaginsäure; siehe Jetten et al. (Applied Microbiology and Biotechnology 43, 76-82 (995)) und Zugangsnummer L16848),

LysC T380I (Austausch von L-Threonin an Position 380 des kodierten Aspartatkinaseproteins gemäß SEQ ID NO: 12 gegen L-Isoleucin; siehe WO 01/49854 und Zugangsnummer AX192358), und

LysC S381F (Austausch von L-Serin an Position 381 des kodierten Aspartatkinaseproteins gemäß SEQ ID NO: 12 gegen L-Phenylalanin; siehe EP 0435132)

umfasst.

Besonders bevorzugt werden das lysC$^{FBR}$-Allel lysC T311I (Austausch von Threonin an Position 311 des kodierten Aspartatkinaseproteins gemäß SEQ ID NO: 12 gegen Isoleucin) und ein lysC$^{FBR}$-Allel enthaltend mindestens einen Austausch ausgewählt aus der Gruppe A279T (Austausch von Alanin an Position 279 des kodierten Aspartatkinaseproteins gemäß SEQ ID NO: 12 gegen Threonin), S381F (Austausch von Serin an Position 381 des kodierten Aspartatkinaseproteins gemäß SEQ ID NO: 12 gegen Phenylalanin) und S317A (Austausch von Serin an Position 317 des kodierten Aspartatkinaseproteins gemäß SEQ ID NO: 12 gegen Alanin).

Ganz besonders bevorzugt wird das lysC$^{FBR}$-Allel lysC T311I (Austausch von Threonin an Position 311 des kodierten Aspartatkinaseproteins gemäß SEQ ID NO: 12 gegen Isoleucin).

Der Stamm DSM 16833 (WO 06/063660) besitzt ebenso wie der Stamm ATCC 21543 (US 3,708,395) ein lysC$^{FBR}$-Allel, das für ein Aspartatkinaseprotein kodiert, welches den Aminosäureaustausch T311I enthält.

Der Stamm NRRL B-11474 (US 4,275,157)besitzt ein lysC$^{FBR}$-Allel, das für ein Aspartatkinaseprotein kodiert, welches die Aminosäureaustausche A279T und S381F enthält.

Nach der oben beschriebenen Art und Weise wurde, ausgehend von Stamm ATCC 21543, eine als DM1916 bezeichnete Mutante isoliert, die ein prpD1-Allel enthält, das für ein Polypeptid kodiert, bei dem an Position 272 der Aminosäuresequenz L-Leucin enthalten ist. Die Nukleotidsequenz der Kodierregion des prpD1-Allels der Mutante DM1916 ist als SEQ ID NO:5 und die Aminosäuresequenz des kodierten Polypeptids als SEQ ID NO:6 bzw. 8 dargestellt.

Darüberhinaus können L-Lysin ausscheidende coryneforme Bakterien verwendet werden, die Eigenschaften besitzen, wie sie aus dem Stand der Technik bekannt sind.

L-Tryptophan produzierende coryneforme Bakterien besitzen typischerweise eine "feed back" resistente bzw. desensibilisierte Anthranilatsynthase. Unter "feed back" resistenter Anthranilatsynthase (TrpE) versteht man Anthranilatsynthasen, die im Vergleich zur Wildform eine um mindestens 5 bis 10%, oder mindestens 10% bis 15% oder mindestens 10% bis 20% geringere Empfindlichkeit gegenüber der Hemmung durch Tryptophan oder 5-Fluorotryptophan (Matsui et al., Journal of Bacteriology 169 (11): 5330 - 5332(1987)) oder ähnliche Analoga aufweisen. Die für diese desensibilisierten Anthranilatsynthasen kodierenden Gene bzw. Allele werden auch als trpE$^{FBR}$ -Allele bezeichnet. Beispiele für derartige Mutanten bzw. Allele sind beispielsweise in der US 6180373 und EP0338474 beschrieben.

L-Valin produzierende coryneforme Bakterien besitzen typischerweise eine "feed back" resistente bzw. desensibilisierte Acetolactat Synthase (Acetohydroxyacid Synthase) [EC Nr. 2.2.1.6].

Unter "feed back" resistenter Acetolactat Synthase versteht man eine Acetolactat Synthase die im Vergleich zur Wildform eine geringere Empfindlichkeit gegenüber der Hemmung durch eine oder mehrere der Aminosäuren ausgewählt aus der Gruppe L-Valin, L-Isoleucin und L-Leucin, bevorzugt L-Valin aufweist.

Die Acetolactat Synthase (IlvB, IlvN) von Corynebacterium besteht aus einer sogenannten grossen vom ilvB-Gen kodierten Untereinheit und einer sogenannten kleinen vom ilvN-Gen kodierten Untereinheit (Keilhauer et al., Journal of Bacteriology 175(17), 5595-5603 (1993)). In der WO 05/003357 und bei Elisakova et al. (Applied and Environmental Microbiology 71(1):207-13 (2005)) wird von Varianten der IlvN-Untereinheit berichtet, die der Acetolactat Synthase Resistenz gegenüber L-Valin, L-Isoleucin und L-Leucin verleihen. Eine Variante enthält an Position 21 der Aminosäuresequenz L-Asparaginsäure anstelle von L-Isoleucin (IlvN I21D) und an Position 22 L-Phenylalanin anstelle von L-Isoleucin (IlvN I22F). Die zweite Variante enthält an Position 20 der Aminosäuresequenz L-Asparaginsäure anstelle Glycin (IlvN G20D), an Position 21 der Aminosäuresequenz L-Asparaginsäure anstelle von L-Isoleucin (IlvN I21D) und an Position 22 L-Phenylalanin anstelle von L-Isoleucin (IlvN I22F).

L-Isoleucin produzierende coryneforme Bakterien besitzen typischerweise eine "feed back" resistente bzw.

desensibilisierte Threonin-Dehydratase (= Threonin-Deaminase).

Unter "feed back" resistenter Threonin-Dehydratase versteht man eine Threonin-Dehydratase (EC Nr. 4.3.1.19), die im Vergleich zur Wildform eine geringere Empfindlichkeit gegenüber der Hemmung durch L-Isoleucin aufweist. Die für diese desensibilisierten Threonindehydratase kodierenden Gene bzw. Allele werden auch als $ilvA^{FBR}$ -Allele bezeichnet.

Die Kodierregion des Wildtyp ilvA-Gens von Corynebacterium glutamicum entsprechend der Zugangsnummer L01508 der NCBI Datenbank ist in SEQ ID NO:17 und das von diesem Gen kodierte Polypeptid in SEQ ID NO:18 dargestellt.

Die in der US 6,107,063 und bei Morbach et al. (Applied and Environmental Microbiology 61 (12), 4315-4320 (1995)) beschriebenen Threonindehydratasevarianten enthalten einen oder mehreren der Aminosäureaustausche ausgewählt aus der Gruppe:

IlvA M199V (Austausch von L-Methionin an Position 199 des kodierten Threonindehydrataseproteins gemäß SEQ ID NO: 18 gegen L-Valin; siehe US 6,107,063),

IlvA A257G (Austausch von L-Alanin an Position 257 des kodierten Threonindehydrataseproteins gemäß SEQ ID NO: 18 gegen L-Arginin; siehe US 6,107,063),

IlvA H278R (Austausch von L-Histidin an Position 278 des kodierten Threonindehydrataseproteins gemäß SEQ ID NO: 18 gegen L-Arginin; siehe US 6,107,063),

IlvA V323A (Austausch von L-Valin an Position 323 des kodierten Threonindehydrataseproteins gemäß SEQ ID NO: 18 gegen L-Alanin; siehe Morbach et al.),

IlvA L351S (Austausch von L-Leucin an Position 351 des kodierten Threonindehydrataseproteins gemäß SEQ ID NO: 18 gegen L-Serin; siehe US 6,107,063)

IlvA D378G(Austausch von L-Asparaginsäure an Position 378 des kodierten Threonindehydrataseproteins gemäß SEQ ID NO: 18 gegen Glycin; siehe Morbach et al.)

Die erhaltenen Mutanten zeigen eine im Vergleich zum eingesetzten Ausgangsstamm bzw. Elternstamm erhöhte Ausscheidung bzw. Produktion der gewünschten Aminosäure in einem Fermentationsprozess.

Gegenstand der Erfindung ist ebenfalls ein isoliertes Polynukleotid, das für ein Polypeptid mit 2-Methylcitrat-Dehydratase Enzymaktivität kodiert, welches an der Position 272 der Aminosäuresequenz von SEQ ID NO:2 entsprechenden Position L-Leucin enthält
oder welches die Aminosäuresequenz von SEQ ID NO: 2 umfasst, wobei an Position 272 L-Leucin enthalten ist.

Das erfindungsgemäße Polynukleotid kann aus einer erfindungsgemäßen Mutante isoliert werden.

Weiterhin können für die Mutagenese des prpD1-Gens in-vitro Methoden eingesetzt werden. Bei der Verwendung von in-vitro Methoden werden isolierte Polynukleotide, welche ein prpD1-Gen eines coryneformen Bakteriums, vorzugsweise das im Stand der Technik beschriebene Wildtyp-Gen von Corynebacterium glutamicum, enthalten, einer mutagenen Behandlung unterzogen.

Bei den isolierten Polynukleotiden kann es sich beispielsweise um isolierte Gesamt-DNA bzw. chromosomale DNA oder auch um Amplifikate des prpD1-Gens handeln, die mit Hilfe der Polymerasekettenreaktion (PCR) hergestellt wurden. Derartige Amplifikate werden auch als PCR-Produkte bezeichnet. Anleitungen zur Amplifikation von DNA-Sequenzen mit Hilfe der Polymerase-Kettenreaktion findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Es ist ebenfalls möglich, das zu mutagenisierende prpD1-Gen zunächst in einen Vektor, beispielsweise in einen Bakteriophagen oder in ein Plasmid einzubauen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Gegenstand der Erfindung ist weiterhin ein isoliertes Polynukleotid, dass für ein Polypeptid mit 2-Methylcitrat-Dehydratase Enzymaktivität kodiert, welches die Aminosäuresequenz von SEQ ID NO:2 umfasst, wobei an Position 272 der Aminosäuresequenz L-Leucin enthalten ist.

Gegenstand der Erfindung ist weiterhin ein isoliertes Polynukleotid, dass für ein Polypeptid mit 2-Methylcitrat-Dehydratase Enzymaktivität kodiert, welches eine Aminosäuresequenz mit einer Länge von 498 Aminoäuren umfasst und wobei das Polypeptid an der Position 272 der Aminosäuresequenz von SEQ ID NO:2 entsprechenden Position L-Leucin enthält

oder wobei das Polypeptid die Aminosäuresequenz von SEQ ID NO: 2 umfasst, bei der an Position 272 L-Leucin enthalten ist.

Gegenstand der Erfindung ist weiterhin ein isoliertes Polynukleotid, das für ein Polypeptid mit 2-Methylcitrat-Dehydratase Enzymaktivität kodiert, das an Position 252 bis 291 der Aminosäuresequenz die Aminosäuresequenz entsprechend Position 252 bis 291 von SEQ ID NO:6 bzw. 8 enthält. Bevorzugt enthält die Aminosäuresequenz des kodierten Polypeptids eine Aminosäuresequenz entsprechend Position 202 bis 341 von SEQ ID NO:6 bzw. 8 oder Position 152 bis 391 von SEQ ID NO:6 bzw. 8 oder Position 102 bis 441 von SEQ ID NO:6 bzw. 8 oder Position 52 bis 491 von SEQ ID NO:6 bzw. 8 oder Position 2 bis 495 von SEQ ID NO:6 bzw. 8 oder Position 2 bis 496 von SEQ ID NO:6 bzw. 8 oder Position 2 bis 497 von SEQ ID NO:6 bzw. 8. Ganz besonders bevorzugt umfasst die Länge des kodierten Polypeptids 498 Aminosäuren.

Gegenstand der Erfindung ist weiterhin ein isoliertes Polynukleotid, das für ein Polypeptid mit 2-Methylcitrat-Dehydratase Enzymaktivität kodiert, welches an der Position 272 der Aminosäuresequenz von SEQ ID NO:2 entsprechenden Position L-Leucin enthält

oder welches die Aminosäuresequenz von SEQ ID NO: 2 umfasst, wobei an Position 272 L-Leucin enthalten ist und das eine Nukleotidsequenz umfasst, welche mit der Nukleotidsequenz eines Polynukleotids identisch ist, das durch eine Polymerasekettenreaktion (PCR) unter Verwendung des Primerpaars erhältlich ist, deren Nukleotidsequenzen jeweils mindestens 15 aufeinanderfolgende Nukleotide umfassen, die aus der Nukleotidsequenz zwischen Position 1 und 750 von SEQ ID NO:3 oder SEQ ID NO:7 und aus der komplementären Nukleotidsequenz zwischen Position 3000 und 2245 von SEQ ID NO:3 oder SEQ ID NO:7 ausgewählt werden. Ein Beispiel für ein derartiges Primerpaar ist in SEQ ID NO:9 und SEQ ID NO:10 dargestellt. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Gegenstand der Erfindung ist weiterhin ein isoliertes Polynukleotid, das mit der zu SEQ ID NO:5 komplementären Nukleotidsequenz unter stringenten Bedingungen hybridisiert und für ein Polypeptid mit 2-Methylcitrat-Dehydratase Enzymaktivität kodiert, welches an der Position 272 der Aminosäuresequenz von SEQ ID NO:2 entsprechenden Position L-Leucin enthält

oder welches die Aminosäuresequenz von SEQ ID NO: 2 umfasst, wobei an Position 272 L-Leucin enthalten ist.

Anleitungen zur Hybridisierung von Nukleinsäuren bzw. Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO:5 komplementäre Nukleotidsequenz und die Zielsequenz, d. h. die mit der Sonde behandelten bzw. identifizierten Polynukleotide, mindestens 90% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 90% Identität zur Nukleotidequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 90% oder mindestens 91%, bevorzugt mindestens 92% oder mindestens 93% oder mindestens 94% oder mindestens 95% oder mindestens 96% und ganz besonders bevorzugt mindestens 97% oder mindestens 98% oder mindestens 99% Identität zur Sequenz bzw. komplementären Sequenz der eingesetzten Sonde besitzen und für ein Polypeptid mit 2-Methylcitrat-Dehydratase Enzymaktivität kodieren, welches den erfindungsgemäßen Aminosäureaustausch enthält. Die Nukleotidsequenz des auf diese Weise erhaltenen Polynukleotids wird mit bekannten Methoden bestimmt. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Die so erhaltenen Nukleotidsequenzen kodieren für Polypeptide mit 2-Methylcitrat-Dehydratase Enzymaktivität, die mindestens 90% bevorzugt mindestens 92% oder mindestens 94% oder mindestens 96%, und ganz besonders bevorzugt mindestens 97% oder mindestens 98% oder mindestens 99% identisch sind mit der Aminosäuresequenz von SEQ ID NO:6 bzw. SEQ ID NO:8 und den erfindungsgemäßen Aminosäureaustausch enthalten.

Gegenstand der Erfindung ist weiterhin ein isoliertes Polynukleotid, das für ein Polypeptid mit 2-Methylcitrat-Dehydratase Enzymaktivität kodiert, welches an der Position 272 der Aminosäuresequenz von SEQ ID NO:2 entsprechenden Position L-Leucin enthält
oder welches die Aminosäuresequenz von SEQ ID NO: 2 umfasst, wobei an Position 272 L-Leucin enthalten ist und das eine Aminosäuresequenz umfasst, die außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Aminosäuresequenz von SEQ ID NO:6 bzw. SEQ ID NO:8.

Gegenstand der Erfindung ist weiterhin ein isoliertes Polynukleotid, das für ein Polypeptid mit 2-Methylcitrat-Dehydratase Enzymaktivität kodiert, welches an der Position 272 der Aminosäuresequenz von SEQ ID NO:2 entsprechenden Position L-Leucin enthält
oder welches die Aminosäuresequenz von SEQ ID NO: 2 umfasst, wobei an Position 272 L-Leucin enthalten ist und das eine Nukleotidsequenz umfasst, die außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Nukleotidsequenz von SEQ ID NO:5.

Darüberhinaus sind solche isolierte Polynukleotide bevorzugt, die für ein Polypeptid mit 2-Methylcitrat-Dehydratase Enzymaktivität kodieren, welches an der Position 272 der Aminosäuresequenz von SEQ ID NO:2 entsprechenden Position L-Leucin enthält
oder welches die Aminosäuresequenz von SEQ ID NO: 2 umfasst, wobei an Position 272 L-Leucin enthalten ist und die mindestens ein Sequenzmotiv bzw. eine Aminosäuresequenz ausgewählt aus der Gruppe Arg-Glu-Leu-Asp-Phe-His-Asp-Thr-Phe-Leu-Ala-Ala-Asp/Glu-Tyr-Ser-His-Pro, Gly-Ile-Cys-Leu-His-Glu-His-Lys-Ile-Asp-His-Val-Ala-His-Leu-Gly-Pro und Pro-Ala-Pro-Ile-Trp-Glu-Gly-Glu-Asp-Gly-Val-Ile-Ala-Trp-Leu-Leu enthalten.

Die Bezeichnungen "Asp/Glu" bedeuten, dass an der entsprechenden Position "Asp oder Glu" vorliegen kann.

Gegenstand der Erfindung ist weiterhin ein isoliertes Polynukleotid, das für ein Polypeptid mit 2-Methylcitrat-Dehydratase Enzymaktivität kodiert, welches die Aminosäuresequenz von SEQ ID NO:6 bzw. 8 umfasst. Gegebenenfalls enthält das kodierte Polypeptid einen (1) oder mehrere konservative Aminosäuraustausch(e). Bevorzugt enthält das Polypeptid höchstens zwei (2), höchstens drei (3), höchstens vier (4) oder höchstens fünf (5) konservative Aminosäureaustausche.

Gegenstand der Erfindung ist weiterhin ein isoliertes Polynukleotid, das für ein Polypeptid mit 2-Methylcitrat-Dehydratase Enzymaktivität kodiert, welches die Aminosäuresequenz von SEQ ID NO:6 bzw. 8 einschließlich einer Verlängerung am N- oder C-Terminus um mindestens eine (1) Aminosäure umfasst. Diese Verlängerung beträgt nicht mehr als 50, 40, 30, 20, 10, 5, 3 oder 2 Aminosäuren bzw. Aminosäurereste.

Gegenstand der Erfindung sind schließlich auch prpD1-Allele, die für Polypeptid Varianten von SEQ ID NO:6 bzw. 8 kodieren, die eine oder mehrere Insertionen oder Deletionen enthalten. Bevorzugt enthalten diese maximal 5, maximal 4, maximal 3 oder maximal 2 Insertionen oder Deletionen von Aminosäuren. Die Sequenzmotiv Arg-Glu-Leu-Asp-Phe-His-Asp-Thr-Phe-Leu-Ala-Ala-Asp/Glu-Tyr-Ser-His-Pro und/oder Gly-Ile-Cys-Leu-His-Glu-His-Lys-Ile-Asp-His-Val-Ala-His-Leu-Gly-Pro und/oder Pro-Ala-Pro-Ile-Trp-Glu-Gly-Glu-Asp-Gly-Val-Ile-Ala-Trp-Leu-Leu wird/werden durch derartige Insertionen/Deletionen vorzugsweise nicht zerrissen.

Gegenstand der Erfindung ist weiterhin ein isoliertes Polynukleotid, das die Nukleotidsequenz gemäß SEQ ID NO:5 oder 7 umfasst.

Gegenstand der Erfindung ist schließlich ein isoliertes Polynukleotid enthaltend das prpD1-Allel der Mutante DM1916.

Gegenstand der Erfindung ist außerdem ein isoliertes Polynukleotid, das einen Teil der Kodierregion eines erfindungsgemäßen prpD1-Allels umfasst, wobei das isolierte Polynukleotid in jedem Fall den Teil der Kodierregion umfasst, der den Aminosäureaustausch an der Position 272 der Aminosäuresequenz des kodierten Polypeptids enthält.

Insbesondere ist ein Nukleinsäure-Molekül bzw. DNA-Fragment umfasst, das für mindestens eine Aminosäuresequenz entsprechend Position 252 bis 291 von SEQ ID NO:2, oder das für mindestens eine Aminosäuresequenz entsprechend Position 202 bis 341 von SEQ ID NO:2, oder das für mindestens eine Aminosäuresequenz entsprechend Position 152 bis 391 von SEQ ID NO:2, oder das für mindestens eine Aminosäuresequenz entsprechend Position 102 bis 441 von SEQ ID NO:2, oder das für mindestens eine Aminosäuresequenz entsprechend Position 52 bis 491 von SEQ ID NO:2, oder das für mindestens eine Aminosäuresequenz entsprechend Position 2 bis 495 von SEQ ID NO:2 kodiert, oder das für mindestens eine Aminosäuresequenz entsprechend Position 2 bis 496 von SEQ ID NO:2 kodiert, oder das für mindestens eine Aminosäuresequenz entsprechend Position 2 bis 497 von SEQ ID NO:2 kodiert, wobei an der Position entsprechend 272 von SEQ ID NO:2 L-Leucin enthalten ist.

Ein Beispiel eines Leserasters umfassend ein Polynukleotid, das für mindestens die Aminosäuresequenz von Position 252 bis 291 entsprechend SEQ ID NO:2 kodiert, wobei an der Position entsprechend 272 der Aminosäuresequenz eine der proteinogenen Aminosäuren (Xaa) ausgenommen L-Prolin enthalten ist, ist nachfolgend aufgeführt:

```
gcg tgg ctg tta tcg ggc aaa gat cat gtt tat cat gtg cca
Ala Trp Leu Leu Ser Gly Lys Asp His Val Tyr His Val Pro
            255                 260                 265

ttg ccg gaa cac ggc gag nnn aag ctg ggg att cta gag
Leu Pro Glu His Gly Glu Xaa Lys Leu Gly Ile Leu Glu
                270                 275

act tac aca aag gaa cat tca gcg gaa tat caa tcg cag
Thr Tyr Thr Lys Glu His Ser Ala Glu Tyr Gln Ser Gln
    280                 285                 290
```

Es ist ebenfalls als SEQ ID NO:17 dargestellt. Die von diesem Leseraster kodierte Aminosäuresequenz ist als SEQ ID NO:18 dargestellt. Die Position 21 in SEQ ID NO:18 entspricht der Position 272 von SEQ ID NO:2, 4, 6 bzw. 8.

Bevorzugt werden Nukleinsäuremoleküle die für mindestens eine Aminosäuresequenz entsprechend Position 252 bis 291 von SEQ ID NO:6 bzw. 8, oder mindestens entsprechend Position 202 bis 341 von SEQ ID NO: 6 bzw. 8, oder mindestens entsprechend Position 152 bis 391 von SEQ ID NO: 6 bzw. 8, oder mindestens entsprechend Position 102 bis 441 von SEQ ID NO: 6 bzw. 8, oder mindestens entsprechend Position 52 bis 491 von SEQ ID NO: 6 bzw. 8, oder mindestens entsprechend Position 2 bis 495 von SEQ ID NO: 6 bzw. 8, oder mindestens entsprechend Position 2 bis 496 von SEQ ID NO: 6 bzw. 8, oder mindestens entsprechend Position 2 bis 497 von SEQ ID NO: 6 bzw. 8 kodieren.

Ein Beispiel eines erfindungsgemäßen Leserasters umfassend ein Polynukleotid, das für mindestens die Aminosäuresequenz entsprechend Position 252 bis 291 von SEQ ID NO:6 bzw. 8 kodiert, ist nachfolgend aufgeführt:

```
gcg tgg ctg tta tcg ggc aaa gat cat gtt tat cat gtg cca
Ala Trp Leu Leu Ser Gly Lys Asp His Val Tyr His Val Pro
            255                 260                 265

ttg ccg gaa cac ggc gag ctc aag ctg ggg att cta gag
Leu Pro Glu His Gly Glu Leu Lys Leu Gly Ile Leu Glu
                270                 275

act tac aca aag gaa cat tca gcg gaa tat caa tcg cag
Thr Tyr Thr Lys Glu His Ser Ala Glu Tyr Gln Ser Gln
    280                 285                 290
```

Das Leseraster ist ebenfalls als SEQ ID NO:19 dargestellt. SEQ ID NO:20 zeigt die von diesem Leseraster kodierte Aminosäuresequenz. Die Position 21 in SEQ ID NO:20 entspricht der Position 272 von SEQ ID NO:2, 4, 6 bzw. 8.

Ganz besonders bevorzugt werden Nukleinsäuremoleküle, die mindestens eine Nukleotidsequenz entsprechend Position 1504 bis 1623 von SEQ ID NO:7, oder mindestens eine Nukleotidsequenz entsprechend Position 1354 bis 1773 von SEQ ID NO: 7, oder mindestens eine Nukleotidsequenz entsprechend Position 1204 bis 1923 von SEQ ID NO:7, oder mindestens eine Nukleotidsequenz entsprechend Position 1054 bis 2073 von SEQ ID NO:7, oder mindestens eine Nukleotidsequenz entsprechend Position 904 bis 2223 von SEQ ID NO:7, oder mindestens eine Nukleotidsequenz entsprechend Position 754 bis 2235 von SEQ ID NO:7, oder mindestens eine Nukleotidsequenz entsprechend Position 754 bis 2238 von SEQ ID NO:7, oder mindestens eine Nukleotidsequenz entsprechend Position 754 bis 2241 von SEQ ID NO:7, umfassen.

Die erfindungsgemäßen Leseraster, wie sie beispielhaft in SEQ ID NO:17 und 19 als Nukleotidsequenz und in SEQ ID NO:18 und SEQ ID NO:20 in Form der kodierten Aminosäuresequenz gezeigt sind, können darüberhinaus eine oder mehrere Mutationen enthalten, die zu einem oder mehreren konservativen Aminosäureaustauschen führen.

Bevorzugt führen die Mutationen zu maximal 4%, zu maximal 2% oder zu maximal 1% konservativen Aminosäureaustauschen. Weiterhin können die erfindungsgemäßen Leseraster eine oder mehrere stille Mutationen enthalten. Bevorzugt enthalten die erfindungsgemäßen Leseraster höchstens 4% und besonders bevorzugt höchstens 2% bis höchstens 1% stille Mutationen.

Die erfindungsgemäßen, isolierten Polynukleotide können dazu verwendet werden, um rekombinante Stämme von Mikroorganismen herzustellen, die im Vergleich zum Ausgangs- bzw. Elternstamm in verbesserter Weise Aminosäuren in das sie umgebende Medium abgeben oder im Zellinneren anhäufen.

Eine verbreitete Methode zum Einbau von Mutationen in Gene coryneformer Bakterien ist die des Allelaustausches, die auch unter der Bezeichnung "gene replacement" bekannt ist. Bei diesem Verfahren wird ein DNA-Fragment, welches die interessierende Mutation enthält, in den gewünschten Stamm eines coryneformen Bakteriums transferiert und die Mutation durch wenigstens zwei Rekombinationsereignisse bzw. "cross over"-Ereignisse in das Chromosom des gewünschten Stammes inkorporiert bzw. die im betreffenden Stamm vorhandene Sequenz eines Gens gegen die mutierte Sequenz ausgetauscht.

Von Schwarzer und Pühler (Bio/Technology 9, 84-87 (1991) wurde diese Methode verwendet um ein lysA-Allel, welches eine Deletion trug, und um ein lysA-Allel, welches eine Insertion trug, in das Chromosom von C. glutamicum anstelle des Wildtypgens einzubauen. Von Schäfer et al. (Gene 145, 69-73 (1994)) wurde diese Methode eingesetzt, um eine Deletion in das hom-thrB Operon von C. glutamicum einzubauen. Von Nakagawa et al. (EP 1108790) und Ohnishi et al. (Applied Microbiology and Biotechnology 58(2), 217-223 (2002)) wurde diese Methode eingesetzt um verschiedene Mutationen ausgehend von den isolierten Allelen in das Chromosom von C. glutamicum einzubauen. Auf diese Weise gelang es Nakagawa et al. eine als Val59Ala bezeichnete Mutation in das Homoserin-Dehydrogenase Gen (hom), eine als Thr311Ile bezeichnete Mutation in das Aspartatkinase-Gen (lysC bzw. ask), eine als Pro458Ser bezeichnete Mutation in das Pyruvat-Carboxylase-Gen (pyc) und eine als Ala213Thr bezeichnete Mutation in das Glucose-6-phosphat-Dehydrogenase Gen (zwf) von C. glutamicum Stämmen einzubauen.

Für ein erfindungsgemäßes Verfahren kann ein erfindungsgemäßes Polynukleotid verwendet werden, welches die gesamte Kodierregion umfasst, wie beispielsweise in SEQ ID NO:5 gezeigt, oder welches einen Teil der Kodierregion umfasst, wie beispielsweise die Nukleotidsequenz, die für mindestens die Aminosäuresequenz entsprechend Position 252 bis 291 von SEQ ID NO:6 bzw. 8 kodiert und die als SEQ ID NO:17 und 19 dargestellt ist. Der Teil der Kodierregion entsprechend SEQ ID NO:17 und 19 umfasst eine Länge von $\geq$ 120 Nukleobasen. Bevorzugt werden solche Teile von SEQ ID NO:7, die mindestens die Sequenz zwischen Position 1354 und 1773, oder mindestens zwischen Position 1204 und 1923, oder mindestens zwischen Position 1054 und 2073, umfassen und dementsprechend eine Länge von $\geq$ 420, $\geq$ 720 oder $\geq$ 1020 Nukleobasen besitzen.

Das DNA-Fragment enthaltend die interessierende Mutation liegt bei dieser Methode typischerweise in einem Vektor, insbesondere einem Plasmid, vor, das vorzugsweise von dem mit der Mutation zu versehenden Stamm nicht oder nur begrenzt repliziert wird. Als Hilfs- oder Zwischenwirt, in dem der Vektor replizierbar ist, wird im Allgemeinen ein Bakterium der Gattung Escherichia bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli aber nicht in coryneformen Bakterien replikativ. Besonders geeignet sind Vektoren, die ein konditional negativ dominant wirkendes Gen wie beispielsweise das sacB-Gen (Levansucrase-Gen) von beispielsweise Bacillus oder das galK-Gen (Galaktosekinase-Gen) von beispielsweise Escherichia coli enthalten. (Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat.) Diese ermöglichen die Selektion auf Rekombinationsereignisse, bei denen der Vektor aus dem Chromosom eliminiert wird. Weiterhin wurde von Nakamura et al. (US-A-6,303,2723) ein Temperatur-sensitives Plasmid für coryneforme Bakterien beschrieben, das lediglich bei Temperaturen unterhalb von 31°C replizieren kann.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das coryneforme Bakterium transferiert. Gegebenenfalls kann der Transfer der DNA auch durch Partikelbeschuss erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Die Beschreibung offenbart dementsprechend ein Verfahren zur Herstellung eines coryneformen Bakteriums, bei dem man

a) ein erfindungsgemäßes Polynukleotid in ein coryneformes Bakterium transferiert,

b) das im Chromosom des coryneformen Bakteriums vorhandene 2-Methylcitrat-Dehydratase Gen, das für eine Aminosäuresequenz mit L-Prolin an Position 272 oder an einer vergleichbaren Position der Aminosäuresequenz kodiert, gegen das Polynukleotid aus a) austauscht, das für eine Aminosäuresequenz kodiert, die an der Position 272 oder an einer vergleichbaren Position der Aminosäuresequenz eine andere proteinogene L-Aminosäure, vorzugsweise L-Leucin, besitzt, und

c) das gemäß Schritt a) und b) erhaltene coryneforme Bakterium vermehrt.

Auf diese Weise erhält man ein rekombinantes coryneformes Bakterium, welches anstelle des Wildtyp prpD1-Gens ein (1) erfindungsgemäßes prpD1-Allel enthält.

Ein Verfahren zur Herstellung eines Mikroorganismus besteht darin, dass man

a) ein erfindungsgemäßes Polynukleotid, das für ein Polypeptid mit 2-Methylcitrat-Dehydratase Enzymaktivität kodiert, in einen Mikrorganismus transferiert,

b) das Polynukleotid in dem Mikroorganismus repliziert, und

c) den gemäß Schritt a) und b) erhaltenen Mikroorganismus vermehrt.

Auf diese Weise erhält man einen rekombinanten Mikroorganismus, welcher mindestens eine (1) Kopie oder mehrere Kopien eines erfindungsgemäßen Polynukleotids enthält. Weitere Gegenstände der Erfindung sind dementsprechend Wirte bzw. Wirtszellen, bevorzugt Mikroorganismen, besonders bevorzugt coryneforme Bakterien und Bakterien der Gattung Escherichia, die die erfindungsgemäßen Polynukleotide enthalten. Gegenstand der Erfindung sind gleichfalls Mikrorganismen, die unter Verwendung der isolierten Polynukleotide hergestellt wurden. Derartige Mikroorganismen oder Bakterien werden auch als rekombinante Mikroorganismen oder rekombinante Bakterien bezeichnet. In gleicher Weise sind Vektoren, die die erfindungsgemäßen Polynukleotide enthalten, Gegenstand der Erfindung. Schließlich sind Wirte, die diese Vektoren enthalten, ebenfalls Gegenstand der Erfindung.

Zusätzlich kann es für die verbesserte Produktion von L-Aminosäuren, insbesondere L-Lysin, L-Tryptophan, L-Valin, L-Isoleucin und L-Homoserin, vorteilhaft sein, in den erfindungsgemäßen Mutanten oder rekombinanten Stämme verschiedene Gene zu überexprimieren. Die Verwendung endogener Gene wird im Allgemeinen bevorzugt.

Unter "endogenen Genen" oder "endogenen Nukleotidsequenzen" versteht man die in der Population einer Art vorhandenen Gene bzw. Nukleotidsequenzen oder Allele.

So kann für die Herstellung von L-Lysin eines oder mehrere der Gene, ausgewählt aus der Gruppe

- ein für ein Dihydrodipicolinat-Synthase (DapA, EC Nr. 4.2.1.52) kodierendes Gen dapA, wie beispielsweise das in der EP 0 197 335 beschriebene dapA-Gen des Wildtyps von Corynebacterium glutamicum,
- ein für eine Diaminopimelat-Decarboxylase (LysA, EC Nr. 4.1.1.20) kodierendes lysA-Gen, wie beispielsweise das in in der US 6,090,597 beschriebene lysA-Gen von Corynebacterium glutamicum ATCC13869,
- ein für eine Glucose-6-Phosphat Dehydrogenase (Zwf, EC Nr. 1.1.1.49) kodierendes Gen zwf, wie beispielsweise das in der JP-A-09224661 und EP-A-1108790 beschriebene zwf-Gen des Wildtyps von Corynebacterium glutamicum,
- die in der US-2003-0175911-A1 beschriebenen zwf-Allele von Corynebacterium glutamicum, die für ein Protein mit Glucose-6-Phosphat Dehydrogenase Aktivität kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist,
- ein für eine Pyruvat-Carboxylase (Pyc, EC Nr. 6.4.1.1) kodierendes Gen pyc, wie beispielsweise das in der DE-A-198 31 609 und EP 1108790 beschriebene pyc-Gen des Wildtyps von Corynebacterium glutamicum,
- das in der EP 1 108 790 beschriebene pyc-Allel von Corynebacterium glutamicum, das für ein Protein mit Pyruvat-Carboxylase Aktivität kodiert, bei dem L-Prolin an Position 458 der Aminosäuresequenz durch L-Serin ersetzt ist,

- die in der WO 02/31158 und insbesondere EP1325135B1 beschriebene pyc-Allele von Corynebacterium glutamicum, die für Proteine mit Pyruvat-Carboxylase Aktivität kodieren, welche einen oder mehrere der Aminosäureaustausche ausgewählt aus der Gruppe L-Valin an Position 1 ersetzt durch L-Methionin, L-Glutaminsäure an Position 153 ersetzt durch L-Asparaginsäure, L-Alanin an Position 182 ersetzt durch L-Serin, L-Alanin an Position 206 ersetzt durch L-Serin, L-Histidin an Position 227 ersetzt durch L-Arginin, L-Alanin an Position 455 ersetzt durch Glycin und L-Asparaginsäure an Position 1120 ersetzt durch L-Glutaminsäure tragen,

- ein für eine Aspartatkinase (LysC, EC Nr. 2.7.2.4) kodierendes lysC Gen wie beispielsweise das als SEQ ID NO:281 in der EP-A-1108790 (Siehe auch Zugangsnummer AX120085 und 120365) und das als SEQ ID NO:25 in der WO 01/00843 (Siehe Zugangsnummer AX063743) beschriebene von lysC-Gen des Wildtyps von Corynebacterium glutamicum,

- ein für eine feed-back resistente Aspartatkinase Variante kodierendes $lysC^{FBR}$ Allel, insbesondere entsprechend Tabelle 1,

- ein für ein Lysin-Export-Protein (LysE) kodierendes Gen lysE, wie beispielsweise das in der DE-A-195 48 222 beschriebene lysE-Gen von des Wildtyps Corynebacterium glutamicum,

- das für eine Aspartat-Aminotransferase (Aat, EC Nr. 2.6.1.1) kodierende aat-Gen (Das aat-Gen von Corynebacterium glutamicum ATCC13032 ist beispielsweise bei Kalinowski et al (Journal of Biotechnology 104 (1-3), 5-25 (2003); siehe auch Zugangsnummer NC_006958) beschrieben. Es wird dort als aspB-Gen bezeichnet. In der US 6,004,773 wird ein für eine Aspartat Aminotransferase kodierendes Gen als aspC bezeichnet. Marienhagen et al (Journal of Bacteriology 187 (22), 7693-7646 (2005) bezeichnen das aat-Gen als aspT-Gen.)), und

- das für das Zwa1-Protein kodierende Gen zwa1 des Wildtyps von Corynebacterium glutamicum (US 6,632,644)

überexprimiert werden.

Unter Überexpression versteht man allgemein eine Erhöhung der intrazellulären Konzentration oder Aktivität einer Ribonukleinsäure, eines Proteins oder eines Enzyms. Im Falle der vorliegenden Erfindung werden prpD1-Allele bzw. Polynukleotide überexprimiert, die für 2-Methylcitrat-Dehydratasen kodieren, die an Position 272 der Aminosäuresequenz des kodierten Polypeptids eine proteinogene Aminosäure ausgenommen L-Prolin enthalten, wobei der Austausch gegen L-Leucin bevorzugt wird. Es ist bekannt, dass durch wirtseigene Enzyme - sogenannte Aminopeptidasen - N-terminale Aminosäuren, insbesondere das N-terminale Methionin, vom gebildeten Polypeptid abgespalten werden können. Die genannte Erhöhung der Konzentration oder Aktivität eines Genproduktes lässt sich beispielsweise dadurch erzielen, dass man die Kopienzahl der entsprechenden Polynukleotide um mindestens eine Kopie erhöht.

Eine weit verbreitete Methode zur Erhöhung der Kopienzahl besteht darin, dass man das entsprechende Gen oder Allel in einen Vektor, bevorzugt ein Plasmid, einbaut, der von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al. (Journal of Biotechnology 104, 27-40 (2003)).

Eine andere verbreitete Methode zur Erzielung einer Überexpression ist das Verfahren der chromosomalen Genamplifikation. Bei dieser Methode wird mindestens eine zusätzliche Kopie des interessierenden Gens oder Allels in das Chromosom eines coryneformen Bakteriums eingefügt.

In einer Ausführungsform, wie sie beispielsweise bei Reinscheid et al. (Applied and Environmental Microbiology 60, 126-132 (1994)) für das hom-thrB-Operon beschrieben ist, wird ein in C. glutamicum nicht-replikatives Plasmid, welches das interessierende Gen enthält, in ein coryneformes Bakterium transferiert. Nach homologer Rekombination mittels eines "cross over"-Ereignisses enthält der resultierende Stamm mindestens zwei Kopien des betreffenden Gens bzw. Allels.

In einer anderen Ausführungsform, die in der WO 03/040373 und US-2003-0219881-A1 beschrieben ist, wird eine oder mehrere Kopie(n) des interessierenden Gens mittels mindestens zweier Rekombinationsereignisse in einen gewünschten Ort des Chromosoms von C. glutamicum eingefügt. Auf diese Weise wurde beispielsweise eine Kopie eines lysC-Allels, das für eine L-Lysin insensitive Aspartatkinase kodiert, in das gluB-Gen von C. glutamicum eingebaut.

In einer weiteren Ausführungsform, die in der WO 03/014330 und US-2004-0043458-A1 beschrieben ist, wird mittels mindestens zweier Rekombinationsereignisse am natürlichen Ort mindestens eine weitere Kopie, vorzugsweise in tandem-Anordnung zu dem bereits vorhandenen Gen oder Allel, des interessierenden Gens eingebaut. Auf diese Weise wurde beispielsweise eine tandem-Duplikation eines $lysC^{FBR}$-Allels am natürlichen lysC-Genort erzielt.

Schließlich ist es möglich die Kopienzahl mit Hilfe von Transposons und IS-Elementen zu erhöhen (Siehe: US 5,804,414, US 5, 591, 577).

Eine weitere Methode zur Erzielung einer Überexpression besteht darin, das entsprechende Gen bzw. Allel in funktioneller Weise (operably linked) mit einem Promotor bzw. einer Expressionskassette zu verknüpfen. Geeignete Promotoren für Corynebacterium glutamicum sind beispielsweise in dem Übersichtsartikel von Patek et al. (Journal of Biotechnology 104(1-3), 311-323 (2003) beschrieben. In gleicher Weise können die von Vasicova et al (Journal of Bacteriology 181, 6188-6191 (1999)) beschrieben Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten, von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden.

Darüberhinaus kann die Promotor- und Regulationsregion oder die Ribosomenbindungsstelle, die sich stromaufwärts des Strukturgens befindet, mutiert werden. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Alternativ kann weiterhin eine Überexpression des betreffenden Gens oder Allels durch Veränderung der Medienzusammensetzung und Kulturführung erreicht werden.

Durch die Maßnahmen der Überexpression wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000%, bezogen auf die Aktivität oder Konzentration des Proteins im Ausgangs-Mikroorganismus bzw. Elternstammes erhöht. Unter einem Ausgangs-Mikroorganismus oder Elternstamm versteht man einen Mikroorganismus, an dem die Massnahmen der Erfindung durchgeführt werden.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 272: 2630-2647 (1999)) bestimmt werden.

Weiterhin kann es für die Produktion von L-Lysin vorteilhaft sein, neben der Verwendung der erfindungsgemäßen Allele des prpD1-Gens, gegebenenfalls bei gleichzeitige Überexpression von mindestens einem der Gene ausgewählt aus der vorstehend genannten Gruppe von Genen" gleichzeitig eines oder mehrere der endogenen Gene, ausgewählt aus der Gruppe

- ein für die Glucose-6-Phosphat-Isomerase (Pgi, EC Nr. 5.3.1.9) kodierendes Gen pgi, wie beispielsweise das in der US 6,586,214 und US 6,465,238 beschriebene pgi-Gen von Corynebacterium glutamicum,
- ein für die Homoserin-Dehydrogenase(Hom, EC Nr. 1.1.1.3) kodierendes Gen hom, wie beispielsweise das in der EP-A-0131171 beschriebene hom-Gen von Corynebacterium glutamicum,
- ein für die Homoserin-Kinase (ThrB, EC Nr. 2.7.1.39) kodierendes Gen thrB, wie beispielsweise das von Peoples et al. (Molecular Microbiology 2 (1988): 63 - 72)) beschriebene thrB-Gen von Corynebacterium glutamicum und
- ein für die Phosphofruktokinase(PfkB, EC Nr. 2.7.1.56) kodierendes Gen pfkB, wie beispielsweise das in der WO 01/00844 (Sequenz Nr. 57) beschriebene pfkB-Gen von Corynebacterium glutamicum,
- ein für die Malat-Dehydrogenase (Mdh, EC Nr. 1.1.1.37) kodierendes Gen mdh, wie beispielsweise in der WO 02/02778 beschrieben,
- ein für die Malat-Chinon Oxidoreduktase (Mqo, EC Nr. 1.1.99.16) kodierendes Gen mqo, wie beispielsweise in der US 7,094,106 und PCT/EP2005/057216 beschrieben

abzuschwächen oder auszuschalten.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, bzw. der Aktivität oder Konzentration des Proteins im Ausgangs-Mikroorganismus, herabgesenkt.

Als Mutationen zur Erzeugung einer Abschwächung kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stopp-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stopp-Kodon im Kodierbereich, so führt dies ebenfalls zu einem vorzeitigen Abbruch der Translation. Die genannten Massnahmen werden vorzugsweise im 5'-terminalen Teil der Kodierregion durchgeführt, welcher für den N-Terminus des Polypeptids kodiert. Bezeichnet man die Gesamtlänge eines Polypeptids (gemessen als Anzahl der chemisch verbundenen L-Aminosäuren) mit 100%, so gehört - im Rahmen der vorliegenden Erfindung - zum N-Terminus des Polypeptids der Teil der Aminosäuresequenz, welcher, gerechnet ab der Start-Aminosäure L-Formyl-Methionin 80% der nachfolgenden L-Aminosäuren enthält.

Weitere Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Weitere Maßnahmen sind im Stand der Technik beschrieben.

Ein Methode zur gezielten Verringerung der Genexpression besteht darin, das abzuschwächende Gen unter die Kontrolle eines durch Zugabe dosierter Mengen von IPTG (Isopropyl-β-D-thiogalactopyranosid) induzierbaren Promotors, wie zum Beispiel des trc-Promotors oder des tac-Promotors, zu stellen. Hierzu eignen sich Vektoren wie beispielsweise der Escherichia coli Expressionsvektor pXK99E (WO0226787; hinterlegt gemäß Budapester Vertrag am 31. Juli 2001 in DH5alpha/pXK99E als DSM14440 bei der Deutschen Sammlung für Mikroorganismen und Zellkulturen (DSMZ, Braunschweig, Deutschland)) oder pVWEx2 (Wendisch, Ph. D. thesis, Berichte des Forschungszentrums Jülich, Jül-3397, ISSN 0994-2952, Jülich, Deutschland (1997)), die eine IPTG-abhängige Expression des klonierten Gens in Corynebacterium glutamicum ermöglichen.

Eingesetzt wurde diese Methode beispielsweise in der Patentschrift WO0226787 zur regulierten Expression des deaD-Gens durch Integration des Vektors pXK99EdeaD in das Genom von Corynebacterium glutamicum und von Simic et al. (Applied and Environmental Microbiology 68: 3321-3327 (2002)) zur regulierten Expression des glyA-Gens durch Integration des Vektors pK18mobglyA' in Corynebacterium glutamicum.

Eine weitere Methode zur spezifischen Verringerung der Genexpression ist die Antisense-Technik, wobei kurze Oligodesoxnyukleotide oder Vektoren zur Synthese längerer Antisense-RNA in die Zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371).

Die durch die Massnahmen der Erfindung erhaltenen isolierten coryneformen Bakterien zeigen eine im Vergleich zum eingesetzten Ausgangsstamm bzw. Elternstamm erhöhte Ausscheidung bzw. Produktion der gewünschten Aminosäure in einem Fermentationsprozess.

Unter isolierten Bakterien sind die erfindungsgemäßen, isolierten bzw. erzeugten Mutanten und rekombinanten Bakterien, insbesonders coryneformer Bakterien, zu verstehen, welche ein prpD1-Allel enthalten, das für eine 2-Methylcitrat-Dehydratase kodiert, die den beschriebenen Aminosäureaustausch an Position 272 der Aminosäuresequenz enthält.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der ProduktAusbeute (gebildetes Produkt pro verbrauchter KohlenstoffQuelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Die erfindungsgemäßen, isolierten coryneformen Bakterien können kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium bzw. Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und'Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt'werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5,605,818, in der US 5,275,940, in der US 4,275,157 und in der US 4,224,409 beschrieben.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Gegenstand der Erfindung ist dementsprechend ein Verfahren zur Herstellung von L-Lysin bei dem man

a) ein isoliertes coryneformes Bakterium in einem geeignetem Medium fermentiert, wobei das Bakterium ein für ein Polypeptid mit 2-Methylcitrat-Dehydratase Enzymaktivität kodierendes Gen enthält, wobei das Polypeptid an der Position 272 der Aminosäuresequenz von SEQ ID NO:2 entsprechenden Position L-Leucin enthält oder wobei das Polypeptid die Aminosäuresequenz von SEQ ID NO: 2 umfasst, bei der an Position 272 L-Leucin enthalten ist, und

b) die L-Aminosäure in der Fermentationsbrühe oder in den Zellen des isolierten coryneformen Bakteriums akkumuliert.

Daran schließt sich im Allgemeinen das Sammeln (collecting) der im Nährmedium bzw. in der Fermentationsbrühe und/oder in den Zellen der Bakterien akkumulierten L-Aminosäure an, um zu einem festen oder flüssigen Produkt zu gelangen.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium bzw. die während der Fermentation eingesetzten Medien enthält/enthalten sämtliche Substanzen bzw. Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete, gewünschte Aminosäure, c) die im Laufe der Fermentation gebildeten organischen Nebenprodukte und d) die durch die Fermentation nicht verbrauchten Bestandteile des/der eingesetzten Fermentationsmediums/ Fermentationsmedien bzw. der Einsatzstoffe wie beispielsweise Vitamine wie Biotin, Aminosäuren wie Homoserin oder Salze wie Magnesiumsulfat.

Zu den organischen Nebenprodukten gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben typischerweise einen Aminosäuregehalt von 30 g/kg bis 200 g/kg oder 40 g/kg bis 180 g/kg oder 50 g/kg bis 150 g/kg. Der Gehalt an Biomasse (als getrocknete Biomasse) beträgt im Allgemeinen 20 bis 50 g/kg.

Im Falle der Aminosäure L-Lysin sind im Stand der Technik im wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem bzw. reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenenfalls die Biomasse des eingesetzten Mikroorganismus mit einem Anteil von >0% - 100%.

Im Falle der Aminosäuren L-Valin, L-Isoleucin, L-Prolin, L-Tryptophan und L-Homoserin sind im Stand der Technik im wesentlichen Produktformen bekannt, die die betreffenden Aminosäuren in aufgereinigter bzw. reiner Form ($\geq$ 95 Gew.-% oder $\geq$ 98 Gew.-%) enthalten.

Entsprechend der verschiedenen Produktformen kennt man verschiedenste Verfahren, bei denen die L-Aminosäure aus der Fermentationsbrühe gesammelt, isoliert oder gereinigt wird, um das L-Aminosäure haltige Produkt oder die gereinigte L-Aminosäure herzustellen.

Zur Herstellung von festen, reinen L-Aminosäuren werden im wesentlichen Methoden der Ionenaustauschchromatographie gegebenenfalls unter Verwendung von Aktivkohle und Methoden der Kristallisierung angewendet. Im Falle des Lysin erhält man auf diese Weise die entsprechende Base oder ein entsprechendes Salz wie beispielsweise das Monohydrochlorid (Lys-HCl) oder das Lysinsulfat ($Lys_2$-$H_2SO_4$).

Im Falle des Lysin ist in der EP-B-0534865 ein Verfahren zur Herstellung wässriger, basischer L-Lysin haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden auch solche Verfahren eingesetzt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse bzw. die Biomasse enthaltene Fermentationsbrühe während eines geeigneten Verfahrensschrittes inaktiviert beispielsweise durch thermische Behandlung (Erhitzen) oder durch Säurezugabe.

Die chemischen Bestandteile der Biomasse sind unter anderem die Zellhülle, beispielsweise das Peptidoglykan und das Arabinogalaktan, das Protein bzw. Polypeptid, beispielsweise das 2-Methylcitrat-Dehydratase Polypeptid, Lipide und Phospholipide und Nukleinsäuren (DNA und RNA), beispielsweise Polynukleotide enthaltend die erfindungsgemäße Mutation. Als Folge der Massnahmen der Inaktivierung und/oder der weitereren Verfahrensschritte (beispielsweise Ansäuerung,Sprühtrocknung, Granulation etc.) liegen Nukleinsäuren typischerweise als Fragmente mit eine Länge von unter anderem ≥40 - 60 bp, >60 - 80 bp, >80 - 100 bp, >100 - 200 bp, >200 - 300 bp, >300 - 400 bp, >400 - 500 bp, >500 - 750 bp, >750 - 1000 bp, >1000 -1250 bp, >1250 - 1500 bp, >1500 - 1750 bp, >1750 - 2000 bp, >2000 - 2500 bp, >2500 - 3000 bp, >3000 - 4000 bp, >4000 - 5000 bp vor.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern (US 6,340,486 oder US 6, 465, 025). Schließlich kann die Brühe auch durch Zusatz von Natriumbisulfit ($NaHSO_3$, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen bzw. eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 μm Durchmesser gemeint.

Mit "grobkörnig" ist ein Produkt mit einem überwiegendem Anteil (> 50 %) einer Korngröße von 200 bis 2000 μm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerbares und weitgehend staubfreies Produkt überführt werden.

Der Begriff "staubfrei" bedeutet, daß das Produkt lediglich geringe Anteile (< 5 %) an Körngrößen unter 100 μm Durchmesser enthält.

"Lagerbar", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann ohne das ein wesentlicher Verlust (< 5%) der jeweiligen Aminosäure auftritt.

Ein weiterer Gegenstand der Erfindung ist dementsprechend ein Verfahren zur Herstellung eines L-Lysin Produktes, bevorzugt Tierfuttermittel-Additivs, aus Fermentationsbrühen, gekennzeichnet durch die Schritte

a) Kultivierung und Fermentation eines L-Aminosäure ausscheidenden coryneformen Bakteriums, welches mindestens ein prpD1-Allel enthält, das für ein Polypeptid mit 2-Methylcitrat-Dehydratase Aktivität kodiert, wobei das Polypeptid an der Position 272 der Aminosäuresequenz von SEQ ID NO:2 entsprechenden Position L-Leucin enthält

oder wobei das Polypeptid die Aminosäuresequenz von SEQ ID NO: 2 umfasst, bei der an Position 272 L-Leucin enthalten ist, in einem Fermentationsmedium,

b) Entfernung der während der Fermentation gebildeten Biomasse in einer Menge von 0 bis 100 Gew.-%, und

c) Trocknung der gemäß a) und/oder b) erhaltenen Fermentationsbrühe, um das Produkt in der gewünschten Pulver- oder Granulatform zu erhalten

wobei gegebenenfalls vor Schritt b) oder c) eine Säure ausgewählt aus der Gruppe Schwefelsäure, Phosphorsäure oder Salzsäure hinzugefügt wird. Vorzugsweise wird im Anschluss an Schritt a) oder b) Wasser aus der L-Aminosäure haltigen Fermentationsbrühe entfernt (Aufkonzentration).

Die Beschreibung offenbart auch ein Verfahren zur Herstellung eines Lysinsulfat haltigen Produktes, das in seinen Grundzügen in der DE 102006016158 beschrieben ist, und bei dem die unter Verwendung der erfindungsgemäßen Mikroorganismen erhaltene Fermentationsbrühe, aus der die Biomasse gegebenenfalls ganz oder teilweise abgetrennt wurde, weiterverarbeitet wird, indem man ein Verfahren durchführt das mindestens folgende Schritte umfasst:

a) den pH-Wert durch Zugabe von Schwefelsäure auf 4,0 bis 5,2, insbesondere 4,9 bis 5,1, absenkt, und ein molares Sulfat/L-Lysin-Verhältnis von 0,85 bis 1,2, bevorzugt 0,9 bis 1,0, besonders bevorzugt >0,9 bis <0,95, in'der Brühe einstellt, gegebenenfalls durch Zugabe von einer weiteren oder mehreren Sulfat-haltigen Verbindung(n) und

b) das so erhaltene Gemisch durch Wasserentzug aufkonzentriert, und gegebenenfalls granuliert,
wobei gegebenenfalls vor Schritt a) eine oder beide der folgenden Massnahmen durchgeführt wird/werden:

c) Messung des molaren Verhältnisses von Sulfat/L-Lysin zur Ermittlung der benötigten Menge an Sulfat-haltigen Verbindung(n)

d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfit, besonders bevorzugt Natriumhydrogensulfit in einer Konzentration von 0,01 bis 0,5 Gew.-%, bevorzugt 0,1 bis 0,3 Gew.-%, besonders bevorzugt 0,1 bis 0,2 Gew.-% bezogen auf die Fermentationsbrühe hinzugesetzt.

Als bevorzugte Sulfat-haltigen Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [$SO_4^{2-}$] / [L-Lysin] berechnet. Diese Formel berücksichtigt die Tatsache, dass das $SO_4^{2-}$ Anion zweiwertig ist. Ein Verhältnis V = 1 bedeutet, dass ein stöchiometrisch zusammengesetztes $Lys_2(SO_4)$ vorliegt, während bei einem Verhältnis von V = 0,9 ein 10%iger Sulfatunterschuss und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, bzw. Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzlich Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.-% besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz bzw. dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Ein weiterer Gegenstand der Erfindung ist ein Verfahren zur Herstellung eines festen Lysin-haltigen Produktes, wie es in seinen Grundzügen in der US 20050220933 beschrieben ist, und das die Aufarbeitung der unter Verwendung der erfindungsgemäßen Mikroorganismen erhaltenen Fermentationsbrühe in folgenden Schritten umfasst:

a) Filtration der Fermentationsbrühe, bevorzugt mit einem Membranfilter, so dass ein Biomasse-haltiger Schlamm und ein Filtrat erhalten wird,

b) Aufkonzentration des Filtrates, bevorzugt so, dass ein Feststoffgehalt von 48 bis 52 Gew.-% erhalten wird,

c) Granulation des in Schritt b) erhaltenen Konzentrates, bevorzugt bei einer Temperatur von 50°C bis 62°C, und

d) Beschichtung des in c) erhaltenen Granulates mit einem oder mehreren der Beschichtungsmittel (coating agent(s))

Zur Beschichtung in Schritt d) werden bevorzugt Beschichtungsmittel verwendet, welche ausgewählt werden aus der Gruppe, bestehend aus

d1) der in Schritt a) erhaltenen Biomasse,

d2) einer L-Lysin-haltigen Verbindung, bevorzugt ausgewählt aus der Gruppe L-Lysinhydrochlorid oder L-Lysinsulfat,

d3) einem im wesentlichen L-Lysin-freien Stoff mit L-Lysingehalt < 1 Gew.-%, bevorzugt < 0,5 Gen.-%, bevorzugt ausgewählt aus der Gruppe Stärke, Karageenan, Agar, Kieselsäuren, Silikate, Schrote, Kleien und Mehle, und

d4) einem wasserabstoßenden Stoff, bevorzugt ausgewählt aus der Gruppe Öle, Polyethylenglykole und flüssige Paraffine.

Im Falle des Lysin wird bei der Herstellung Lysin-haltiger Produkte das Verhältnis der Ionen bevorzugt so eingestellt, dass das äquivalente Ionenverhältnis entsprechend nachstehender Formel

$$2x[SO_4^{2-}]+[Cl^-]-[NH_4^+]-[Na^+]-[K^+]-2x[Mg^{2+}]-2x[Ca^{2+}]/[L\text{-}Lys]$$

0,68 bis 0,95, bevorzugt 0,68 bis 0,90 ergibt, so wie von Kushiki et al. in der US 20030152633 beschrieben (In den "[]" sind die molaren Konzentrationen anzugeben.).

Im Falle des Lysin hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Im Falle einer elektrisch neutralen Aminosäure wie dem L-Tryptophan hat das auf diese Weise hergestellte feste Produkt auf Fermentationsbrühebasis einen Aminosäuregehalt von mindestens 5 Gew.-%, 10 Gew.-%, 20 Gew.-%, 30 Gew.-% und maximal 50 Gew.-%, 60 Gew.-%, 70 Gew.-%, 80 Gew.-%, 90 Gew.-% oder bis 95 Gew.-%.

Der Wassergehalt des festen Produktes beträgt bis zu 5 Gew.-%, bevorzugt bis zu 4 Gew.-%, und besonders bevorzugt weniger als 3 Gew.-%.

Die Beschreibung offenbart auch ein L-Lysin haltiges Futtermitteladditiv auf Fermentationsbrühebasis, welches folgende Merkmale aufweist

a) einen Lysingehalt (als Base) von mindestens 10 Gew.-% bis maximal 73 Gew.-%,

b) einen Wassergehalt von höchstens 5 Gew.-%, und

c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Die Beschreibung offenbart auch ein L-Tryptophan haltiges Futtermitteladditiv auf Fermentationsbrühebasis, welches folgende Merkmale aufweist

a) einen Tryptophangehalt von mindestens 5 Gew.-% bis maximal 95 Gew.-%,

b) einen Wassergehalt von höchstens 5 Gew.-%, und

c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Der Stamm MH20-22B wurde am 28 Oktober 2004 bei der Deutschen Sammlung für Mikroorganismen und Zellkulturen (DSMZ, Braunschweig, Deutschland) als DSM 16835 hinterlegt.
Die erfindungsgemäße Mutante Corynebacterium glutamicum DM1916, die L-Leucin an Position 272 der Aminosäuresequenz des PrpD1-Polypeptids enthält, wurde am 15. Mai 2006 bei der Deutschen Sammlung für Mikroorganismen und Zellkulturen (DSMZ, Braunschweig, Deutschland) als DSM 18258 hinterlegt.
Die vorliegende Erfindung wird im Folgenden anhand von Ausführungsbeispielen näher erläutert.

Beispiel 1

Mutagenese des L-Lysin-produzierenden Stammes DM416

Der Corynebacterium glutamicum Stamm DM416 wurde als Ausgangsstamm für die Mutagenese mit N-Methyl-N'-Nitro-N-Nitrosoguanidin (MNNG) eingesetzt. Der Stamm DM416 ist eine Homoserin- und Leucin-bedürftige Mutante von Corynebacterium glutamicum und unter der Bezeichnung ATCC21543 bei der American Type Culture Collection (Manassas, VA, USA) hinterlegt.
Der Stamm DM416 wurde in 10 ml LB-Bouillon (Merck, Darmstadt, Germany), die in einem 100 ml Erlenmeyerkolben enthalten waren, für 24 Stunden bei 33°C und 200 rpm auf einem Rotations-Schüttler vom Typ Certomat BS-1 (B. Braun Biotech International, Melsungen, Deutschland) kultiviert. Anschließend wurde die Kultur abzentrifugiert, das Sediment in 10 ml 0,9% NaCl-Lösung resuspendiert, die erhaltene Suspension erneut abzentrifugiert und das erhaltene Sediment in 10 ml 0,9% NaCl-Lösung aufgenommen. 5 ml dieser Zellsuspension wurden mit 400µg/ml MNNG für 15 Minuten bei 30°C und 200 rpm auf einem Schüttler (siehe oben) behandelt. Anschließend wurde der Mutageneseansatz abzentrifugiert und das Sediment in 10 ml 2% Na-Thiosulfat in 0,9% NaCl-Puffer (pH = 6,0) aufgenommen. Die Zellsuspension wurde anschließend im Verhältnis 1:1000, 1:10000 und 1:100000 mit 0,9% NaCl-Lösung verdünnt, und Aliquots auf Hirn-Herz-Agar (Merck, Darmstadt, Deutschland) ausplattiert. Auf diese Weise wurden ungefähr 4000 Mutanten isoliert.

Beispiel 2

Leistungstest der Mutanten des Stammes DM416

Die in Beispiel 1 erhaltenen Mutanten wurden in einem zur Produktion von Lysin geeigneten Nährmedium kultiviert und der Lysingehalt im Kulturüberstand bestimmt.
Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

Medium MM

| | |
|---|---|
| CSL | 5 g/l |
| MOPS | 20 g/l |
| Glucose (getrennt autoklaviert) | 50 g/l |
| Salze: | |
| $(NH_4)_2SO_4)$ | 25 g/l |
| $KH_ZPO_4$ | 0,1 g/l |
| $MgSO_4$ * 7 $H_2O$ | 1,0 g/l |
| $CaCl_2$ * 2 $H_2O$ | 10 mg/l |
| $FeSO_4$ * 7 $H_2O$ | 10 mg/l |
| $MnSO_4$ * $H_2O$ | 5,0mg/l |
| Biotin (sterilfiltriert) | 0,3 mg/l |
| Thiamin * HCl (sterilfiltriert) | 0,2 mg/l |
| L-Leucin (sterilfiltriert) | 0,2 g/l |
| L-Homoserin (sterilfiltriert) | 0,2 g/l |
| $CaCO_3$ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken aütoklavierte $CaCO_3$ zugesetzt.

Die Kultivierung erfolgte in Volumina von 10 ml, die in 100 ml Erlenmeyerkolben mit Schikanen enthalten waren. Die Temperatur betrug 33°C, die Umdrehungszahl 250 rpm und die Luftfeuchte 80%.

Nach 24 Stunden wurde die optische Dichte (OD) bei einer Meßwellenlänge von 660 nm mit dem Biomek 1000 (Beckmann Instruments GmbH, München, Deutschland) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Eine Mutante, die sich durch eine erhöhte Lysinbildung auszeichnete, wurde als DM1916 bezeichnet.

Tabelle 1

| Stamm | OD(660) | Lysin-HCl (g/l) |
|---|---|---|
| DM416 | 6,3 | 1,43 |
| DM1916 | 6,3 | 1,61 |

Beispiel 3

Sequenzierung des prpD1-Allels der Mutante DM1916

Aus dem Klon DM1916 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher das prpD1-Gen bzw. Allel trägt. Aufgrund der für C. glutamicum bekannten Sequenz des prpD1-Gens (Sequenz Nr. 770 aus EP1108790) wurden folgende Primer-Oligonukleotide für die PCR ausgewählt:

prpD1_XL_A1 (SEQ ID NO: 9):

5' gcgaattcta aactgcgtga ggttgtgg 3'

prpD1_XL_A2 (SEQ ID NO: 10):

5' gcgaattctc cccacatcaa caccattc 3'

Die dargestellten Primer wurden von der Firma MWG Biotech (Ebersberg, Deutschland) synthetisiert und nach der Standard-PCR-Methode von Innis et al. (PCR Protocols. A Guide to Methods and Applications, 1990, Academic Press) die PCR Reaktion durchgeführt. Die Primer ermöglichen die Amplifizierung eines ca. 1,63 kb langen DNA-Abschnittes, welcher das prpD1-Gen bzw. Allel trägt. Außerdem enthalten die Primer die Sequenz für eine Schnittstelle der Restriktionsendonuklease EcoRI, die in der oben dargestellten Nukleotidabfolge durch Unterstreichen markiert ist.

Das amplifizierte DNA-Fragment von ca. 1,63 kb Länge, welches das prpD1-Allel des Stammes DM1916 trägt, wurde durch Elektrophorese in einem 0,8%igen Agarosegel identifiziert, aus dem Gel isoliert und mit den üblichen Methoden aufgereinigt (QIAquick Gel Extraction Kit, Qiagen, Hilden).

Die Nukleotidsequenz des amplifizierten DNA-Fragmentes bzw. PCR-Produktes wurde von der Firma Agowa (Berlin, Deutschland) durch Sequenzierung ermittelt. Die Sequenz des PCR-Produktes ist in der SEQ ID NO: 13 dargestellt. Die Sequenz der Kodierregion ist zusätzlich in der SEQ ID NO: 5 dargestellt. Die sich mit Hilfe des Programmes Patentin ergebende Aminosäuresequenz des dazugehörigen Methylcitrat-Dehydratase-Proteins ist in der SEQ ID NO: 6 dargestellt.

An der Position 815 der Nukleotidsequenz der Kodierregion des prpD1-Allels von Stamm DM1916 befindet sich die Base Thymin (SEQ ID NO: 5). An der entsprechenden Position des Wildtypgens befindet sich die Base Cytosin (SEQ ID NO: 1).

An der Position 272 der Aminosäuresequenz des Methylcitrat-Dehydratase-Proteins von Stamm DM1916 befindet sich die Aminosäure Leucin (SEQ ID NO: 6). An der entsprechenden Position des Wildtyp-Proteins befindet sich die Aminosäure Prolin (SEQ ID NO: 2).

Das prpD1-Allel, welches an der Position 815 der Kodierregion die Base Thymin enthält und dementsprechend für ein Methylcitrat-Dehydratase-Protein kodiert, welches an Position 272 der Aminosäuresequenz die Aminosäure Leucin enthält, wird im Folgenden als prpD1_P272L-Allel bezeichnet. Bei der Bezeichnung "prpD1_P272L" steht P für L-Prolin, L für L-Leucin und 272 gibt die Position des Aminosäureaustausches an (Siehe SEQ ID NO: 2 und 6) .

Die Corynebacterium glutamicum Mutante DM1916, die L-Leucin an Position 272 der Aminosäuresequenz des PrpD1-Polypeptids enthält, wurde am 15. Mai 2006 bei der Deutschen Sammlung für Mikroorganismen und Zellkulturen (DSMZ, Braunschweig, Deutschland) als DSM 18258 hinterlegt.

## Beispiel 4

### Austausch des prpD1-Wildtypgens von Stamm DM416 gegen das prpD1_P272L-Allel

### 4.1 Konstruktion des Austauschvektors pK418mobsacB_prpD1_P272L

Das in Beispiel 3 beschriebene, mittels PCR hergestellte DNA-Fragment von ca. 1,63 kb Länge, welches das prpD1_P272L-Allel trägt, wurde mittels Austauschmutagenese unter Zuhilfenahme des bei Schäfer et al. (Gene, 14, 69-73 (1994)) beschriebenen sacB-Systems in das Chromosom des in Beispiel 1 beschriebenen C. glutamicum Stammes DM416 eingebaut. Dieses System ermöglicht die Herstellung bzw. die Selektion von Allel-Austauschen, die sich durch homologe Rekombination vollziehen.

Dazu wurde das ca. 1,63 kb große prpD1_P272L-Fragment mit der Restriktionsendonuklease EcoRI gespalten, durch Elektrophorese in einem 0,8%igen Agarosegel identifiziert und anschließend aus dem Gel isoliert und mit den üblichen Methoden aufgereinigt (QIAquick Gel Extraction Kit, Qiagen, Hilden).

Der mobilisierbare Klonierungsvektor pK18mobsacB wurde mit dem Restriktionsenzym EcoRI verdaut und die Enden mit alkalischer Phosphatase (Alkaline Phosphatase, Boehringer Mannheim, Deutschland) dephosphoryliert. Der so vorbereitete Vektor wurde mit dem ca. 1,63 kb prpD1 P272L-Fragment gemischt und der Ansatz mit T4-DNA-Ligase (Amersham-Pharmacia, Freiburg, Deutschland) behandelt.

Anschließend wurde der E. coli Stamm S17-1 (Simon et al., Bio/Technology 1: 784-791, 1993) mit dem Ligationsansatz transformiert (Hanahan, In. DNA Cloning. A Practical Approach. Vol. 1, ILR-Press, Cold Spring Harbor, New York, 1989). Die Selektion der Plasmid-tragenden Zellen erfolgte durch Ausplattieren des Tansformationsansatztes auf LB-Agar (Sambrook et al., Molecular Cloning: A Laboratory Manual. 2nd Ed., Cold Spring Harbor, New York, 1989), der mit 25 mg/l Kanamycin supplementiert wurde.

Plasmid-DNA wurde aus einer Transformante mit Hilfe des QIAprep Spin Miniprep Kit der Firma Qiagen isoliert und durch Restriktionsspaltung mit dem Enzym PstI und anschließender Agarosegel-Elektrophorese überprüft. Das Plasmid wird pK18mobsacB_prpD1_P272L genannt und ist in Figur 1 dargestellt.

### 4.2 Allelaustausch

Der in Beispiel 4.1 genannte Vektor pK18mobsacB_prpD1_P272L wurde nach einem Protokoll von Schäfer et al. (Journal of Microbiology 172: 1663-1666 (1990)) in den C. glutamicum Stamm DM416 durch Konjugation transferiert. Der Vektor kann in DM416 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_prpD1_P272L erfolgte durch Ausplattieren des Konjugationsansatzes auf LB-Agar (Sambrook et al., Molecular Cloning: A Laboratory Manual. 2nd Ed. Cold Spring Harbor, New York, 1989), der mit 15 mg/l Kanamycin und 50 mg/l Nalidixinsäure supplementiert wurde. Kanamycin-resistente Transkonjuganten wurden auf LB-Agarplatten mit 25 mg/l Kanamycin ausgestrichen und für 24 Stunden bei 33°C inkubiert. Zur Selektion von Mutanten, bei denen als Folge eines zweiten Rekombinationsereignisses die Exzision des Plasmides stattgefunden hat, wurden die Klone 30 Stunden unselektiv in LB-Flüssigmedium kultiviert, anschließend auf LB-Agar mit 10% Sucrose ausgestrichen und 16 Stunden bebrütet.

Das Plasmid pK18mobsacB_prpD1_P272L enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression führt zur Bildung der Levan-Sucrase, die die Synthese des für C. glutamicum toxischen Produktes Levan katalysiert. Auf LB-Agar mit Sucrose wachsen daher nur solche Klone an, bei denen das integrierte pK18mobsacB_prpD1_P272L als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Ungefähr 40 bis 50 Kolonien wurden auf den Phänotyp "Wachstum in Gegenwart von Sucrose" und "Nicht-Wachstum in Gegenwart von Kanamycin" geprüft. Bei 4 Kolonien, die den Phänotyp "Wachstum in Gegenwart von Sucrose" und "Nicht-Wachstum in Gegenwart von Kanamycin" aufwiesen, wurde ein die Mutation P272L überspannender Bereich des prpD1-Gens, ausgehend von dem Sequenzierprimer pr1-2 (entspricht der Nukleotidsequenz Position 424-443 der Kodierregion des prpD1-Gens aus SEQ ID NO: 1), von der Firma Agowa (Berlin, Deutschland) sequenziert, um nachzuweisen, dass die Mutation des prpD1_P272L-Allels im Chromosom vorliegt. Der verwendete Primer pr1-2 wurde dazu von der Firma Agowa synthetisiert:

pr1-2:

5' ggt atc gcc act gcc tat ga 3'

Auf diese Weise wurde ein Klon identifiziert, der an der Position 815 der Kodierregion des prpD1-Gens die Base Thymin enthält und somit das prpD1_P272L-Allel besitzt. Dieser Klon wird als Stamm DM416prpD1_P272L bezeichnet.

<u>Beispiel 6</u>

Vergleich der Leistung des Stammes DM416prpD1_P272L mit der des Ausgangsstammes DM416

Der Leistungstest des in Beispiel 5 erhaltenen C. glutamicum Stammes DM416prpD1_P272L wurde wie in Beispiel 2 beschrieben durchgeführt: Das Ergebnis des Versuchs ist in Tabelle 2 dargestellt.

Tabelle 2

| Stamm | OD (660 nm) | Lysin-HCl g/l |
|---|---|---|
| DM416 | 6,2 | 1,40 |
| DM416prpD1_P272L | 6,2 | 1,64 |

Kurze Beschreibung der Figur:

Figur 1: Karte des Plasmids pK18mobsacB_prpD1_P272L.

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.

| | |
|---|---|
| Kan: | Kanamycin Resistenz-Gen |
| EcoRI: | Schnittstelle des Restriktionsenzyms EcoRI |
| PstI: | Schnittstelle des Restriktionsenzyms PstI |
| prpD1: | prpD1_P272L-Allel |
| sacB: | sacB-Gen |
| RP4-mob: | mob-Region mit dem Replikationsursprung für den Transfer (oriT) |
| oriV: | Replikationsursprung V |

**Patentansprüche**

1. Isolierte Mutanten coryneformer Bakterien, welche ein Gen enthalten, das für ein Polypeptid mit 2-Methylcitrat-Dehydratase Aktivität kodiert, **dadurch gekennzeichnet,**
**dass** das Polypeptid an der Position 272 der Aminosäuresequenz von SEQ ID NO:2 entsprechenden Position L-Leucin enthält
oder dass das Polypeptid die Aminosäuresequenz von SEQ ID NO: 2 umfasst, wobei an Position 272 L-Leucin enthalten ist.

2. Mutanten coryneformer Bakterien gemäß Anspruch 1,
**dadurch gekennzeichnet, dass** es sich bei den coryneformen Bakterien um ein Bakterium ausgewählt aus der Gruppe Corynebacterium efficiens, Corynebacterium glutamicum, Corynebacterium thermoaminogenes und Corynebacterium aminogenes, bevorzugt
um Corynebacterium glutamicum handelt.

3. Mutanten coryneformer Bakterien gemäß Anspruch 1, oder 2, **dadurch gekennzeichnet, dass** es sich um L-Aminosäure ausscheidende Bakterien, bevorzugt
um L-Lysin, L-Valin, L-Isoleucin, L-Tryptophan oder um L-Homoserin ausscheidende Bakterien handelt.

4. Mutanten coryneformer Bakterien gemäß Anspruch 1 bis 3, **dadurch gekennzeichnet, dass** das Gen eine Nukleotidsequenz umfasst, die mit der Nukleotidsequenz eines Polynukleotids identisch ist, das durch eine Polymerasekettenreaktion (PCR) unter Verwendung von DNA gewonnen aus einem coryneformen Bakterium und unter Verwendung eines Primerpaares bestehend aus einem ersten Primer, umfassend mindestens 15 aufeinanderfolgende Nukleotide ausgewählt aus der Nukleotidsequenz zwischen Position 1 und 750 von SEQ ID NO:3 oder SEQ ID NO:7, und einem zweiten Primer, umfassend mindestens 15 aufeinanderfolgende Nukleotide ausgewählt aus der komplementären Nukleotidsequenz zwischen Position 3000 und 2245 von SEQ ID NO:3 oder SEQ ID NO: 7, erhältlich ist oder,
dass das kodierte Polypeptid eine Aminosäuresequenz mit einer Länge entsprechend 498 L-Aminosäuren umfasst oder,
dass das kodierte Polypeptid von Position 252 bis 291 der Aminosäuresequenz die Aminosäuresequenz entsprechend Position 252 bis 291 von SEQ ID NO:6 enthält oder,
dass das kodierte Polypeptid eine Aminosäuresequenz umfasst, die mindestens 90% identisch ist mit der Aminosäuresequenz von SEQ ID NO:6 und an Position 272 L-Leucin enthält oder,
dass das Gen eine Nukleotidsequenz umfasst, die mindestens 90% identisch ist mit der Nukleotidsequenz von SEQ ID NO:5.

5. Mutanten coryneformer Bakterien gemäß Anspruch 1, **dadurch gekennzeichnet, dass** das kodierte Protein eine Aminosäuresequenz ausgewählt aus der Gruppe

   a) Aminosäuresequenz gemäß SEQ ID NO:6,
   b) Aminosäuresequenz gemäß SEQ ID NO:6 einschließlich maximal 5 konservativer Aminosäureaustausche, und
   c) Aminosäuresequenz gemäß SEQ ID NO:6 einschließlich maximal 5 Insertionen oder Deletionen von Aminosäuren,

   umfasst.

6. Mutanten coryneformer Bakterien gemäß Anspruch 4, **dadurch gekennzeichnet, dass** das Gen die Nukleotidsequenz von SEQ ID NO:5 oder SEQ ID NO:7 umfasst.

7. Ein isoliertes Polynukleotid, das für ein Polypeptid mit 2-Methylcitrat-Dehydratase Enzymaktivität kodiert, welches an der Position 272 der Aminosäuresequenz von SEQ ID NO:2 entsprechenden Position L-Leucin enthält oder welches die Aminosäuresequenz von SEQ ID NO: 2 umfasst, wobei an Position 272 L-Leucin enthalten ist.

8. Isoliertes Polynukleotid gemäß Anspruch 7 , **dadurch gekennzeichnet, dass** das kodierte Polypeptid eine Aminosäuresequenz mit einer Länge von 498 Aminosäuren umfasst oder,
dass das kodierte Polypeptid von Position 252 bis 291 der Aminosäuresequenz die Aminosäuresequenz entsprechend Position 252 bis 291 von SEQ ID NO:6 enthält oder,

dass das isolierte Polynukleotid mit der Nukleotidsequenz eines Polynukleotids identisch ist, das durch eine Polymerasekettenreaktion (PCR) unter Verwendung von DNA gewonnen aus einem coryneformen Bakterium und unter Verwendung eines Primerpaares bestehend aus einem ersten Primer, umfassend mindestens 15 aufeinanderfolgende Nukleotide ausgewählt aus der Nukleotidsequenz zwischen Position 1 und 750 von SEQ ID NO:3 oder SEQ ID NO:7, und einem zweiten Primer, umfassend mindestens 15 aufeinanderfolgende Nukleotide ausgewählt aus der komplementären Nukleotidsequenz zwischen Position 3000 und 2245 von SEQ ID NO:3 oder SEQ ID NO: 7 erhältlich ist oder,
dass das isolierte Polynukleotid mit zu SEQ ID NO:5, komplementären Nukleotidsequenz unter stringenten Bedingungen hybridisiert oder,
dass das kodierte Polypeptid eine Aminosäuresequenz umfasst, die mindestens 90% identisch ist mit der Aminosäuresequenz von SEQ ID NO:6 oder
dass das Polynukleotid eine Nukleotidsequenz umfasst, die mindestens 90%, identisch ist mit der Nukleotidsequenz von SEQ ID NO:5 oder,
dass das kodierte Protein eine Aminosäuresequenz umfasst, ausgewählt aus der Gruppe

a) Aminosäuresequenz gemäß SEQ ID NO:6,
b) Aminosäuresequenz gemäß SEQ ID NO:6, einschließlich maximal 5 konservativer Aminosäureaustausche, und
c) Aminosäuresequenz gemäß SEQ ID NO:6, einschließlich maximal 5 Insertionen oder Deletionen von Aminosäuren.

9. Isoliertes Polynukleotid gemäß Anspruch 7 oder 8 **dadurch gekennzeichnet, dass** das kodierte Polypeptid die Aminosäuresequenz von SEQ ID NO:6 umfasst.

10. Isoliertes Polynukleotid gemäß Anspruch 9, **dadurch gekennzeichnet, dass** es die Nukleotidsequenz SEQ ID NO:5 umfasst.

11. Ein isoliertes Polynukleotid, welches ein Nukleinsäure-Molekül umfasst, das mindestens für ein Leseraster mit einer Aminosäuresequenz entsprechend Position 252 bis 291 von SEQ ID NO:2 kodiert, wobei an der Position entsprechend Position 272 von SEQ ID NO:2 L-Leucin enthalten ist.

12. Isoliertes Polynukleotid gemäß Anspruch 11, **dadurch gekennzeichnet, dass** es mindestens für ein Leseraster mit einer Aminosäuresequenz entsprechend Position 252 bis 291 von SEQ ID NO:6 kodiert oder,
dass die Aminosäuresequenz eine oder mehrere konservative Aminosäureaustausche enthält, wobei die konservativen Aminosäureaustausche nicht die Position 272 betreffen oder,
dass das isolierte Polynukleotid eine oder mehrere stille Mutationen enthält oder,
dass das isolierte Polynukleotid mindestens die Nukleotidsequenz entsprechend Position 1504 bis 1623 von SEQ ID NO:7 umfasst.

13. Ein rekombinanter Mikroorganismus, der das isolierte Polynukleotid gemäß Anspruch 7 bis 12 enthält.

14. Rekombinanter Mikroorganismus gemäß Anspruch 13, **dadurch gekennzeichnet, dass** es sich um ein coryneformes Bakterium oder um ein Bakterium der Gattung Escherichia,bevorzugt
um die Gattung Corynebacterium und besonders bevorzugt
um die Art Corynebacterium glutamicum handelt.

15. Ein Vektor, der das isolierte Polynukleotid gemäß Anspruch 7 bis 12 enthält.

16. Ein rekombinanter Mikroorganismus, der den Vektor gemäß Anspruch 15 enthält.

17. Rekombinanter Mikroorganismus gemäß Anspruch 16, **dadurch gekennzeichnet, dass** es sich um ein coryneformes Bakterium oder um ein Bakterium der Gattung Escherichia, bevorzugt
um die Gattung Corynebacterium und besonders bevorzugt
um die Art Corynebacterium glutamicum handelt.

18. Verfahren zur Herstellung von L-Lysin **dadurch gekennzeichnet, dass** man

a) ein isoliertes coryneformes Bakterium in einem geeignetem Medium fermentiert, wobei das Bakterium mindestens eine Kopie eines für ein Polypeptid mit 2-Methylcitrat-Dehydratase Enzymaktivität kodierendes Gen enthält,
wobei das Polypeptid an der Position 272 der Aminosäuresequenz von SEQ ID NO:2 entsprechenden Position L-Leucin enthält
oder wobei das Polypeptid die Aminosäuresequenz von SEQ ID NO: 2 umfasst, bei der an Position 272 L-Leucin enthalten ist, und
b) die L-Aminosäure in der Fermentationsbrühe oder in den Zellen des Bakteriums anreichert.

19. Verfahren gemäß Anspruch 18, **dadurch gekennzeichnet, dass** es sich bei dem isolierten coryneformen Bakterium um eine Mutante gemäß Anspruch 1 bis 6 handelt oder um ein rekombinantes coryneformes Bakterium handelt, das ein isoliertes Polynukleotid gemäß Anspruch 7 bis 12 enthält oder das unter Verwendung desselben hergestellt wurde oder,
dass man die L-Aminosäure isoliert oder sammelt, bevorzugt
reinigt.

20. Verfahren nach Anspruch 18, **dadurch gekennzeichnet, dass** man L-Lysin gemeinsam mit Bestandteilen aus der Fermentationsbrühe und/oder der Biomasse (> 0 bis 100 %) isoliert oder sammelt oder,
dass man

a) aus der in Schritt b) von Anspruch 18 erhaltenen Fermentationsbrühe die gebildete Biomasse in einer Menge von 0 bis 100 % entfernt, und
b) aus der in Schritt a) erhaltenen Brühe ein im wesentlichen trockenes und geformtes Produkt durch eine Methode ausgewählt aus der Gruppe Granulation, Kompaktierung, Sprühtrocknung und Extrusion herstellt.

21. Verfahren nach Anspruch 20, **dadurch gekennzeichnet, dass** man der Fermentationsbrühe vor oder nach Schritt a) eine Säure ausgewählt aus der Gruppe Schwefelsäure, Salzsäure und Phosphorsäure hinzufügt oder
dass man aus der erhaltenen Brühe vor oder in Schritt a) Wasser entfernt oder
dass man das in oder während Schritt b) erhaltene geformte Produkt mit einem Öl besprüht.

22. Verfahren gemäß Anspruch 18, **dadurch gekennzeichnet, dass** man folgende Schritte durchführt

a) Filtration der Fermentationsbrühe, bevorzugt mit einem Membranfilter, so dass ein Biomassehaltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s)).

## Claims

1. Isolated mutants of coryneform bacteria which comprise a gene which codes for a polypeptide having 2-methylcitrate dehydratase activity, **characterized in that** the polypeptide comprises L-leucine at the position corresponding to position 272 of the amino acid sequence of SEQ ID NO: 2 or in that the polypeptide includes the amino acid sequence of SEQ ID NO: 2, with L-leucine being present at position 272.

2. Mutants of coryneform bacteria according to Claim 1, **characterized in that** the coryneform bacteria take the form of a bacterium selected from the group of Corynebacterium efficiens, Corynebacterium glutamicum, Corynebacterium thermoaminogenes and Corynebacterium aminogenes, preferably take the form of Corynebacterium glutamicum.

3. Mutants of coryneform bacteria according to Claim 1 or 2, **characterized in that** they are L-amino acid-secreting bacteria, preferably bacteria secreting L-lysine, L-valine, L-isoleucine, L-tryptophan or L-homoserine.

4. Mutants of coryneform bacteria according to Claim 1 to 3, **characterized in that** the gene includes a nucleotide sequence which is identical to the nucleotide sequence of a polynucleotide which is obtainable by a polymerase chain reaction (PCR) using DNA obtained from a coryneform bacterium and using a primer pair consisting of a first primer including at least 15 consecutive nucleotides selected from the nucleotide sequence between position 1 and 750 of SEQ ID NO:3 or SEQ ID NO:7, and a second primer including at least 15 consecutive nucleotides selected from the complementary nucleotide sequence between position 3000 and 2245 of SEQ ID NO:3 or SEQ ID NO: 7, or in that the encoded polypeptide includes an amino acid sequence having a length corresponding to 498 L-amino acids, or **in that** the encoded polypeptide comprises from position 252 to 291 of the amino acid sequence the amino acid sequence corresponding to position 252 to 291 of SEQ ID NO:6, or **in that** the encoded polypeptide includes an amino acid sequence which is at least 90% identical to the amino acid sequence of SEQ ID NO:6 and comprises L-leucine at position 272, or in that the gene includes a nucleotide sequence which is at least 90% identical to the nucleotide sequence of SEQ ID NO:5.

**5.** Mutants of coryneform bacteria according to Claim 1, **characterized in that** the encoded protein includes an amino acid sequence selected from the group

a) amino acid sequence according to SEQ ID NO:6,
b) amino acid sequence according to SEQ ID NO:6 including at most 5 conservative amino acid exchanges, and
c) amino acid sequence according to SEQ ID NO:6 including at most 5 insertions or deletions of amino acids.

**6.** Mutants of coryneform bacteria according to Claim 4, **characterized in that** the gene includes the nucleotide sequence of SEQ ID NO:5 or SEQ ID NO:7.

**7.** An isolated polynucleotide which codes for a polypeptidde having 2-methylcitrate dehydratase enzymic activity, which comprises L-leucine at the position corresponding to position 272 of the amino acid sequence of SEQ ID NO: 2 or which includes the amino acid sequence of SEQ ID NO:2, with L-leucine being present at position 272.

**8.** Isolated polynucleotide according to Claim 7, **characterized in that** the encoded polypeptide includes an amino acid sequence having a length of 498 amino acids, or **in that** the encoded polypeptide comprises from position 252 to 291 of the amino acid sequence the amino acid sequence corresponding to position 252 to 291 of SEQ ID NO:6, or **in that** the isolated polynucleotide is identical to the nucleotide sequence of a polynucleotide which is obtainable by a polymerase chain reaction (PCR) using DNA obtained from a coryneform bacterium and using a primer pair consisting of a first primer including at least 15 consecutive nucleotides selected from the nucleotide sequence between position 1 and 750 of SEQ ID NO:3 or SEQ ID NO:7, and a second primer including at least 15 consecutive nucleotides selected from the complementary nucleotide sequence between position 3000 and 2245 of SEQ ID NO:3 or SEQ ID NO: 7, or **in that** the isolated polynucleotide hybridizes with nucleotide sequence complementary to SEQ ID NO:5 under stringent conditions, or **in that** the encoded polypeptide includes an amino acid sequence which is at least 90% identical to the amino acid sequence of SEQ ID NO:6, or **in that** the polynucleotide includes a nucleotide sequence which is at least 90% identical to the nucleotide sequence of SEQ ID NO:5, or **in that** the encoded protein includes an amino acid sequence selected from the group

a) amino acid sequence according to SEQ ID NO:6,
b) amino acid sequence according to SEQ ID NO:6 including at most 5 conservative amino acid exchanges, and
c) amino acid sequence according to SEQ ID NO:6 including at most 5 insertions or deletions of amino acids.

**9.** Isolated polynucleotide according to Claim 7 or 8, **characterized in that** the encoded polypeptide includes the amino acid sequence of SEQ ID NO:6.

**10.** Isolated polynucleotide according to Claim 9, **characterized in that** it includes the nucleotide sequence of SEQ ID NO:5.

**11.** An isolated polynucleotide which includes a nucleic acid molecule which codes at least for a reading frame having an amino acid sequence corresponding to position 252 to 291 of SEQ ID NO:2, where L-leucine is present at the position corresponding to position 272 of SEQ ID NO:2.

**12.** Isolated polynucleotide according to Claim 11, **characterized in that** it codes at least for a reading frame having an amino acid sequence corresponding to position 252 to 291 of SEQ ID NO:6, or **in that** the amino acid sequence comprises one or more conservative amino acid exchanges, where the conservative amino acid exchanges do not affect position 272, or **in that** the isolated polynucleotide comprises one or more silent mutations, or **in that** the isolated polynucleotide includes at least the nucleotide sequence corresponding to position 1504 to 1623 of SEQ ID NO: 7.

**13.** A recombinant microorganism which comprises the isolated polynucleotide according to Claims 7 to 12.

**14.** Recombinant microorganism according to Claim 13, **characterized in that** it is a coryneform bacterium or a bacterium of the genus Escherichia, preferably the genus Corynebacterium and particularly preferably the species Corynebacterium glutamicum.

**15.** A vector which comprises the isolated polynucleotide according to Claims 7 to 12.

**16.** A recombinant microorganism which comprises the vector according to Claim 15.

**17.** Recombinant microorganism according to Claim 16 **characterized in that** it is a Coryneform bacterium or a bacterium of the genus Escherichia, preferably and particularly preferably the species Corynebacterium glutamicum.

**18.** Process for producing L-lysine, **characterized in that**

a) an isolated coryneform bacterium is fermented in a suitable medium, where the bacterium comprises at least one copy of a gene coding for a polypeptide having 2-methylcitrate dehydratase enzymic activity, where the polypeptide comprises L-leucine at the position corresponding to position 272 of the amino acid sequence of SEQ ID NO: 2, and
or where the polypeptide includes the amino acid sequence of SEQ ID NO: 2 in which L-leucine is present at position 272, and
b) the L-amino acid is accumulated in the fermentation broth or in the cells of the bacterium.

**19.** Process according to Claim 18, **characterized in that** the isolated coryneform bacterium is a mutant according to Claim 1 to 6, or is a recombinant coryneform bacterium which comprises an isolated polynucleotide according to Claim 7 to 12 or which has been produced using the same, or **in that** the L-amino acid is isolated or collected, preferably purified.

**20.** Process according to Claim 18, **characterized in that** L-lysine is isolated or collected together with constituents of the fermentation broth and/or of the biomass (> 0 to 100 %), or **in that**

a) the biomass formed is removed in an amount of from 0 to 100% from the fermentation broth obtained in step b) of Claim 18, and
b) a substantially dry and shaped product is produced, by a method selected from the group of granulation, compaction, spray drying and extrusion, from the broth obtained in step a).

**21.** Process according to Claim 20, **characterized in that** an acid selected from the group of sulphuric acid, hydrochloric acid and phosphoric acid is added to the fermentation broth before or after step a), or **in that** water is removed from the resulting broth before or in step a), or **in that** the shaped product obtained in or during step b) is sprayed with an oil.

**22.** Process according to Claim 18, **characterized in that** the following steps are carried out

a) filtration of the fermentation broth, preferably with a membrane filter, to result in a biomass-containing sludge and a filtrate,
b) concentration of the filtrate, preferably so as to result in a solids content of from 48 to 52% by weight,
c) granulation of the concentrate obtained in step b), preferably at a temperature of from 50°C to 62°C, and
d) coating of the granules obtained in c) with one or more of the coating agent(s).

**Revendications**

**1.** Mutants isolés de bactéries corynéformes, qui contiennent un gène qui code pour un polypeptide ayant une activité de 2-méthylcitrate-déshydratase, **caractérisés en ce que** le polypeptide contient une leucine sur la position correspondant à la position 272 de la séquence d'acides aminés de SEQ ID NO:2, ou que le polypeptide comprend la séquence d'acides aminés de SEQ ID NO:2, qui en position 272 contient une L-leucine.

**2.** Mutants de bactéries corynéformes selon la revendication 1, **caractérisés en ce que**, pour ce qui concerne les bactéries corynéformes, il s'agit d'une bactérie choisie dans le groupe consistant en Corynebacterium efficiens, Corynebacterium glutamicum, Corynebacterium thermoaminogenes et Corynebacterium aminogenes, de préférence de Corynebacterium glutamicum.

3. Mutants de bactéries corynéformes selon la revendication 1 ou 2, **caractérisés en ce qu'**il s'agit de bactéries sécrétant un acide L-aminé, de préférence de bactéries sécrétant la L-lysine, la L-valine, la L-isoleucine, le tryptophane ou la L-homosérine.

4. Mutants de bactéries corynéformes selon les revendications 1 à 3, **caractérisés en ce que** le gène comprend une séquence nucléotidique qui est identique à la séquence nucléotidique d'un polypeptide qui peut être obtenu par une réaction en chaîne par polymérase (PCR) par utilisation d'un ADN obtenu à partir d'une bactérie corynéforme et par utilisation d'une paire d'amorces consistant en une première amorce comprenant au moins 15 nucléotides successifs choisis dans la séquence nucléotidique entre les positions 1 et 750 de SEQ ID NO:3 ou SEQ ID NO:7, et en une deuxième amorce, comprenant au moins 15 nucléotides successifs choisis dans la séquence nucléotidique complémentaire entre les positions 3000 et 2245 de SEQ ID NO:3 ou SEQ ID NO:7, ou
que le polypeptide codé comprend une séquence d'acides aminés ayant une longueur correspondant à 498 acides L-aminés, ou
que le polypeptide codé contient, de la position 252 à la position 291 de la séquence d'acides aminés la séquence d'acides aminés correspondant aux positions 252 à 291 de SEQ ID NO:6, ou
que le polypeptide codé comprend une séquence d'acides aminés qui a une identité d'au moins 90 % avec la séquence d'acides aminés de SEQ ID NO:6 et qui en position 272 contient une L-leucine, ou
que le gène comprend une séquence nucléotidique qui a une identité d'au moins 90 % avec la séquence nucléotidique de SEQ ID NO:5.

5. Mutants de bactéries corynéformes selon la revendication 1, **caractérisés en ce que** la protéine codée comprend une séquence d'acides aminés choisie dans le groupe

   a) la séquence d'acides aminés selon SEQ ID NO:6,
   b) la séquence d'acides aminés selon SEQ ID NO:6, y compris au maximum 5 échanges conservatifs d'acides aminés, et
   c) la séquence d'acides aminés selon SEQ ID NO:6, y compris au maximum 5 insertions ou délétions d'acides aminés.

6. Mutants de bactéries corynéformes selon la revendication 4, **caractérisés en ce que** le gène comprend la séquence nucléotidique de SEQ ID NO:5 ou de SEQ ID NO: 7.

7. Polynucléotide isolé qui code pour un polypeptide ayant une activité enzymatique de 2-méthylcitrate-déshydratase, qui contient une L-leucine sur la position correspondant à la position 272 de la séquence d'acides aminés de SEQ ID NO:2, ou qui comprend la séquence d'acides aminés de SEQ ID NO:2, qui en position 272 contient une L-leucine.

8. Polynucléotide isolé selon la revendication 7, **caractérisé en ce que** le polypeptide codé comprend une séquence d'acides aminés ayant une longueur de 498 acides aminés, ou
que le polypeptide codé contient des positions 252 à 291 de la séquence d'acides aminés la séquence d'acides aminés correspondant aux positions 252 à 291 de SEQ ID NO:6, ou
que le polynucléotide isolé est identique à la séquence nucléotidique d'un polynucléotide qui peut être obtenu par une réaction en chaîne par polymérase (PCR) par utilisation d'un ADN obtenu à partir d'une bactérie corynéforme et par utilisation d'une paire d'amorces consistant en une première amorce comprenant au moins 15 nucléotides successifs choisis dans la séquence nucléotidique entre les positions 1 et 750 de SEQ ID NO:3 ou SEQ ID NO:7, et en une deuxième amorce comprenant au moins 15 nucléotides successifs choisis dans la séquence nucléotidique complémentaire entre les positions 3000 et 2245 de SEQ ID NO:3 ou de SEQ ID NO:7, ou
que le polynucléotide isolé s'hybride dans des conditions stringentes à la séquence nucléotidique complémentaire de SEQ ID NO:5, ou
que le polypeptide codé comprend une séquence d'acides aminés qui a une identité d'au moins 90 % avec la séquence d'acides aminés de SEQ ID NO:6, ou
que le polypeptide comprend une séquence nucléotidique qui a une identité d'au moins 90 % avec la séquence nucléotidique de SEQ ID NO:5, ou
que la protéine codée comprend une séquence d'acides aminés choisie dans le groupe

   a) la séquence d'acides aminés selon SEQ ID NO:6,
   b) la séquence d'acides aminés selon SEQ ID NO:6, y compris au maximum 5 échanges conservatifs d'acides aminés, et
   c) la séquence d'acides aminés selon SEQ ID NO:6, y compris au maximum 5 insertions ou délétions d'acides aminés.

9. Polynucléotide isolé selon la revendication 7 ou 8, **caractérisé en ce que** le polypeptide codé comprend la séquence d'acides aminés de SEQ ID NO:6.

10. Polynucléotide isolé selon la revendication 9, **caractérisé en ce qu'**il comprend la séquence nucléotidique SEQ ID NO:5.

11. Polynucléotide isolé qui comprend une molécule d'acide nucléique qui code au moins pour un cadre de lecture ayant une séquence d'acides aminés correspondant aux positions 252 à 291 de SEQ ID NO:2, une L-leucine étant présente sur la position correspondant à la position 272 de SEQ ID NO:2.

12. Polynucléotide isolé selon la revendication 11, **caractérisé en ce qu'**il code au moins pour un cadre de lecture ayant une séquence d'acides aminés correspondant aux positions 252 à 291 de SEQ ID NO:6, ou
que la séquence d'acides aminés contient un ou plusieurs échanges conservatifs d'acides aminés, les échanges conservatifs d'acides aminés ne concernant pas la position 272, ou
que le polynucléotide isolé contient une ou plusieurs mutations silencieuses, ou
que le polynucléotide isolé comprend au moins la séquence nucléotidique correspondant aux positions 1504 à 1623 de SEQ ID NO:7.

13. Microorganisme recombinant qui contient le polynucléotide isolé selon les revendications 7 à 12.

14. Microorganisme recombinant selon la revendication 13, **caractérisé en ce qu'**il s'agit d'une bactérie corynéforme ou d'une bactérie du genre Escherichia, de préférence du genre Corynebacterium et d'une manière particulièrement préférée de l'espèce Corynebacterium glutamicum.

15. Vecteur qui contient le polynucléotide isolé selon les revendications 7 à 12.

16. Microorganisme recombinant, qui contient le vecteur selon la revendication 15.

17. Microorganisme recombinant selon la revendication 16, **caractérisé en ce qu'**il s'agit d'une bactérie corynéforme ou d'une bactérie du genre Escherichia, de préférence du genre Corynebacterium et d'une manière particulièrement préférée de l'espèce Corynebacterium glutamicum.

18. Procédé de préparation de L-lysine, **caractérisé en ce que**

a) on fermente une bactérie corynéforme isolée dans un milieu approprié, la bactérie contenant au moins une copie d'un gène codant pour un polypeptide ayant une activité enzymatique de 2-méthylcitrate-déshydratase, le polypeptide contenant une L-leucine sur la position correspondant à la position 272 de la séquence d'acides aminés de SEQ ID NO:2, ou le polypeptide comprenant la séquence d'acides aminés de SEQ ID NO:2, qui en position 272 contient une L-leucine,
et
b) on enrichit l'acide L-aminé dans le bouillon de fermentation ou dans les cellules de la bactérie.

19. Procédé selon la revendication 18, **caractérisé en ce que**, pour ce qui concerne la bactérie corynéforme isolée, il s'agit d'un mutant selon les revendications 1 à 6, ou d'une bactérie corynéforme recombinante qui contient un polynucléotide isolé selon les revendications 7 à 12 et qui a été préparée par utilisation de ce dernier, ou
qu'on isole ou recueille, de préférence purifie l'acide aminé.

20. Procédé selon la revendication 18, **caractérisé en ce qu'**on isole ou recueille la L-lysine en même temps que les constituants du bouillon de fermentation et/ou de la biomasse (> 0 à 100 %) ou que

a) on élimine du bouillon de fermentation obtenu dans l'étape b) de la revendication 18 la biomasse formée, en une quantité de 0 à 100 %, et
b) à partir du bouillon obtenu dans l'étape a), on prépare un produit pour l'essentiel sec et façonné, par un procédé choisi dans le groupe consistant en une granulation, un compactage, un séchage par atomisation et une extrusion.

**21.** Procédé selon la revendication 20, **caractérisé en ce qu'**on ajoute au bouillon de fermentation, avant ou après l'étape a), un acide choisi dans le groupe consistant en l'acide sulfurique, l'acide chlorhydrique et l'acide phosphorique, ou
qu'on élimine l'eau du bouillon de fermentation avant l'étape a) ou au cours de cette dernière, ou
qu'on pulvérise une huile sur le produit façonné obtenu au cours de l'étape b) ou pendant cette dernière.

**22.** Procédé selon la revendication 18, **caractérisé en ce qu'**on met en oeuvre les étapes suivantes :

a) filtration du bouillon de fermentation, de préférence avec une membrane filtrante, de façon à obtenir une boue contenant une biomasse, et un filtrat,
b) concentration du filtrat, de préférence de façon à arriver à une teneur en extrait sec de 48 à 52 % en poids,
c) granulation du concentré obtenu dans l'étape b), de préférence à une température de 50 à 62°C, et
d) revêtement du granulé obtenu dans l'étape c) avec un ou plusieurs des agents de revêtement (coating agent(s)).

Figur 1: Plasmid pK18mobsacB_prpD1_P272L

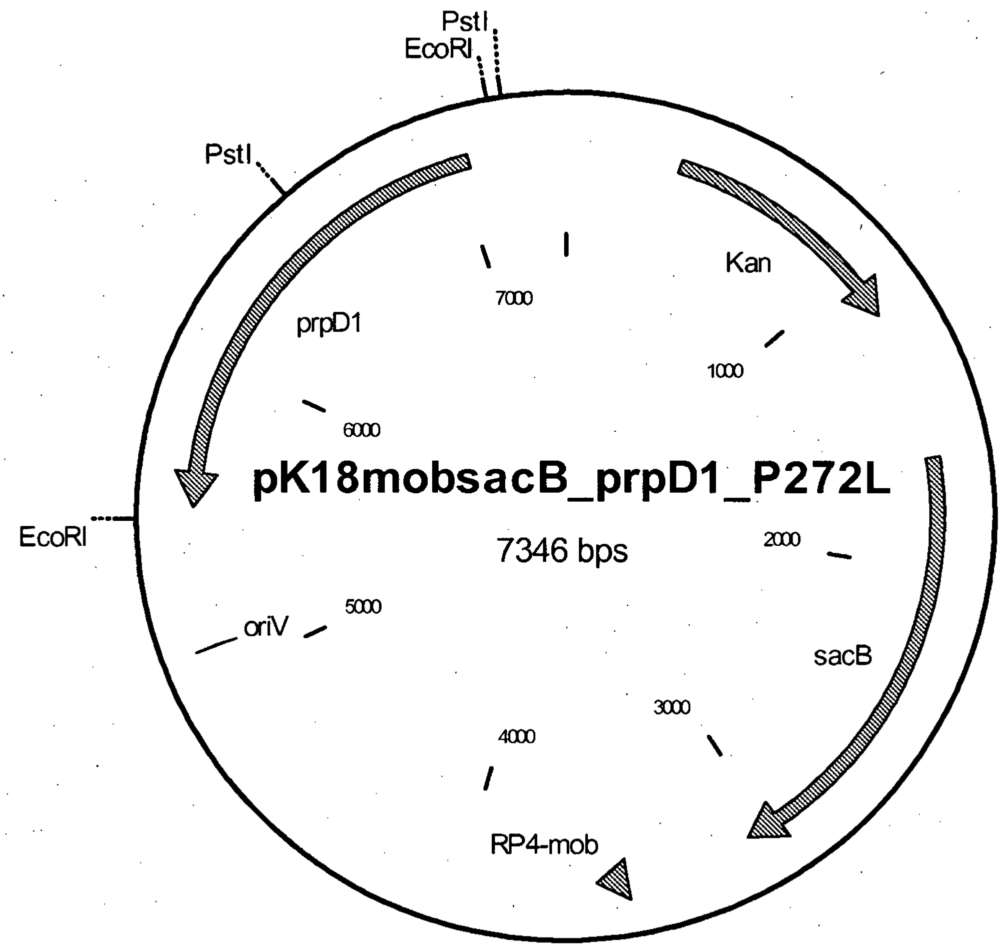

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1108790 A
- EP 0358940 A
- US 4275157 A
- US 5250423 A
- US 5563052 A
- US 5605818 A
- US 5235940 A
- US 5188948 A
- US 5521074 A
- US 4656135 A
- US 5294547 A
- US 3189526 A
- US 5250434 A
- US 6221636 B
- US 5840551 A
- US 5770409 A
- US 5275940 A
- US 4224409 A
- US 5688671 A
- JP 6261766 A
- DE 102006026328
- US 6844176 B
- WO 00632728 A
- US 6893848 B
- WO 0149854 A
- EP 0435132 A
- WO 06063660 A
- US 3708395 A
- US 6180373 B
- EP 0338474 A
- WO 05003357 A
- US 6107063 A
- WO 02070685 A
- WO 03014362 A
- US 63032723 A, Nakamura
- EP 0197335 A
- US 6090597 A
- JP 09224661 A
- US 20030175911 A1
- DE 19831609 A
- WO 0231158 A
- EP 1325135 B1
- WO 0100843 A
- DE 19548222 A
- US 6004773 A
- US 6632644 B
- WO 03040373 A
- US 20030219881 A1
- WO 03014330 A
- US 20040043458 A1
- US 5804414 A
- US 5591577 A
- EP 06007373 A
- US 6586214 B
- US 6465238 B
- EP 0131171 A
- WO 0100844 A
- WO 0202778 A
- US 7094106 B
- EP 2005057216 W
- WO 0226787 A
- EP 2004008882 W
- EP 0534865 B
- US 6340486 B
- US 6465025 B
- EP 0533039 B
- GB 1439728 A
- EP 1331220 A
- EP 2004006655 W
- DE 102006016158
- EP 0743016 A
- WO 04054381 A
- DE 4100920 C
- US 20050220933 A
- US 20030152633 A


### In der Beschreibung aufgeführte Nicht-Patentliteratur


- **KALINOWSKI et al.** *Journal of Biotechnology,* 2003, vol. 104, 5-25
- **NISHIO et al.** *Genome Res.,* 2003, vol. 13 (7), 1572-1579
- Microbial Production of L-Amino Acids. **MUELLER ; HUEBNER.** Advances in Biochemical Engineering. Springer Verlag, 2003, 79
- A New Era in Corynebacterium glutamicum Biotechnology. des Journal of Biotechnology. 2003, vol. 104
- Handbook of Corynebacterium glutamicum. CRC Press, Taylor & Francis Group, 2005
- **CLAES et al.** *Journal of Bacteriology,* 2002, vol. 184 (10), 2728-2739

- **MENKEL et al.** *Applied and Environmental Microbiology,* 1989, vol. 55 (3), 684-688
- **SEILER.** *Journal of General Microbiology,* 1983, vol. 129, 1433-1477
- Glutamic Acid Bacteria. **KINOSHITA.** Biology of Industrial Microorganisms. The Benjamin/Cummins Publishing Co, 1983, 115-142
- **KÄMPFER ; KROPPENSTEDT.** *Canadian Journal of Microbiology,* 1996, vol. 42, 989-1005
- **LIEBL et al.** *International Journal of Systematic Bacteriology,* 1991, vol. 41, 255-260
- **THOMPSON et al.** *Nucleic Acids Research,* 1994, vol. 22, 4637-4680
- **HORSWILL ; ESCALANTE-SEMERENA.** *Biochemistry,* 2001, vol. 40 (15), 4703-4713
- Manual of Methods for General Bacteriology. American Society for Microbiology, 1981
- **TOSAKA et al.** *Agricultural and Biological Chemistry,* 1978, vol. 42 (4), 745-752
- **KONICEK et al.** *Folia Microbiologica,* 1988, vol. 33, 337-343
- **ZIMMERMANN et al.** VDI Berichte Nr. 1841. VDI-Verlag, 2004, 439-443
- **ZIMMERMANN.** *Chemie Ingenenieur Technik,* 2005, vol. 77 (4), 426-428
- **LOTTSPEICH ; ZORBAS.** Bioanalytik. Spektrum Akademischer Verlag, 1998
- **DIEFFENBACH ; DVEKSLER.** PCR Primer - A Laboratory Manual. Cold Spring Harbor Laboratory Press, 1995
- **RYCHLIK et al.** *Nucleic Acids Research,* vol. 18 (21), 6409-6412
- **INNIS ; FELFAND ; SNINSKY.** PCR-Strategies. Academic Press , Inc, 1995
- **DIEFENBACH ; DVEKSLER.** PCR Primer - a laboratory manual. Cold Spring Harbor Laboratory Press, 1995
- **GAIT.** Oligonukleotide synthesis: A Practical Approach. IRL Press, 1984
- **NEWTON ; GRAHAM.** PCR. Spektrum Akademischer Verlag, 1994
- **SANGER et al.** *Proceedings of the National Academies of Sciences, U.S.A.,* 1977, vol. 74, 5463-5467
- **ZIMMERMANN et al.** *Nucleic Acids Research,* 1990, vol. 18, 1067
- **GASTEIGER et al.** *Nucleic Acids Research,* 2003, vol. 31, 3784-3788
- **MARGULIES et al.** *Nature,* 2005, vol. 437 (7057), 376-2720
- **VELICER et al.** *Proceedings of the National Academy of Sciences, U.S.A.,* 2006, vol. 103 (21), 8107-8112
- **KALINOWSKI et al.** *Molecular and General Genetics,* 1990, vol. 224, 317-324
- **JETTEN et al.** *Applied Microbiology and Biotechnology,* 1995, vol. 43, 76-82
- **MATSUI et al.** *Journal of Bacteriology,* 1987, vol. 169 (11), 5330-5332
- **KEILHAUER et al.** *Journal of Bacteriology,* 1993, vol. 175 (17), 5595-5603
- **ELISAKOVA et al.** *Applied and Environmental Microbiology,* 2005, vol. 71 (1), 207-13
- **MORBACH et al.** *Applied and Environmental Microbiology,* 1995, vol. 61 (12), 4315-4320
- **GAIT.** Oligonucleotide Synthesis: A Practical Approach. IRL Press, 1984
- A Short Course in Bacterial Genetics. **MILLER, J. H.** A Laboratory Manual and Handbook for Escherichia coli and Related Bacteria. Cold Spring Harbor Laboratory Press, 1992
- **T. A. BROWN.** Gentechnologie für Einsteiger. Spektrum Akademischer Verlag, 1993
- **R. M. HORTON.** *PCR-Mediated Recombination and Mutagenesis, Molecular Biotechnology,* 1995, vol. 3, 93-99
- **KNIPPERS.** Molekulare Genetik. Georg Thieme Verlag, 1995
- **WINNACKER.** Gene und Klone. VCH Verlagsgesellschaft, 1990
- **HAGEMANN.** Allgemeine Genetik. Gustav Fischer Verlag, 1986
- The DIG System Users Guide for Filter Hybridization. 1993
- Hybaid Hybridisation Guide. Hybaid Limited, 1996
- *The DIG System User's Guide for Filter Hybridisation,* 1995
- **SCHWARZER ; PÜHLER.** *Bio/Technology,* 1991, vol. 9, 84-87
- **SCHÄFER et al.** *Gene,* 1994, vol. 145, 69-73
- **OHNISHI et al.** *Applied Microbiology and Biotechnology,* 2002, vol. 58 (2), 217-223
- **SCHÄFER.** *Journal of Bacteriology,* 1990, vol. 172, 1663-1666
- **DUNICAN ; SHIVNAN.** *Bio/Technology,* 1989, vol. 7, 1067-1070
- **THIERBACH et al.** *Applied Microbiology and Biotechnology,* 1988, vol. 29, 356-362
- **LAY et al.** *Clinical Chemistry,* 1997, vol. 43, 2262-2267
- **KALINOWSKI et al.** *Journal of Biotechnology,* 2003, vol. 104 (1-3), 5-25
- **MARIENHAGEN et al.** *Journal of Bacteriology,* 2005, vol. 187 (22), 7693-7646
- **MENKEL et al.** *Applied and Environmental Microbiology,* 1989, vol. 64, 549-554
- **TAUCH et al.** *Journal of Biotechnology,* 2002, vol. 99, 79-91
- **TAUCH et al.** *Journal of Biotechnology,* 2003, vol. 104, 27-40
- **REINSCHEID et al.** *Applied and Environmental Microbiology,* 1994, vol. 60, 126-132

- **PATEK et al.** *Journal of Biotechnology,* 2003, vol. 104 (1-3), 311-323
- **VASICOVA et al.** *Journal of Bacteriology,* 1999, vol. 181, 6188-6191
- **AMANN et al.** *Gene,* 1988, vol. 69 (2), 301-315
- **AMANN ; BROSIUS.** *Gene,* 1985, vol. 40 (2-3), 183-190
- **HERMANN et al.** *Electrophoresis,* 2001, vol. 22, 1712-23
- **SAMBROOK et al.** Molecular cloning: a laboratory manual. Cold Spring Harbor Laboratory Press, 1989
- **LOHAUS ; MEYER.** *Biospektrum,* 1998, vol. 5, 32-39
- **LOTTSPEICH.** *Angewandte Chemie,* 1999, vol. 272, 2630-2647
- **PEOPLES et al.** *Molecular Microbiology,* 1988, vol. 2, 63-72
- **WENDISCH.** *Ph. D. thesis,* 1997, ISSN 0994-2952
- **SIMIC et al.** *Applied and Environmental Microbiology,* 2002, vol. 68, 3321-3327
- **SRIVASTAVA et al.** *Applied Environmental Microbiology,* Oktober 2000, vol. 66 (10), 4366-4371
- **CHMIEL.** Bioprozesstechnik 1. Einführung in die Bioverfahrenstechnik. Gustav Fischer Verlag, 1991
- **STORHAS.** Bioreaktoren und periphere Einrichtungen. Vieweg Verlag, 1994
- Manual of Methods for General Bacteriology. American Society for Bacteriology, 1981
- **SPACKMAN et al.** *Analytical Chemistry,* 1958, vol. 30, 1190
- **LINDROTH et al.** *Analytical Chemistry,* 1979, vol. 51, 1167-1174
- *Klein: Seifen, Öle, Fette, Wachse,* 1968, vol. 94, 12
- **R. H. MÜLLER ; R. SCHUHMANN.** Teilchengrößenmessung in der Laborpraxis. Wissenschaftliche Verlagsgesellschaft, 1996
- **M. RHODES.** Introduction to Particle Technology. Verlag Wiley & Sons, 1998
- *Die Mühle + Mischfuttertechnik,* 1995, vol. 132 (49), 817
- **EIKMANNS et al.** *Microbiology,* 1994, vol. 140, 1817-1828
- **INNIS et al.** PCR Protocols. A Guide to Methods and Applications. Academic Press, 1990
- **SCHÄFER et al.** *Gene,* 1994, vol. 14, 69-73
- **SIMON et al.** *Bio/Technology,* 1993, vol. 1, 784-791
- **HANAHAN.** DNA Cloning. A Practical Approach. ILR-Press, 1989, vol. 1
- **SAMBROOK et al.** Molecular Cloning: A Laboratory Manual. Cold Spring Harbor, 1989
- **SCHÄFER et al.** *Journal of Microbiology,* 1990, vol. 172, 1663-1666

SEQUENCE LISTING

<110> Evonik Degussa GmbH

<120> Allele des prpD1-Gens aus coryneformen Bakterien

<130> 2006E00233

<160> 20

<170> PatentIn version 3.3

<210> 1
<211> 1497
<212> DNA
<213> Corynebacterium glutamicum

<220>
<221> CDS
<222> (1)..(1494)
<223> prpD1-Wildtyp-Gen

<220>
<221> misc_feature
<222> (815)..(815)
<223> cytosin

<400> 1

```
atg cgc atc cac gat gtt tat acc cac ctt tcg gcc gat aac ttt ccc      48
Met Arg Ile His Asp Val Tyr Thr His Leu Ser Ala Asp Asn Phe Pro
1               5                   10                  15

aaa gca gag cac ctt gcg tgg aaa ttc tcc gag ctt gcc acc gac ccc      96
Lys Ala Glu His Leu Ala Trp Lys Phe Ser Glu Leu Ala Thr Asp Pro
            20                  25                  30

gtg gag gtg aca ccg gat gtt tcg gag atg atc atc aac cgg atc atc     144
Val Glu Val Thr Pro Asp Val Ser Glu Met Ile Ile Asn Arg Ile Ile
        35                  40                  45

gac aac gcg gcg gtg tct gcc gcg tcg gtg ttg cgc cgg cct gtg act     192
Asp Asn Ala Ala Val Ser Ala Ala Ser Val Leu Arg Arg Pro Val Thr
    50                  55                  60

gtg gcc agg caa caa gcg cag tcc cat ccg cgg gaa aag ggc gga aaa     240
Val Ala Arg Gln Gln Ala Gln Ser His Pro Arg Glu Lys Gly Gly Lys
65                  70                  75                  80

gtt ttt gga att tca ggc agc tac tca cca gag tgg gct gcc ttt gct     288
Val Phe Gly Ile Ser Gly Ser Tyr Ser Pro Glu Trp Ala Ala Phe Ala
                85                  90                  95

aat ggt gtg gcc gta cgt gaa ttg gac ttc cac gat aca ttt tta gca     336
Asn Gly Val Ala Val Arg Glu Leu Asp Phe His Asp Thr Phe Leu Ala
            100                 105                 110

gct gaa tac tcc cat ccc ggc gac aat att cca cca ctt ctt gca gta     384
Ala Glu Tyr Ser His Pro Gly Asp Asn Ile Pro Pro Leu Leu Ala Val
        115                 120                 125

gcg cag gct cag aga agc agc ggc agg gat ctc att cgg ggt atc gcc     432
Ala Gln Ala Gln Arg Ser Ser Gly Arg Asp Leu Ile Arg Gly Ile Ala
    130                 135                 140

act gcc tat gag gtg cag gtg gaa ttg gtg agg ggg atc tgc ttg cat     480
Thr Ala Tyr Glu Val Gln Val Glu Leu Val Arg Gly Ile Cys Leu His
145                 150                 155                 160
```

```
gag cac aaa att gat cac gta gcc cac ctt ggt ccc agc gct gca gcg      528
Glu His Lys Ile Asp His Val Ala His Leu Gly Pro Ser Ala Ala Ala
                165                 170                 175

ggt ttg ggc acg ttg ttg cat gta gat gag gaa acc atc tac cag gcg      576
Gly Leu Gly Thr Leu Leu His Val Asp Glu Glu Thr Ile Tyr Gln Ala
            180                 185                 190

atc ggc cag gca ttg cac acc acg acg gct acg agg cag tcg cga aaa      624
Ile Gly Gln Ala Leu His Thr Thr Thr Ala Thr Arg Gln Ser Arg Lys
        195                 200                 205

ggt gag att tcc agc tgg aag gcg ttc gcg cca gcg ttt gcg gga aag      672
Gly Glu Ile Ser Ser Trp Lys Ala Phe Ala Pro Ala Phe Ala Gly Lys
    210                 215                 220

atg gcc att gag gcg atg gat cgt gcg atg cgt ggg gag ggt tcg ccc      720
Met Ala Ile Glu Ala Met Asp Arg Ala Met Arg Gly Glu Gly Ser Pro
225                 230                 235                 240

gca ccg att tgg gag ggc gaa gac ggg gtc atc gcg tgg ctg tta tcg      768
Ala Pro Ile Trp Glu Gly Glu Asp Gly Val Ile Ala Trp Leu Leu Ser
                245                 250                 255

ggc aaa gat cat gtt tat cat gtg cca ttg ccg gaa cac ggc gag ccc      816
Gly Lys Asp His Val Tyr His Val Pro Leu Pro Glu His Gly Glu Pro
            260                 265                 270

aag ctg ggg att cta gag act tac aca aag gaa cat tca gcg gaa tat      864
Lys Leu Gly Ile Leu Glu Thr Tyr Thr Lys Glu His Ser Ala Glu Tyr
        275                 280                 285

caa tcg cag gca ccg att gat ctg gcg cgc agg atg aag cca ctg gtt      912
Gln Ser Gln Ala Pro Ile Asp Leu Ala Arg Arg Met Lys Pro Leu Val
    290                 295                 300

gac gcg gct ggc gga acg gaa cac att gca gag att gtg ctg cgc acc      960
Asp Ala Ala Gly Gly Thr Glu His Ile Ala Glu Ile Val Leu Arg Thr
305                 310                 315                 320

agt cac cac acg cat tat gtg att ggc act ggg gcg aac gat ccg cag     1008
Ser His His Thr His Tyr Val Ile Gly Thr Gly Ala Asn Asp Pro Gln
                325                 330                 335

aag atg gat ccg cag gcc tcg cgt gaa acc ctg gat cat tcc atc atg     1056
Lys Met Asp Pro Gln Ala Ser Arg Glu Thr Leu Asp His Ser Ile Met
            340                 345                 350

tac att ttc gcc gtc gcg ctt caa gat ggc gtg tgg cac cac gag ttt     1104
Tyr Ile Phe Ala Val Ala Leu Gln Asp Gly Val Trp His His Glu Phe
        355                 360                 365

tcc tac acc cgc aag cgt tcc acc cgc ccg gaa act gtg gag ctg tgg     1152
Ser Tyr Thr Arg Lys Arg Ser Thr Arg Pro Glu Thr Val Glu Leu Trp
    370                 375                 380

cac aag att cgc acc gtg gag gat cct gaa tgg acg cgc cga tac cat     1200
His Lys Ile Arg Thr Val Glu Asp Pro Glu Trp Thr Arg Arg Tyr His
385                 390                 395                 400

tct gat gat cct gca aaa aag gcc ttt ggt gcg aaa gca gtg atc aca     1248
Ser Asp Asp Pro Ala Lys Lys Ala Phe Gly Ala Lys Ala Val Ile Thr
                405                 410                 415

atg gct gat ggc acc gtg att gag gat gaa ttg gct gtc gcg gat gcc     1296
Met Ala Asp Gly Thr Val Ile Glu Asp Glu Leu Ala Val Ala Asp Ala
            420                 425                 430

cac ccg ctg ggt gct cgg ccg ttt gcg cgg gag aat tac att gaa aaa     1344
```

```
His Pro Leu Gly Ala Arg Pro Phe Ala Arg Glu Asn Tyr Ile Glu Lys
        435                 440                 445

ttc cgc aca ctc gcg cag ggg att gtc att gat tca gaa cag gaa cgc      1392
Phe Arg Thr Leu Ala Gln Gly Ile Val Ile Asp Ser Glu Gln Glu Arg
    450                 455                 460

ttc ttg cat gcc gtg caa agc ctg cct gac ctg gat gat ctt gat cag      1440
Phe Leu His Ala Val Gln Ser Leu Pro Asp Leu Asp Asp Leu Asp Gln
465                 470                 475                 480

ctc aac atc gaa gtc gac ata agc aac cag gcc gcg acg aaa gcg ggg      1488
Leu Asn Ile Glu Val Asp Ile Ser Asn Gln Ala Ala Thr Lys Ala Gly
                485                 490                 495

ctg tta tga                                                          1497
Leu Leu
```

<210> 2
<211> 498
<212> PRT
<213> Corynebacterium glutamicum

<400> 2

Met Arg Ile His Asp Val Tyr Thr His Leu Ser Ala Asp Asn Phe Pro
1 5 10 15

Lys Ala Glu His Leu Ala Trp Lys Phe Ser Glu Leu Ala Thr Asp Pro
20 25 30

Val Glu Val Thr Pro Asp Val Ser Glu Met Ile Ile Asn Arg Ile Ile
35 40 45

Asp Asn Ala Ala Val Ser Ala Ala Ser Val Leu Arg Arg Pro Val Thr
50 55 60

Val Ala Arg Gln Gln Ala Gln Ser His Pro Arg Glu Lys Gly Gly Lys
65 70 75 80

Val Phe Gly Ile Ser Gly Ser Tyr Ser Pro Glu Trp Ala Ala Phe Ala
85 90 95

Asn Gly Val Ala Val Arg Glu Leu Asp Phe His Asp Thr Phe Leu Ala
100 105 110

Ala Glu Tyr Ser His Pro Gly Asp Asn Ile Pro Pro Leu Leu Ala Val
115 120 125

Ala Gln Ala Gln Arg Ser Ser Gly Arg Asp Leu Ile Arg Gly Ile Ala
130 135 140

Thr Ala Tyr Glu Val Gln Val Glu Leu Val Arg Gly Ile Cys Leu His
145 150 155 160

Glu His Lys Ile Asp His Val Ala His Leu Gly Pro Ser Ala Ala Ala
165 170 175

```
Gly Leu Gly Thr Leu Leu His Val Asp Glu Glu Thr Ile Tyr Gln Ala
            180                 185                 190

Ile Gly Gln Ala Leu His Thr Thr Thr Ala Thr Arg Gln Ser Arg Lys
        195                 200                 205

Gly Glu Ile Ser Ser Trp Lys Ala Phe Ala Pro Ala Phe Ala Gly Lys
    210                 215                 220

Met Ala Ile Glu Ala Met Asp Arg Ala Met Arg Gly Glu Gly Ser Pro
225                 230                 235                 240

Ala Pro Ile Trp Glu Gly Glu Asp Gly Val Ile Ala Trp Leu Leu Ser
                245                 250                 255

Gly Lys Asp His Val Tyr His Val Pro Leu Pro Glu His Gly Glu Pro
            260                 265                 270

Lys Leu Gly Ile Leu Glu Thr Tyr Thr Lys Glu His Ser Ala Glu Tyr
        275                 280                 285

Gln Ser Gln Ala Pro Ile Asp Leu Ala Arg Arg Met Lys Pro Leu Val
    290                 295                 300

Asp Ala Ala Gly Gly Thr Glu His Ile Ala Glu Ile Val Leu Arg Thr
305                 310                 315                 320

Ser His His Thr His Tyr Val Ile Gly Thr Gly Ala Asn Asp Pro Gln
                325                 330                 335

Lys Met Asp Pro Gln Ala Ser Arg Glu Thr Leu Asp His Ser Ile Met
            340                 345                 350

Tyr Ile Phe Ala Val Ala Leu Gln Asp Gly Val Trp His His Glu Phe
        355                 360                 365

Ser Tyr Thr Arg Lys Arg Ser Thr Arg Pro Glu Thr Val Glu Leu Trp
    370                 375                 380

His Lys Ile Arg Thr Val Glu Asp Pro Glu Trp Thr Arg Arg Tyr His
385                 390                 395                 400

Ser Asp Asp Pro Ala Lys Lys Ala Phe Gly Ala Lys Ala Val Ile Thr
                405                 410                 415

Met Ala Asp Gly Thr Val Ile Glu Asp Glu Leu Ala Val Ala Asp Ala
            420                 425                 430

His Pro Leu Gly Ala Arg Pro Phe Ala Arg Glu Asn Tyr Ile Glu Lys
        435                 440                 445
```

Phe Arg Thr Leu Ala Gln Gly Ile Val Ile Asp Ser Glu Gln Glu Arg
450 455 460

Phe Leu His Ala Val Gln Ser Leu Pro Asp Leu Asp Asp Leu Asp Gln
465 470 475 480

Leu Asn Ile Glu Val Asp Ile Ser Asn Gln Ala Ala Thr Lys Ala Gly
485 490 495

Leu Leu

<210> 3
<211> 3000
<212> DNA
<213> Corynebacterium glutamicum

<220>
<221> misc_feature
<222> (1)..(750)
<223> stromaufwärts der CDS gelegene Sequenz

<220>
<221> CDS
<222> (751)..(2244)
<223> prpD1-Wildtyp-Gen

<220>
<221> misc_feature
<222> (1565)..(1565)
<223> Cytosin

<220>
<221> misc_feature
<222> (2245)..(3000)
<223> stromabwärts der CDS gelegene Sequenz

<400> 3

```
ggttccacct tcgccataac gcttgacgac gcctccctcc ttcactctcg gcgcatcatt    60
ttggcccacg gcgccgttga cgatctgcca gaggtagaag gactgtcaga tttttgggga   120
accaaagtgt tgcactgcgc ttactgccac ggctttgagg cccgcgattc tgaaatcgtc   180
gtggtgggta cctcgcccat ggctgcgcac caagcgttga tgttctcgca gttgtccaaa   240
actgtcagct tggtgggcac gatcgacatt gatgaacaaa ccagcgagag cctagatagt   300
gctggagtaa aagtgttggg caccaatgcg gtgcgcgtat ccgccgaagg tgatggcctg   360
tctgtggaac tgtccgaagg cgatcattta agctgcgaca acatcgtggt ggcatctcgt   420
ccactggtgg atggcacgct gtacacccaa cttggtggtc agatggaaga aaacccgatg   480
ggcaggttca ttccaggtac ccaaaccggg cgcactccta ttgaaggtgt gtgggctgcc   540
ggaaacgcgc aagctcccat ggcgatggtc tatggttccg ctgctcaagg cgtgatggct   600
ggagcagaga tcaactttga tctgatcctg gaagatattt ccgtggcaag cgcgcagagc   660
taaactgcgt gaggttgtgg cctgtcacac ataatcggcc tagggtggga ctttaaggaa   720
acagtgcaca aataaatctc aaggagcccc atg cgc atc cac gat gtt tat acc     774
```

```
                                        Met Arg Ile His Asp Val Tyr Thr
                                        1               5

cac ctt tcg gcc gat aac ttt ccc aaa gca gag cac ctt gcg tgg aaa      822
His Leu Ser Ala Asp Asn Phe Pro Lys Ala Glu His Leu Ala Trp Lys
    10                  15                  20

ttc tcc gag ctt gcc acc gac ccc gtg gag gtg aca ccg gat gtt tcg      870
Phe Ser Glu Leu Ala Thr Asp Pro Val Glu Val Thr Pro Asp Val Ser
25                  30                  35                  40

gag atg atc atc aac cgg atc atc gac aac gcg gcg gtg tct gcc gcg      918
Glu Met Ile Ile Asn Arg Ile Ile Asp Asn Ala Ala Val Ser Ala Ala
                45                  50                  55

tcg gtg ttg cgc cgg cct gtg act gtg gcc agg caa caa gcg cag tcc      966
Ser Val Leu Arg Arg Pro Val Thr Val Ala Arg Gln Gln Ala Gln Ser
            60                  65                  70

cat ccg cgg gaa aag ggc gga aaa gtt ttt gga att tca ggc agc tac     1014
His Pro Arg Glu Lys Gly Gly Lys Val Phe Gly Ile Ser Gly Ser Tyr
        75                  80                  85

tca cca gag tgg gct gcc ttt gct aat ggt gtg gcc gta cgt gaa ttg     1062
Ser Pro Glu Trp Ala Ala Phe Ala Asn Gly Val Ala Val Arg Glu Leu
    90                  95                  100

gac ttc cac gat aca ttt tta gca gct gaa tac tcc cat ccc ggc gac     1110
Asp Phe His Asp Thr Phe Leu Ala Ala Glu Tyr Ser His Pro Gly Asp
105                 110                 115                 120

aat att cca cca ctt ctt gca gta gcg cag gct cag aga agc agc ggc     1158
Asn Ile Pro Pro Leu Leu Ala Val Ala Gln Ala Gln Arg Ser Ser Gly
                125                 130                 135

agg gat ctc att cgg ggt atc gcc act gcc tat gag gtg cag gtg gaa     1206
Arg Asp Leu Ile Arg Gly Ile Ala Thr Ala Tyr Glu Val Gln Val Glu
            140                 145                 150

ttg gtg agg ggg atc tgc ttg cat gag cac aaa att gat cac gta gcc     1254
Leu Val Arg Gly Ile Cys Leu His Glu His Lys Ile Asp His Val Ala
        155                 160                 165

cac ctt ggt ccc agc gct gca gcg ggt ttg ggc acg ttg ttg cat gta     1302
His Leu Gly Pro Ser Ala Ala Ala Gly Leu Gly Thr Leu Leu His Val
    170                 175                 180

gat gag gaa acc atc tac cag gcg atc ggc cag gca ttg cac acc acg     1350
Asp Glu Glu Thr Ile Tyr Gln Ala Ile Gly Gln Ala Leu His Thr Thr
185                 190                 195                 200

acg gct acg agg cag tcg cga aaa ggt gag att tcc agc tgg aag gcg     1398
Thr Ala Thr Arg Gln Ser Arg Lys Gly Glu Ile Ser Ser Trp Lys Ala
                205                 210                 215

ttc gcg cca gcg ttt gcg gga aag atg gcc att gag gcg atg gat cgt     1446
Phe Ala Pro Ala Phe Ala Gly Lys Met Ala Ile Glu Ala Met Asp Arg
            220                 225                 230

gcg atg cgt ggg gag ggt tcg ccc gca ccg att tgg gag ggc gaa gac     1494
Ala Met Arg Gly Glu Gly Ser Pro Ala Pro Ile Trp Glu Gly Glu Asp
        235                 240                 245

ggg gtc atc gcg tgg ctg tta tcg ggc aaa gat cat gtt tat cat gtg     1542
Gly Val Ile Ala Trp Leu Leu Ser Gly Lys Asp His Val Tyr His Val
    250                 255                 260

cca ttg ccg gaa cac ggc gag ccc aag ctg ggg att cta gag act tac     1590
Pro Leu Pro Glu His Gly Glu Pro Lys Leu Gly Ile Leu Glu Thr Tyr
```

```
265                 270                 275                 280

aca aag gaa cat tca gcg gaa tat caa tcg cag gca ccg att gat ctg     1638
Thr Lys Glu His Ser Ala Glu Tyr Gln Ser Gln Ala Pro Ile Asp Leu
                285                 290                 295

gcg cgc agg atg aag cca ctg gtt gac gcg gct ggc gga acg gaa cac     1686
Ala Arg Arg Met Lys Pro Leu Val Asp Ala Ala Gly Gly Thr Glu His
            300                 305                 310

att gca gag att gtg ctg cgc acc agt cac cac acg cat tat gtg att     1734
Ile Ala Glu Ile Val Leu Arg Thr Ser His His Thr His Tyr Val Ile
        315                 320                 325

ggc act ggg gcg aac gat ccg cag aag atg gat ccg cag gcc tcg cgt     1782
Gly Thr Gly Ala Asn Asp Pro Gln Lys Met Asp Pro Gln Ala Ser Arg
    330                 335                 340

gaa acc ctg gat cat tcc atc atg tac att ttc gcc gtc gcg ctt caa     1830
Glu Thr Leu Asp His Ser Ile Met Tyr Ile Phe Ala Val Ala Leu Gln
345                 350                 355                 360

gat ggc gtg tgg cac cac gag ttt tcc tac acc cgc aag cgt tcc acc     1878
Asp Gly Val Trp His His Glu Phe Ser Tyr Thr Arg Lys Arg Ser Thr
                365                 370                 375

cgc ccg gaa act gtg gag ctg tgg cac aag att cgc acc gtg gag gat     1926
Arg Pro Glu Thr Val Glu Leu Trp His Lys Ile Arg Thr Val Glu Asp
            380                 385                 390

cct gaa tgg acg cgc cga tac cat tct gat gat cct gca aaa aag gcc     1974
Pro Glu Trp Thr Arg Arg Tyr His Ser Asp Asp Pro Ala Lys Lys Ala
        395                 400                 405

ttt ggt gcg aaa gca gtg atc aca atg gct gat ggc acc gtg att gag     2022
Phe Gly Ala Lys Ala Val Ile Thr Met Ala Asp Gly Thr Val Ile Glu
    410                 415                 420

gat gaa ttg gct gtc gcg gat gcc cac ccg ctg ggt gct cgg ccg ttt     2070
Asp Glu Leu Ala Val Ala Asp Ala His Pro Leu Gly Ala Arg Pro Phe
425                 430                 435                 440

gcg cgg gag aat tac att gaa aaa ttc cgc aca ctc gcg cag ggg att     2118
Ala Arg Glu Asn Tyr Ile Glu Lys Phe Arg Thr Leu Ala Gln Gly Ile
                445                 450                 455

gtc att gat tca gaa cag gaa cgc ttc ttg cat gcc gtg caa agc ctg     2166
Val Ile Asp Ser Glu Gln Glu Arg Phe Leu His Ala Val Gln Ser Leu
            460                 465                 470

cct gac ctg gat gat ctt gat cag ctc aac atc gaa gtc gac ata agc     2214
Pro Asp Leu Asp Asp Leu Asp Gln Leu Asn Ile Glu Val Asp Ile Ser
        475                 480                 485

aac cag gcc gcg acg aaa gcg ggg ctg tta tgaatctctt ttcgaatggt       2264
Asn Gln Ala Ala Thr Lys Ala Gly Leu Leu
    490                 495

gttgatgtgg ggaggcgtcg acaagcattt aaagcggcac tcgccgcacc ccacatcgcc   2324

cggctgcccg gcgcattctc ccctctgatt gcgcgctcca tcgaagaagc cggcttcgaa   2384

ggcgtctacg tttccggcgc cgtcatagct gctgacctgg cactacccga tatcggcttg   2444

acgacgctga ccgaagtcgc ccaccgcgcg cggcaaattg cgcgcgtcac agacctagga   2504

gtgcttgtcg acgccgacac cggctttggc gaacccatgt cggccgcacg caccgtcgcc   2564

gaattggagg acgccggtgt ggccggatgc caccttgaag accaagtcaa ccccaaacgt   2624
```

```
tgcgggcact tggacggcaa agaagtcgtg cgcacagacg tgatggttcg acgcatcgca    2684
gccgccgtct cggcccggcg cgacccgaac tttgtcatct gcgcccgcac cgacgccgct    2744
ggagtggaag gaatcgacgc cgccattgag cgcgcgaaag cctacttaga tgcgggcgcc    2804
gacatgattt tcaccgaagc cctccacagc gaagccgact tccgatactt ccggcacgcc    2864
atccctgatg ccttgttgct ggcgaatatg accgaatttg gcaaaacgac gctgctgtca    2924
gccgacgtgt tggaagagat tggctacaac gccgtgatct accccgtgac cacgctgcgt    2984
attgccatgg gacaag                                                    3000
```

<210> 4
<211> 498
<212> PRT
<213> Corynebacterium glutamicum

<400> 4

Met Arg Ile His Asp Val Tyr Thr His Leu Ser Ala Asp Asn Phe Pro
1 5 10 15

Lys Ala Glu His Leu Ala Trp Lys Phe Ser Glu Leu Ala Thr Asp Pro
20 25 30

Val Glu Val Thr Pro Asp Val Ser Glu Met Ile Ile Asn Arg Ile Ile
35 40 45

Asp Asn Ala Ala Val Ser Ala Ala Ser Val Leu Arg Arg Pro Val Thr
50 55 60

Val Ala Arg Gln Gln Ala Gln Ser His Pro Arg Glu Lys Gly Gly Lys
65 70 75 80

Val Phe Gly Ile Ser Gly Ser Tyr Ser Pro Glu Trp Ala Ala Phe Ala
85 90 95

Asn Gly Val Ala Val Arg Glu Leu Asp Phe His Asp Thr Phe Leu Ala
100 105 110

Ala Glu Tyr Ser His Pro Gly Asp Asn Ile Pro Pro Leu Leu Ala Val
115 120 125

Ala Gln Ala Gln Arg Ser Ser Gly Arg Asp Leu Ile Arg Gly Ile Ala
130 135 140

Thr Ala Tyr Glu Val Gln Val Glu Leu Val Arg Gly Ile Cys Leu His
145 150 155 160

Glu His Lys Ile Asp His Val Ala His Leu Gly Pro Ser Ala Ala Ala
165 170 175

Gly Leu Gly Thr Leu Leu His Val Asp Glu Glu Thr Ile Tyr Gln Ala
180 185 190

Ile Gly Gln Ala Leu His Thr Thr Thr Ala Thr Arg Gln Ser Arg Lys
195 200 205

Gly Glu Ile Ser Ser Trp Lys Ala Phe Ala Pro Ala Phe Ala Gly Lys
210 215 220

Met Ala Ile Glu Ala Met Asp Arg Ala Met Arg Gly Glu Gly Ser Pro
225 230 235 240

Ala Pro Ile Trp Glu Gly Glu Asp Gly Val Ile Ala Trp Leu Leu Ser
245 250 255

Gly Lys Asp His Val Tyr His Val Pro Leu Pro Glu His Gly Glu Pro
260 265 270

Lys Leu Gly Ile Leu Glu Thr Tyr Thr Lys Glu His Ser Ala Glu Tyr
275 280 285

Gln Ser Gln Ala Pro Ile Asp Leu Ala Arg Arg Met Lys Pro Leu Val
290 295 300

Asp Ala Ala Gly Gly Thr Glu His Ile Ala Glu Ile Val Leu Arg Thr
305 310 315 320

Ser His His Thr His Tyr Val Ile Gly Thr Gly Ala Asn Asp Pro Gln
325 330 335

Lys Met Asp Pro Gln Ala Ser Arg Glu Thr Leu Asp His Ser Ile Met
340 345 350

Tyr Ile Phe Ala Val Ala Leu Gln Asp Gly Val Trp His His Glu Phe
355 360 365

Ser Tyr Thr Arg Lys Arg Ser Thr Arg Pro Glu Thr Val Glu Leu Trp
370 375 380

His Lys Ile Arg Thr Val Glu Asp Pro Glu Trp Thr Arg Arg Tyr His
385 390 395 400

Ser Asp Asp Pro Ala Lys Lys Ala Phe Gly Ala Lys Ala Val Ile Thr
405 410 415

Met Ala Asp Gly Thr Val Ile Glu Asp Glu Leu Ala Val Ala Asp Ala
420 425 430

His Pro Leu Gly Ala Arg Pro Phe Ala Arg Glu Asn Tyr Ile Glu Lys
435 440 445

Phe Arg Thr Leu Ala Gln Gly Ile Val Ile Asp Ser Glu Gln Glu Arg
450 455 460

Phe Leu His Ala Val Gln Ser Leu Pro Asp Leu Asp Asp Leu Asp Gln
465 470 475 480

Leu Asn Ile Glu Val Asp Ile Ser Asn Gln Ala Ala Thr Lys Ala Gly
485 490 495

Leu Leu

<210> 5
<211> 1497
<212> DNA
<213> Corynebacterium glutamicum

<220>
<221> CDS
<222> (1)..(1494)
<223> prpD1-Allel

<220>
<221> mutation
<222> (815)..(815)
<223> C -> T Transition

<400> 5

```
atg cgc atc cac gat gtt tat acc cac ctt tcg gcc gat aac ttt ccc      48
Met Arg Ile His Asp Val Tyr Thr His Leu Ser Ala Asp Asn Phe Pro
1               5                   10                  15

aaa gca gag cac ctt gcg tgg aaa ttc tcc gag ctt gcc acc gac ccc      96
Lys Ala Glu His Leu Ala Trp Lys Phe Ser Glu Leu Ala Thr Asp Pro
            20                  25                  30

gtg gag gtg aca ccg gat gtt tcg gag atg atc atc aac cgg atc atc     144
Val Glu Val Thr Pro Asp Val Ser Glu Met Ile Ile Asn Arg Ile Ile
        35                  40                  45

gac aac gcg gcg gtg tct gcc gcg tcg gtg ttg cgc cgg cct gtg act     192
Asp Asn Ala Ala Val Ser Ala Ala Ser Val Leu Arg Arg Pro Val Thr
    50                  55                  60

gtg gcc agg caa caa gcg cag tcc cat ccg cgg gaa aag ggc gga aaa     240
Val Ala Arg Gln Gln Ala Gln Ser His Pro Arg Glu Lys Gly Gly Lys
65                  70                  75                  80

gtt ttt gga att tca ggc agc tac tca cca gag tgg gct gcc ttt gct     288
Val Phe Gly Ile Ser Gly Ser Tyr Ser Pro Glu Trp Ala Ala Phe Ala
                85                  90                  95

aat ggt gtg gcc gta cgt gaa ttg gac ttc cac gat aca ttt tta gca     336
Asn Gly Val Ala Val Arg Glu Leu Asp Phe His Asp Thr Phe Leu Ala
            100                 105                 110

gct gaa tac tcc cat ccc ggc gac aat att cca cca ctt ctt gca gta     384
Ala Glu Tyr Ser His Pro Gly Asp Asn Ile Pro Pro Leu Leu Ala Val
        115                 120                 125

gcg cag gct cag aga agc agc ggc agg gat ctc att cgg ggt atc gcc     432
Ala Gln Ala Gln Arg Ser Ser Gly Arg Asp Leu Ile Arg Gly Ile Ala
    130                 135                 140

act gcc tat gag gtg cag gtg gaa ttg gtg agg ggg atc tgc ttg cat     480
Thr Ala Tyr Glu Val Gln Val Glu Leu Val Arg Gly Ile Cys Leu His
145                 150                 155                 160
```

```
gag cac aaa att gat cac gta gcc cac ctt ggt ccc agc gct gca gcg      528
Glu His Lys Ile Asp His Val Ala His Leu Gly Pro Ser Ala Ala Ala
                165                 170                 175

ggt ttg ggc acg ttg ttg cat gta gat gag gaa acc atc tac cag gcg      576
Gly Leu Gly Thr Leu Leu His Val Asp Glu Glu Thr Ile Tyr Gln Ala
            180                 185                 190

atc ggc cag gca ttg cac acc acg acg gct acg agg cag tcg cga aaa      624
Ile Gly Gln Ala Leu His Thr Thr Thr Ala Thr Arg Gln Ser Arg Lys
        195                 200                 205

ggt gag att tcc agc tgg aag gcg ttc gcg cca gcg ttt gcg gga aag      672
Gly Glu Ile Ser Ser Trp Lys Ala Phe Ala Pro Ala Phe Ala Gly Lys
    210                 215                 220

atg gcc att gag gcg atg gat cgt gcg atg cgt ggg gag ggt tcg ccc      720
Met Ala Ile Glu Ala Met Asp Arg Ala Met Arg Gly Glu Gly Ser Pro
225                 230                 235                 240

gca ccg att tgg gag ggc gaa gac ggg gtc atc gcg tgg ctg tta tcg      768
Ala Pro Ile Trp Glu Gly Glu Asp Gly Val Ile Ala Trp Leu Leu Ser
                245                 250                 255

ggc aaa gat cat gtt tat cat gtg cca ttg ccg gaa cac ggc gag ctc      816
Gly Lys Asp His Val Tyr His Val Pro Leu Pro Glu His Gly Glu Leu
            260                 265                 270

aag ctg ggg att cta gag act tac aca aag gaa cat tca gcg gaa tat      864
Lys Leu Gly Ile Leu Glu Thr Tyr Thr Lys Glu His Ser Ala Glu Tyr
        275                 280                 285

caa tcg cag gca ccg att gat ctg gcg cgc agg atg aag cca ctg gtt      912
Gln Ser Gln Ala Pro Ile Asp Leu Ala Arg Arg Met Lys Pro Leu Val
    290                 295                 300

gac gcg gct ggc gga acg gaa cac att gca gag att gtg ctg cgc acc      960
Asp Ala Ala Gly Gly Thr Glu His Ile Ala Glu Ile Val Leu Arg Thr
305                 310                 315                 320

agt cac cac acg cat tat gtg att ggc act ggg gcg aac gat ccg cag     1008
Ser His His Thr His Tyr Val Ile Gly Thr Gly Ala Asn Asp Pro Gln
                325                 330                 335

aag atg gat ccg cag gcc tcg cgt gaa acc ctg gat cat tcc atc atg     1056
Lys Met Asp Pro Gln Ala Ser Arg Glu Thr Leu Asp His Ser Ile Met
            340                 345                 350

tac att ttc gcc gtc gcg ctt caa gat ggc gtg tgg cac cac gag ttt     1104
Tyr Ile Phe Ala Val Ala Leu Gln Asp Gly Val Trp His His Glu Phe
        355                 360                 365

tcc tac acc cgc aag cgt tcc acc cgc ccg gaa act gtg gag ctg tgg     1152
Ser Tyr Thr Arg Lys Arg Ser Thr Arg Pro Glu Thr Val Glu Leu Trp
    370                 375                 380

cac aag att cgc acc gtg gag gat cct gaa tgg acg cgc cga tac cat     1200
His Lys Ile Arg Thr Val Glu Asp Pro Glu Trp Thr Arg Arg Tyr His
385                 390                 395                 400

tct gat gat cct gca aaa aag gcc ttt ggt gcg aaa gca gtg atc aca     1248
Ser Asp Asp Pro Ala Lys Lys Ala Phe Gly Ala Lys Ala Val Ile Thr
                405                 410                 415

atg gct gat ggc acc gtg att gag gat gaa ttg gct gtc gcg gat gcc     1296
Met Ala Asp Gly Thr Val Ile Glu Asp Glu Leu Ala Val Ala Asp Ala
            420                 425                 430
```

```
cac ccg ctg ggt gct cgg ccg ttt gcg cgg gag aat tac att gaa aaa      1344
His Pro Leu Gly Ala Arg Pro Phe Ala Arg Glu Asn Tyr Ile Glu Lys
        435                 440                 445

ttc cgc aca ctc gcg cag ggg att gtc att gat tca gaa cag gaa cgc      1392
Phe Arg Thr Leu Ala Gln Gly Ile Val Ile Asp Ser Glu Gln Glu Arg
    450                 455                 460

ttc ttg cat gcc gtg caa agc ctg cct gac ctg gat gat ctt gat cag      1440
Phe Leu His Ala Val Gln Ser Leu Pro Asp Leu Asp Asp Leu Asp Gln
465                 470                 475                 480

ctc aac atc gaa gtc gac ata agc aac cag gcc gcg acg aaa gcg ggg      1488
Leu Asn Ile Glu Val Asp Ile Ser Asn Gln Ala Ala Thr Lys Ala Gly
                485                 490                 495

ctg tta tga                                                          1497
Leu Leu
```

<210> 6
<211> 498
<212> PRT
<213> Corynebacterium glutamicum

<400> 6

Met Arg Ile His Asp Val Tyr Thr His Leu Ser Ala Asp Asn Phe Pro
1 5 10 15

Lys Ala Glu His Leu Ala Trp Lys Phe Ser Glu Leu Ala Thr Asp Pro
20 25 30

Val Glu Val Thr Pro Asp Val Ser Glu Met Ile Ile Asn Arg Ile Ile
35 40 45

Asp Asn Ala Ala Val Ser Ala Ala Ser Val Leu Arg Arg Pro Val Thr
50 55 60

Val Ala Arg Gln Gln Ala Gln Ser His Pro Arg Glu Lys Gly Gly Lys
65 70 75 80

Val Phe Gly Ile Ser Gly Ser Tyr Ser Pro Glu Trp Ala Ala Phe Ala
85 90 95

Asn Gly Val Ala Val Arg Glu Leu Asp Phe His Asp Thr Phe Leu Ala
100 105 110

Ala Glu Tyr Ser His Pro Gly Asp Asn Ile Pro Pro Leu Leu Ala Val
115 120 125

Ala Gln Ala Gln Arg Ser Ser Gly Arg Asp Leu Ile Arg Gly Ile Ala
130 135 140

Thr Ala Tyr Glu Val Gln Val Glu Leu Val Arg Gly Ile Cys Leu His
145 150 155 160

Glu His Lys Ile Asp His Val Ala His Leu Gly Pro Ser Ala Ala Ala

165 170 175

Gly Leu Gly Thr Leu Leu His Val Asp Glu Glu Thr Ile Tyr Gln Ala
180 185 190

Ile Gly Gln Ala Leu His Thr Thr Thr Ala Thr Arg Gln Ser Arg Lys
195 200 205

Gly Glu Ile Ser Ser Trp Lys Ala Phe Ala Pro Ala Phe Ala Gly Lys
210 215 220

Met Ala Ile Glu Ala Met Asp Arg Ala Met Arg Gly Glu Gly Ser Pro
225 230 235 240

Ala Pro Ile Trp Glu Gly Glu Asp Gly Val Ile Ala Trp Leu Leu Ser
245 250 255

Gly Lys Asp His Val Tyr His Val Pro Leu Pro Glu His Gly Glu Leu
260 265 270

Lys Leu Gly Ile Leu Glu Thr Tyr Thr Lys Glu His Ser Ala Glu Tyr
275 280 285

Gln Ser Gln Ala Pro Ile Asp Leu Ala Arg Arg Met Lys Pro Leu Val
290 295 300

Asp Ala Ala Gly Gly Thr Glu His Ile Ala Glu Ile Val Leu Arg Thr
305 310 315 320

Ser His His Thr His Tyr Val Ile Gly Thr Gly Ala Asn Asp Pro Gln
325 330 335

Lys Met Asp Pro Gln Ala Ser Arg Glu Thr Leu Asp His Ser Ile Met
340 345 350

Tyr Ile Phe Ala Val Ala Leu Gln Asp Gly Val Trp His His Glu Phe
355 360 365

Ser Tyr Thr Arg Lys Arg Ser Thr Arg Pro Glu Thr Val Glu Leu Trp
370 375 380

His Lys Ile Arg Thr Val Glu Asp Pro Glu Trp Thr Arg Arg Tyr His
385 390 395 400

Ser Asp Asp Pro Ala Lys Lys Ala Phe Gly Ala Lys Ala Val Ile Thr
405 410 415

Met Ala Asp Gly Thr Val Ile Glu Asp Glu Leu Ala Val Ala Asp Ala
420 425 430

His Pro Leu Gly Ala Arg Pro Phe Ala Arg Glu Asn Tyr Ile Glu Lys
435 440 445

```
Phe Arg Thr Leu Ala Gln Gly Ile Val Ile Asp Ser Glu Gln Glu Arg
    450                 455                 460

Phe Leu His Ala Val Gln Ser Leu Pro Asp Leu Asp Asp Leu Asp Gln
465                 470                 475                 480

Leu Asn Ile Glu Val Asp Ile Ser Asn Gln Ala Ala Thr Lys Ala Gly
                485                 490                 495

Leu Leu
```

<210> 7
<211> 3000
<212> DNA
<213> Corynebacterium glutamicum

<220>
<221> misc feature
<222> (1)..(750)
<223> stromaufwärts der CDS gelegene Sequenz

<220>
<221> primer_bind
<222> (661)..(680)
<223> Bindungsstelle Primer prpD1_XL_A1

<220>
<221> CDS
<222> (751)..(2244)
<223> prpD1-Allel

<220>
<221> mutation
<222> (1565)..(1565)
<223> C -> T Transition

<220>
<221> misc_feature
<222> (2245)..(3000)
<223> stromabwärts der CDS gelegene Sequenz

<220>
<221> primer_bind
<222> (2258)..(2277)
<223> Bindungsstelle Primer prpD1_XL_E1

<400> 7

```
ggttccacct tcgccataac gcttgacgac gcctccctcc ttcactctcg gcgcatcatt      60
ttggcccacg gcgccgttga cgatctgcca gaggtagaag gactgtcaga tttttgggga     120
accaaagtgt tgcactgcgc ttactgccac ggctttgagg cccgcgattc tgaaatcgtc     180
gtggtgggta cctcgcccat ggctgcgcac caagcgttga tgttctcgca gttgtccaaa     240
actgtcagct tggtgggcac gatcgacatt gatgaacaaa ccagcgagag cctagatagt     300
gctggagtaa aagtgttggg caccaatgcg gtgcgcgtat ccgccgaagg tgatggcctg     360
tctgtggaac tgtccgaagg cgatcattta agctgcgaca acatcgtggt ggcatctcgt     420
```

ccactggtgg atggcacgct gtacacccaa cttggtggtc agatggaaga aaacccgatg 480

ggcaggttca ttccaggtac ccaaaccggg cgcactccta ttgaaggtgt gtgggctgcc 540

ggaaacgcgc aagctcccat ggcgatggtc tatggttccg ctgctcaagg cgtgatggct 600

ggagcagaga tcaactttga tctgatcctg gaagatattt ccgtggcaag cgcgcagagc 660

taaactgcgt gaggttgtgg cctgtcacac ataatcggcc tagggtggga ctttaaggaa 720

acagtgcaca aataaatctc aaggagcccc atg cgc atc cac gat gtt tat acc 774
Met Arg Ile His Asp Val Tyr Thr
1 5

cac ctt tcg gcc gat aac ttt ccc aaa gca gag cac ctt gcg tgg aaa 822
His Leu Ser Ala Asp Asn Phe Pro Lys Ala Glu His Leu Ala Trp Lys
10 15 20

ttc tcc gag ctt gcc acc gac ccc gtg gag gtg aca ccg gat gtt tcg 870
Phe Ser Glu Leu Ala Thr Asp Pro Val Glu Val Thr Pro Asp Val Ser
25 30 35 40

gag atg atc atc aac cgg atc atc gac aac gcg gcg gtg tct gcc gcg 918
Glu Met Ile Ile Asn Arg Ile Ile Asp Asn Ala Ala Val Ser Ala Ala
45 50 55

tcg gtg ttg cgc cgg cct gtg act gtg gcc agg caa caa gcg cag tcc 966
Ser Val Leu Arg Arg Pro Val Thr Val Ala Arg Gln Gln Ala Gln Ser
60 65 70

cat ccg cgg gaa aag ggc gga aaa gtt ttt gga att tca ggc agc tac 1014
His Pro Arg Glu Lys Gly Gly Lys Val Phe Gly Ile Ser Gly Ser Tyr
75 80 85

tca cca gag tgg gct gcc ttt gct aat ggt gtg gcc gta cgt gaa ttg 1062
Ser Pro Glu Trp Ala Ala Phe Ala Asn Gly Val Ala Val Arg Glu Leu
90 95 100

gac ttc cac gat aca ttt tta gca gct gaa tac tcc cat ccc ggc gac 1110
Asp Phe His Asp Thr Phe Leu Ala Ala Glu Tyr Ser His Pro Gly Asp
105 110 115 120

aat att cca cca ctt ctt gca gta gcg cag gct cag aga agc agc ggc 1158
Asn Ile Pro Pro Leu Leu Ala Val Ala Gln Ala Gln Arg Ser Ser Gly
125 130 135

agg gat ctc att cgg ggt atc gcc act gcc tat gag gtg cag gtg gaa 1206
Arg Asp Leu Ile Arg Gly Ile Ala Thr Ala Tyr Glu Val Gln Val Glu
140 145 150

ttg gtg agg ggg atc tgc ttg cat gag cac aaa att gat cac gta gcc 1254
Leu Val Arg Gly Ile Cys Leu His Glu His Lys Ile Asp His Val Ala
155 160 165

cac ctt ggt ccc agc gct gca gcg ggt ttg ggc acg ttg ttg cat gta 1302
His Leu Gly Pro Ser Ala Ala Ala Gly Leu Gly Thr Leu Leu His Val
170 175 180

gat gag gaa acc atc tac cag gcg atc ggc cag gca ttg cac acc acg 1350
Asp Glu Glu Thr Ile Tyr Gln Ala Ile Gly Gln Ala Leu His Thr Thr
185 190 195 200

acg gct acg agg cag tcg cga aaa ggt gag att tcc agc tgg aag gcg 1398
Thr Ala Thr Arg Gln Ser Arg Lys Gly Glu Ile Ser Ser Trp Lys Ala
205 210 215

ttc gcg cca gcg ttt gcg gga aag atg gcc att gag gcg atg gat cgt 1446
Phe Ala Pro Ala Phe Ala Gly Lys Met Ala Ile Glu Ala Met Asp Arg
220 225 230

```
gcg atg cgt ggg gag ggt tcg ccc gca ccg att tgg gag ggc gaa gac      1494
Ala Met Arg Gly Glu Gly Ser Pro Ala Pro Ile Trp Glu Gly Glu Asp
        235                 240                     245

ggg gtc atc gcg tgg ctg tta tcg ggc aaa gat cat gtt tat cat gtg      1542
Gly Val Ile Ala Trp Leu Leu Ser Gly Lys Asp His Val Tyr His Val
    250                 255                     260

cca ttg ccg gaa cac ggc gag ctc aag ctg ggg att cta gag act tac      1590
Pro Leu Pro Glu His Gly Glu Leu Lys Leu Gly Ile Leu Glu Thr Tyr
265                 270                     275                 280

aca aag gaa cat tca gcg gaa tat caa tcg cag gca ccg att gat ctg      1638
Thr Lys Glu His Ser Ala Glu Tyr Gln Ser Gln Ala Pro Ile Asp Leu
                285                     290                 295

gcg cgc agg atg aag cca ctg gtt gac gcg gct ggc gga acg gaa cac      1686
Ala Arg Arg Met Lys Pro Leu Val Asp Ala Ala Gly Gly Thr Glu His
            300                     305                 310

att gca gag att gtg ctg cgc acc agt cac cac acg cat tat gtg att      1734
Ile Ala Glu Ile Val Leu Arg Thr Ser His His Thr His Tyr Val Ile
        315                 320                     325

ggc act ggg gcg aac gat ccg cag aag atg gat ccg cag gcc tcg cgt      1782
Gly Thr Gly Ala Asn Asp Pro Gln Lys Met Asp Pro Gln Ala Ser Arg
    330                 335                     340

gaa acc ctg gat cat tcc atc atg tac att ttc gcc gtc gcg ctt caa      1830
Glu Thr Leu Asp His Ser Ile Met Tyr Ile Phe Ala Val Ala Leu Gln
345                 350                     355                 360

gat ggc gtg tgg cac cac gag ttt tcc tac acc cgc aag cgt tcc acc      1878
Asp Gly Val Trp His His Glu Phe Ser Tyr Thr Arg Lys Arg Ser Thr
                365                     370                 375

cgc ccg gaa act gtg gag ctg tgg cac aag att cgc acc gtg gag gat      1926
Arg Pro Glu Thr Val Glu Leu Trp His Lys Ile Arg Thr Val Glu Asp
            380                     385                 390

cct gaa tgg acg cgc cga tac cat tct gat gat cct gca aaa aag gcc      1974
Pro Glu Trp Thr Arg Arg Tyr His Ser Asp Asp Pro Ala Lys Lys Ala
        395                 400                     405

ttt ggt gcg aaa gca gtg atc aca atg gct gat ggc acc gtg att gag      2022
Phe Gly Ala Lys Ala Val Ile Thr Met Ala Asp Gly Thr Val Ile Glu
    410                 415                     420

gat gaa ttg gct gtc gcg gat gcc cac ccg ctg ggt gct cgg ccg ttt      2070
Asp Glu Leu Ala Val Ala Asp Ala His Pro Leu Gly Ala Arg Pro Phe
425                 430                     435                 440

gcg cgg gag aat tac att gaa aaa ttc cgc aca ctc gcg cag ggg att      2118
Ala Arg Glu Asn Tyr Ile Glu Lys Phe Arg Thr Leu Ala Gln Gly Ile
                445                     450                 455

gtc att gat tca gaa cag gaa cgc ttc ttg cat gcc gtg caa agc ctg      2166
Val Ile Asp Ser Glu Gln Glu Arg Phe Leu His Ala Val Gln Ser Leu
            460                     465                 470

cct gac ctg gat gat ctt gat cag ctc aac atc gaa gtc gac ata agc      2214
Pro Asp Leu Asp Asp Leu Asp Gln Leu Asn Ile Glu Val Asp Ile Ser
        475                 480                     485

aac cag gcc gcg acg aaa gcg ggg ctg tta tgaatctctt ttcgaatggt        2264
Asn Gln Ala Ala Thr Lys Ala Gly Leu Leu
    490                 495
```

gttgatgtgg ggaggcgtcg acaagcattt aaagcggcac tcgccgcacc ccacatcgcc 2324
cggctgcccg gcgcattctc ccctctgatt gcgcgctcca tcgaagaagc cggcttcgaa 2384
ggcgtctacg tttccggcgc cgtcatagct gctgacctgg cactacccga tatcggcttg 2444
acgacgctga ccgaagtcgc ccaccgcgcg cggcaaattg cgcgcgtcac agacctagga 2504
gtgcttgtcg acgccgacac cggctttggc gaacccatgt cggccgcacg caccgtcgcc 2564
gaattggagg acgccggtgt ggccggatgc caccttgaag accaagtcaa ccccaaacgt 2624
tgcgggcact tggacggcaa agaagtcgtg cgcacagacg tgatggttcg acgcatcgca 2684
gccgccgtct cggcccggcg cgacccgaac tttgtcatct gcgcccgcac cgacgccgct 2744
ggagtggaag gaatcgacgc cgccattgag cgcgcgaaag cctacttaga tgcgggcgcc 2804
gacatgattt tcaccgaagc cctccacagc gaagccgact tccgatactt ccggcacgcc 2864
atccctgatg ccttgttgct ggcgaatatg accgaatttg gcaaaacgac gctgctgtca 2924
gccgacgtgt tggaagagat tggctacaac gccgtgatct accccgtgac cacgctgcgt 2984
attgccatgg gacaag 3000

<210> 8
<211> 498
<212> PRT
<213> Corynebacterium glutamicum

<400> 8

```
Met Arg Ile His Asp Val Tyr Thr His Leu Ser Ala Asp Asn Phe Pro
1               5                   10                  15

Lys Ala Glu His Leu Ala Trp Lys Phe Ser Glu Leu Ala Thr Asp Pro
            20                  25                  30

Val Glu Val Thr Pro Asp Val Ser Glu Met Ile Ile Asn Arg Ile Ile
        35                  40                  45

Asp Asn Ala Ala Val Ser Ala Ala Ser Val Leu Arg Arg Pro Val Thr
    50                  55                  60

Val Ala Arg Gln Gln Ala Gln Ser His Pro Arg Glu Lys Gly Gly Lys
65                  70                  75                  80

Val Phe Gly Ile Ser Gly Ser Tyr Ser Pro Glu Trp Ala Ala Phe Ala
                85                  90                  95

Asn Gly Val Ala Val Arg Glu Leu Asp Phe His Asp Thr Phe Leu Ala
            100                 105                 110

Ala Glu Tyr Ser His Pro Gly Asp Asn Ile Pro Pro Leu Leu Ala Val
        115                 120                 125

Ala Gln Ala Gln Arg Ser Ser Gly Arg Asp Leu Ile Arg Gly Ile Ala
    130                 135                 140
```

Thr Ala Tyr Glu Val Gln Val Glu Leu Val Arg Gly Ile Cys Leu His
145 150 155 160

Glu His Lys Ile Asp His Val Ala His Leu Gly Pro Ser Ala Ala Ala
165 170 175

Gly Leu Gly Thr Leu Leu His Val Asp Glu Glu Thr Ile Tyr Gln Ala
180 185 190

Ile Gly Gln Ala Leu His Thr Thr Thr Ala Thr Arg Gln Ser Arg Lys
195 200 205

Gly Glu Ile Ser Ser Trp Lys Ala Phe Ala Pro Ala Phe Ala Gly Lys
210 215 220

Met Ala Ile Glu Ala Met Asp Arg Ala Met Arg Gly Glu Gly Ser Pro
225 230 235 240

Ala Pro Ile Trp Glu Gly Glu Asp Gly Val Ile Ala Trp Leu Leu Ser
245 250 255

Gly Lys Asp His Val Tyr His Val Pro Leu Pro Glu His Gly Glu Leu
260 265 270

Lys Leu Gly Ile Leu Glu Thr Tyr Thr Lys Glu His Ser Ala Glu Tyr
275 280 285

Gln Ser Gln Ala Pro Ile Asp Leu Ala Arg Arg Met Lys Pro Leu Val
290 295 300

Asp Ala Ala Gly Gly Thr Glu His Ile Ala Glu Ile Val Leu Arg Thr
305 310 315 320

Ser His His Thr His Tyr Val Ile Gly Thr Gly Ala Asn Asp Pro Gln
325 330 335

Lys Met Asp Pro Gln Ala Ser Arg Glu Thr Leu Asp His Ser Ile Met
340 345 350

Tyr Ile Phe Ala Val Ala Leu Gln Asp Gly Val Trp His His Glu Phe
355 360 365

Ser Tyr Thr Arg Lys Arg Ser Thr Arg Pro Glu Thr Val Glu Leu Trp
370 375 380

His Lys Ile Arg Thr Val Glu Asp Pro Glu Trp Thr Arg Arg Tyr His
385 390 395 400

Ser Asp Asp Pro Ala Lys Lys Ala Phe Gly Ala Lys Ala Val Ile Thr
405 410 415

Met Ala Asp Gly Thr Val Ile Glu Asp Glu Leu Ala Val Ala Asp Ala
420 425 430

His Pro Leu Gly Ala Arg Pro Phe Ala Arg Glu Asn Tyr Ile Glu Lys
435 440 445

Phe Arg Thr Leu Ala Gln Gly Ile Val Ile Asp Ser Glu Gln Glu Arg
450 455 460

Phe Leu His Ala Val Gln Ser Leu Pro Asp Leu Asp Asp Leu Asp Gln
465 470 475 480

Leu Asn Ile Glu Val Asp Ile Ser Asn Gln Ala Ala Thr Lys Ala Gly
485 490 495

Leu Leu

<210> 9
<211> 28
<212> DNA
<213> Artificial Sequence

<220>
<223> Primer prpD1_xL_A1

<220>
<221> misc_feature
<222> (1)..(2)

<220>
<221> misc_structure
<222> (3)..(8)
<223> EcoRI-Restriktionsschnittstelle

<400> 9
gcgaattcta aactgcgtga ggttgtgg 28

<210> 10
<211> 28
<212> DNA
<213> Artificial Sequence

<220>
<223> Primer prpD1_XL_E1

<220>
<221> misc_feature
<222> (1)..(2)

<220>
<221> misc_structure
<222> (3)..(8)
<223> EcoRI-Restriktionsschnittstelle

<400> 10

gcgaattctc. cccacatcaa caccattc 28

<210> 11
<211> 1263
<212> DNA
<213> Corynebacterium glutamicum

<220>
<221> CDS
<222> (1)..(1263)
<223> lysC-wildtyp-Gen

<400> 11

```
gtg gcc ctg gtc gta cag aaa tat ggc ggt tcc tcg ctt gag agt gcg      48
Met Ala Leu Val Val Gln Lys Tyr Gly Gly Ser Ser Leu Glu Ser Ala
1               5                   10                  15

gaa cgc att aga aac gtc gct gaa cgg atc gtt gcc acc aag aag gct      96
Glu Arg Ile Arg Asn Val Ala Glu Arg Ile Val Ala Thr Lys Lys Ala
            20                  25                  30

gga aat gat gtc gtg gtt gtc tgc tcc gca atg gga gac acc acg gat     144
Gly Asn Asp Val Val Val Val Cys Ser Ala Met Gly Asp Thr Thr Asp
        35                  40                  45

gaa ctt cta gaa ctt gca gcg gca gtg aat ccc gtt ccg cca gct cgt     192
Glu Leu Leu Glu Leu Ala Ala Ala Val Asn Pro Val Pro Pro Ala Arg
    50                  55                  60

gaa atg gat atg ctc ctg act gct ggt gag cgt att tct aac gct ctc     240
Glu Met Asp Met Leu Leu Thr Ala Gly Glu Arg Ile Ser Asn Ala Leu
65                  70                  75                  80

gtc gcc atg gct att gag tcc ctt ggc gca gaa gcc caa tct ttc acg     288
Val Ala Met Ala Ile Glu Ser Leu Gly Ala Glu Ala Gln Ser Phe Thr
                85                  90                  95

ggc tct cag gct ggt gtg ctc acc acc gag cgc cac gga aac gca cgc     336
Gly Ser Gln Ala Gly Val Leu Thr Thr Glu Arg His Gly Asn Ala Arg
            100                 105                 110

att gtt gat gtc act cca ggt cgt gtg cgt gaa gca ctc gat gag ggc     384
Ile Val Asp Val Thr Pro Gly Arg Val Arg Glu Ala Leu Asp Glu Gly
        115                 120                 125

aag atc tgc att gtt gct ggt ttc cag ggt gtt aat aaa gaa acc cgc     432
Lys Ile Cys Ile Val Ala Gly Phe Gln Gly Val Asn Lys Glu Thr Arg
    130                 135                 140

gat gtc acc acg ttg ggt cgt ggt ggt tct gac acc act gca gtt gcg     480
Asp Val Thr Thr Leu Gly Arg Gly Gly Ser Asp Thr Thr Ala Val Ala
145                 150                 155                 160

ttg gca gct gct ttg aac gct gat gtg tgt gag att tac tcg gac gtt     528
Leu Ala Ala Ala Leu Asn Ala Asp Val Cys Glu Ile Tyr Ser Asp Val
                165                 170                 175

gac ggt gtg tat acc gct gac ccg cgc atc gtt cct aat gca cag aag     576
Asp Gly Val Tyr Thr Ala Asp Pro Arg Ile Val Pro Asn Ala Gln Lys
            180                 185                 190

ctg gaa aag ctc agc ttc gaa gaa atg ctg gaa ctt gct gct gtt ggc     624
Leu Glu Lys Leu Ser Phe Glu Glu Met Leu Glu Leu Ala Ala Val Gly
        195                 200                 205

tcc aag att ttg gtg ctg cgc agt gtt gaa tac gct cgt gca ttc aat     672
Ser Lys Ile Leu Val Leu Arg Ser Val Glu Tyr Ala Arg Ala Phe Asn
    210                 215                 220

gtg cca ctt cgc gta cgc tcg tct tat agt aat gat ccc ggc act ttg     720
Val Pro Leu Arg Val Arg Ser Ser Tyr Ser Asn Asp Pro Gly Thr Leu
```

```
225                 230                 235                 240

att gcc ggc tct atg gag gat att cct gtg gaa gaa gca gtc ctt acc        768
Ile Ala Gly Ser Met Glu Asp Ile Pro Val Glu Glu Ala Val Leu Thr
                245                 250                 255

ggt gtc gca acc gac aag tcc gaa gcc aaa gta acc gtt ctg ggt att        816
Gly Val Ala Thr Asp Lys Ser Glu Ala Lys Val Thr Val Leu Gly Ile
            260                 265                 270

tcc gat aag cca ggc gag gct gcg aag gtt ttc cgt gcg ttg gct gat        864
Ser Asp Lys Pro Gly Glu Ala Ala Lys Val Phe Arg Ala Leu Ala Asp
        275                 280                 285

gca gaa atc aac att gac atg gtt ctg cag aac gtc tct tct gta gaa        912
Ala Glu Ile Asn Ile Asp Met Val Leu Gln Asn Val Ser Ser Val Glu
    290                 295                 300

gac ggc acc acc gac atc acc ttc acc tgc cct cgt tcc gac ggc cgc        960
Asp Gly Thr Thr Asp Ile Thr Phe Thr Cys Pro Arg Ser Asp Gly Arg
305                 310                 315                 320

cgc gcg atg gag atc ttg aag aag ctt cag gtt cag ggc aac tgg acc       1008
Arg Ala Met Glu Ile Leu Lys Lys Leu Gln Val Gln Gly Asn Trp Thr
                325                 330                 335

aat gtg ctt tac gac gac cag gtc ggc aaa gtc tcc ctc gtg ggt gct       1056
Asn Val Leu Tyr Asp Asp Gln Val Gly Lys Val Ser Leu Val Gly Ala
            340                 345                 350

ggc atg aag tct cac cca ggt gtt acc gca gag ttc atg gaa gct ctg       1104
Gly Met Lys Ser His Pro Gly Val Thr Ala Glu Phe Met Glu Ala Leu
        355                 360                 365

cgc gat gtc aac gtg aac atc gaa ttg att tcc acc tct gag att cgt       1152
Arg Asp Val Asn Val Asn Ile Glu Leu Ile Ser Thr Ser Glu Ile Arg
    370                 375                 380

att tcc gtg ctg atc cgt gaa gat gat ctg gat gct gct gca cgt gca       1200
Ile Ser Val Leu Ile Arg Glu Asp Asp Leu Asp Ala Ala Ala Arg Ala
385                 390                 395                 400

ttg cat gag cag ttc cag ctg ggc ggc gaa gac gaa gcc gtc gtt tat       1248
Leu His Glu Gln Phe Gln Leu Gly Gly Glu Asp Glu Ala Val Val Tyr
                405                 410                 415

gca ggc acc gga cgc                                                   1263
Ala Gly Thr Gly Arg
            420
```

<210> 12
<211> 421
<212> PRT
<213> Corynebacterium glutamicum

<400> 12

```
Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45
```

Glu Leu Leu Glu Leu Ala Ala Ala Val Asn Pro Val Pro Pro Ala Arg
50 55 60

Glu Met Asp Met Leu Leu Thr Ala Gly Glu Arg Ile Ser Asn Ala Leu
65 70 75 80

Val Ala Met Ala Ile Glu Ser Leu Gly Ala Glu Ala Gln Ser Phe Thr
85 90 95

Gly Ser Gln Ala Gly Val Leu Thr Thr Glu Arg His Gly Asn Ala Arg
100 105 110

Ile Val Asp Val Thr Pro Gly Arg Val Arg Glu Ala Leu Asp Glu Gly
115 120 125

Lys Ile Cys Ile Val Ala Gly Phe Gln Gly Val Asn Lys Glu Thr Arg
130 135 140

Asp Val Thr Thr Leu Gly Arg Gly Gly Ser Asp Thr Thr Ala Val Ala
145 150 155 160

Leu Ala Ala Ala Leu Asn Ala Asp Val Cys Glu Ile Tyr Ser Asp Val
165 170 175

Asp Gly Val Tyr Thr Ala Asp Pro Arg Ile Val Pro Asn Ala Gln Lys
180 185 190

Leu Glu Lys Leu Ser Phe Glu Glu Met Leu Glu Leu Ala Ala Val Gly
195 200 205

Ser Lys Ile Leu Val Leu Arg Ser Val Glu Tyr Ala Arg Ala Phe Asn
210 215 220

Val Pro Leu Arg Val Arg Ser Ser Tyr Ser Asn Asp Pro Gly Thr Leu
225 230 235 240

Ile Ala Gly Ser Met Glu Asp Ile Pro Val Glu Glu Ala Val Leu Thr
245 250 255

Gly Val Ala Thr Asp Lys Ser Glu Ala Lys Val Thr Val Leu Gly Ile
260 265 270

Ser Asp Lys Pro Gly Glu Ala Ala Lys Val Phe Arg Ala Leu Ala Asp
275 280 285

Ala Glu Ile Asn Ile Asp Met Val Leu Gln Asn Val Ser Ser Val Glu
290 295 300

Asp Gly Thr Thr Asp Ile Thr Phe Thr Cys Pro Arg Ser Asp Gly Arg
305 310 315 320

Arg Ala Met Glu Ile Leu Lys Lys Leu Gln Val Gln Gly Asn Trp Thr
325 330 335

Asn Val Leu Tyr Asp Asp Gln Val Gly Lys Val Ser Leu Val Gly Ala
340 345 350

Gly Met Lys Ser His Pro Gly Val Thr Ala Glu Phe Met Glu Ala Leu
355 360 365

Arg Asp Val Asn Val Asn Ile Glu Leu Ile Ser Thr Ser Glu Ile Arg
370 375 380

Ile Ser Val Leu Ile Arg Glu Asp Asp Leu Asp Ala Ala Ala Arg Ala
385 390 395 400

Leu His Glu Gln Phe Gln Leu Gly Gly Glu Asp Glu Ala Val Val Tyr
405 410 415

Ala Gly Thr Gly Arg
420

<210> 13
<211> 1633
<212> DNA
<213> Corynebacterium glutamicum

<220>
<221> misc_feature
<222> (1)..(2)

<220>
<221> misc_feature
<222> (1)..(1633)
<223> PCR-Produkt

<220>
<221> misc_structure
<222> (3)..(8)
<223> EcoRI-Restriktionsschnittstelle

<220>
<221> CDS
<222> (99)..(1592)

<220>
<221> mutation
<222> (913)..(913)
<223> C -> T Transition

<220>
<221> misc_structure
<222> (1626)..(1631)
<223> ECORI-Restriktionsschnittstelle

<220>

<221> misc_feature
<222> (1632)..(1633)

<400> 13
gcgaattcta aactgcgtga ggttgtggcc tgtcacacat aatcggccta gggtgggact 60

```
ttaaggaaac agtgcacaaa taaatctcaa ggagcccc atg cgc atc cac gat gtt    116
                                          Met Arg Ile His Asp Val
                                          1               5

tat acc cac ctt tcg gcc gat aac ttt ccc aaa gca gag cac ctt gcg      164
Tyr Thr His Leu Ser Ala Asp Asn Phe Pro Lys Ala Glu His Leu Ala
            10                  15                  20

tgg aaa ttc tcc gag ctt gcc acc gac ccc gtg gag gtg aca ccg gat      212
Trp Lys Phe Ser Glu Leu Ala Thr Asp Pro Val Glu Val Thr Pro Asp
        25                  30                  35

gtt tcg gag atg atc atc aac cgg atc atc gac aac gcg gcg gtg tct      260
Val Ser Glu Met Ile Ile Asn Arg Ile Ile Asp Asn Ala Ala Val Ser
    40                  45                  50

gcc gcg tcg gtg ttg cgc cgg cct gtg act gtg gcc agg caa caa gcg      308
Ala Ala Ser Val Leu Arg Arg Pro Val Thr Val Ala Arg Gln Gln Ala
55                  60                  65                  70

cag tcc cat ccg cgg gaa aag ggc gga aaa gtt ttt gga att tca ggc      356
Gln Ser His Pro Arg Glu Lys Gly Gly Lys Val Phe Gly Ile Ser Gly
                75                  80                  85

agc tac tca cca gag tgg gct gcc ttt gct aat ggt gtg gcc gta cgt      404
Ser Tyr Ser Pro Glu Trp Ala Ala Phe Ala Asn Gly Val Ala Val Arg
            90                  95                  100

gaa ttg gac ttc cac gat aca ttt tta gca gct gaa tac tcc cat ccc      452
Glu Leu Asp Phe His Asp Thr Phe Leu Ala Ala Glu Tyr Ser His Pro
        105                 110                 115

ggc gac aat att cca cca ctt ctt gca gta gcg cag gct cag aga agc      500
Gly Asp Asn Ile Pro Pro Leu Leu Ala Val Ala Gln Ala Gln Arg Ser
    120                 125                 130

agc ggc agg gat ctc att cgg ggt atc gcc act gcc tat gag gtg cag      548
Ser Gly Arg Asp Leu Ile Arg Gly Ile Ala Thr Ala Tyr Glu Val Gln
135                 140                 145                 150

gtg gaa ttg gtg agg ggg atc tgc ttg cat gag cac aaa att gat cac      596
Val Glu Leu Val Arg Gly Ile Cys Leu His Glu His Lys Ile Asp His
                155                 160                 165

gta gcc cac ctt ggt ccc agc gct gca gcg ggt ttg ggc acg ttg ttg      644
Val Ala His Leu Gly Pro Ser Ala Ala Ala Gly Leu Gly Thr Leu Leu
            170                 175                 180

cat gta gat gag gaa acc atc tac cag gcg atc ggc cag gca ttg cac      692
His Val Asp Glu Glu Thr Ile Tyr Gln Ala Ile Gly Gln Ala Leu His
        185                 190                 195

acc acg acg gct acg agg cag tcg cga aaa ggt gag att tcc agc tgg      740
Thr Thr Thr Ala Thr Arg Gln Ser Arg Lys Gly Glu Ile Ser Ser Trp
    200                 205                 210

aag gcg ttc gcg cca gcg ttt gcg gga aag atg gcc att gag gcg atg      788
Lys Ala Phe Ala Pro Ala Phe Ala Gly Lys Met Ala Ile Glu Ala Met
215                 220                 225                 230

gat cgt gcg atg cgt ggg gag ggt tcg ccc gca ccg att tgg gag ggc      836
Asp Arg Ala Met Arg Gly Glu Gly Ser Pro Ala Pro Ile Trp Glu Gly
                235                 240                 245

gaa gac ggg gtc atc gcg tgg ctg tta tcg ggc aaa gat cat gtt tat      884
Glu Asp Gly Val Ile Ala Trp Leu Leu Ser Gly Lys Asp His Val Tyr
            250                 255                 260

cat gtg cca ttg ccg gaa cac ggc gag ctc aag ctg ggg att cta gag      932
```

```
His Val Pro Leu Pro Glu His Gly Glu Leu Lys Leu Gly Ile Leu Glu
        265                 270                 275

act tac aca aag gaa cat tca gcg gaa tat caa tcg cag gca ccg att      980
Thr Tyr Thr Lys Glu His Ser Ala Glu Tyr Gln Ser Gln Ala Pro Ile
    280                 285                 290

gat ctg gcg cgc agg atg aag cca ctg gtt gac gcg gct ggc gga acg     1028
Asp Leu Ala Arg Arg Met Lys Pro Leu Val Asp Ala Ala Gly Gly Thr
295                 300                 305                 310

gaa cac att gca gag att gtg ctg cgc acc agt cac cac acg cat tat     1076
Glu His Ile Ala Glu Ile Val Leu Arg Thr Ser His His Thr His Tyr
                315                 320                 325

gtg att ggc act ggg gcg aac gat ccg cag aag atg gat ccg cag gcc     1124
Val Ile Gly Thr Gly Ala Asn Asp Pro Gln Lys Met Asp Pro Gln Ala
            330                 335                 340

tcg cgt gaa acc ctg gat cat tcc atc atg tac att ttc gcc gtc gcg     1172
Ser Arg Glu Thr Leu Asp His Ser Ile Met Tyr Ile Phe Ala Val Ala
        345                 350                 355

ctt caa gat ggc gtg tgg cac cac gag ttt tcc tac acc cgc aag cgt     1220
Leu Gln Asp Gly Val Trp His His Glu Phe Ser Tyr Thr Arg Lys Arg
    360                 365                 370

tcc acc cgc ccg gaa act gtg gag ctg tgg cac aag att cgc acc gtg     1268
Ser Thr Arg Pro Glu Thr Val Glu Leu Trp His Lys Ile Arg Thr Val
375                 380                 385                 390

gag gat cct gaa tgg acg cgc cga tac cat tct gat gat cct gca aaa     1316
Glu Asp Pro Glu Trp Thr Arg Arg Tyr His Ser Asp Asp Pro Ala Lys
                395                 400                 405

aag gcc ttt ggt gcg aaa gca gtg atc aca atg gct gat ggc acc gtg     1364
Lys Ala Phe Gly Ala Lys Ala Val Ile Thr Met Ala Asp Gly Thr Val
            410                 415                 420

att gag gat gaa ttg gct gtc gcg gat gcc cac ccg ctg ggt gct cgg     1412
Ile Glu Asp Glu Leu Ala Val Ala Asp Ala His Pro Leu Gly Ala Arg
        425                 430                 435

ccg ttt gcg cgg gag aat tac att gaa aaa ttc cgc aca ctc gcg cag     1460
Pro Phe Ala Arg Glu Asn Tyr Ile Glu Lys Phe Arg Thr Leu Ala Gln
    440                 445                 450

ggg att gtc att gat tca gaa cag gaa cgc ttc ttg cat gcc gtg caa     1508
Gly Ile Val Ile Asp Ser Glu Gln Glu Arg Phe Leu His Ala Val Gln
455                 460                 465                 470

agc ctg cct gac ctg gat gat ctt gat cag ctc aac atc gaa gtc gac     1556
Ser Leu Pro Asp Leu Asp Asp Leu Asp Gln Leu Asn Ile Glu Val Asp
                475                 480                 485

ata agc aac cag gcc gcg acg aaa gcg ggg ctg tta tgaatctctt           1602
Ile Ser Asn Gln Ala Ala Thr Lys Ala Gly Leu Leu
            490                 495

ttcgaatggt gttgatgtgg ggagaattcg c                                   1633
```

<210> 14
<211> 498
<212> PRT
<213> Corynebacterium glutamicum

<400> 14

```
Met Arg Ile His Asp Val Tyr Thr His Leu Ser Ala Asp Asn Phe Pro
1               5                   10                  15

Lys Ala Glu His Leu Ala Trp Lys Phe Ser Glu Leu Ala Thr Asp Pro
            20                  25                  30

Val Glu Val Thr Pro Asp Val Ser Glu Met Ile Ile Asn Arg Ile Ile
        35                  40                  45

Asp Asn Ala Ala Val Ser Ala Ala Ser Val Leu Arg Arg Pro Val Thr
    50                  55                  60

Val Ala Arg Gln Gln Ala Gln Ser His Pro Arg Glu Lys Gly Gly Lys
65                  70                  75                  80

Val Phe Gly Ile Ser Gly Ser Tyr Ser Pro Glu Trp Ala Ala Phe Ala
                85                  90                  95

Asn Gly Val Ala Val Arg Glu Leu Asp Phe His Asp Thr Phe Leu Ala
            100                 105                 110

Ala Glu Tyr Ser His Pro Gly Asp Asn Ile Pro Pro Leu Leu Ala Val
        115                 120                 125

Ala Gln Ala Gln Arg Ser Ser Gly Arg Asp Leu Ile Arg Gly Ile Ala
    130                 135                 140

Thr Ala Tyr Glu Val Gln Val Glu Leu Val Arg Gly Ile Cys Leu His
145                 150                 155                 160

Glu His Lys Ile Asp His Val Ala His Leu Gly Pro Ser Ala Ala Ala
                165                 170                 175

Gly Leu Gly Thr Leu Leu His Val Asp Glu Glu Thr Ile Tyr Gln Ala
            180                 185                 190

Ile Gly Gln Ala Leu His Thr Thr Thr Ala Thr Arg Gln Ser Arg Lys
        195                 200                 205

Gly Glu Ile Ser Ser Trp Lys Ala Phe Ala Pro Ala Phe Ala Gly Lys
    210                 215                 220

Met Ala Ile Glu Ala Met Asp Arg Ala Met Arg Gly Glu Gly Ser Pro
225                 230                 235                 240

Ala Pro Ile Trp Glu Gly Glu Asp Gly Val Ile Ala Trp Leu Leu Ser
                245                 250                 255

Gly Lys Asp His Val Tyr His Val Pro Leu Pro Glu His Gly Glu Leu
            260                 265                 270

Lys Leu Gly Ile Leu Glu Thr Tyr Thr Lys Glu His Ser Ala Glu Tyr
```

```
                275                 280                 285

        Gln Ser Gln Ala Pro Ile Asp Leu Ala Arg Arg Met Lys Pro Leu Val
            290                 295                 300

        Asp Ala Ala Gly Gly Thr Glu His Ile Ala Glu Ile Val Leu Arg Thr
        305                 310                 315                 320

        Ser His His Thr His Tyr Val Ile Gly Thr Gly Ala Asn Asp Pro Gln
                        325                 330                 335

        Lys Met Asp Pro Gln Ala Ser Arg Glu Thr Leu Asp His Ser Ile Met
                    340                 345                 350

        Tyr Ile Phe Ala Val Ala Leu Gln Asp Gly Val Trp His His Glu Phe
                355                 360                 365

        Ser Tyr Thr Arg Lys Arg Ser Thr Arg Pro Glu Thr Val Glu Leu Trp
            370                 375                 380

        His Lys Ile Arg Thr Val Glu Asp Pro Glu Trp Thr Arg Arg Tyr His
        385                 390                 395                 400

        Ser Asp Asp Pro Ala Lys Lys Ala Phe Gly Ala Lys Ala Val Ile Thr
                        405                 410                 415

        Met Ala Asp Gly Thr Val Ile Glu Asp Glu Leu Ala Val Ala Asp Ala
                    420                 425                 430

        His Pro Leu Gly Ala Arg Pro Phe Ala Arg Glu Asn Tyr Ile Glu Lys
                435                 440                 445

        Phe Arg Thr Leu Ala Gln Gly Ile Val Ile Asp Ser Glu Gln Glu Arg
            450                 455                 460

        Phe Leu His Ala Val Gln Ser Leu Pro Asp Leu Asp Asp Leu Asp Gln
        465                 470                 475                 480

        Leu Asn Ile Glu Val Asp Ile Ser Asn Gln Ala Ala Thr Lys Ala Gly
                        485                 490                 495

        Leu Leu
```

<210> 15
<211> 1311
<212> DNA
<213> Corynebacterium glutamicum

<220>
<221> CDS
<222> (1)..(1308)
<223> ilvA-Wildtyp-Gen

<400> 15

atg agt gaa aca tac gtg tct gag aaa agt cca gga gtg atg gct agc 48
Met Ser Glu Thr Tyr Val Ser Glu Lys Ser Pro Gly Val Met Ala Ser
1 5 10 15

gga gcg gag ctg att cgt gcc gcc gac att caa acg gcg cag gca cga 96
Gly Ala Glu Leu Ile Arg Ala Ala Asp Ile Gln Thr Ala Gln Ala Arg
20 25 30

att tcc tcc gtc att gca cca act cca ttg cag tat tgc cct cgt ctt 144
Ile Ser Ser Val Ile Ala Pro Thr Pro Leu Gln Tyr Cys Pro Arg Leu
35 40 45

tct gag gaa acc gga gcg gaa atc tac ctt aag cgt gag gat ctg cag 192
Ser Glu Glu Thr Gly Ala Glu Ile Tyr Leu Lys Arg Glu Asp Leu Gln
50 55 60

gat gtt cgt tcc tac aag atc cgc ggt gcg ctg aac tct gga gcg cag 240
Asp Val Arg Ser Tyr Lys Ile Arg Gly Ala Leu Asn Ser Gly Ala Gln
65 70 75 80

ctc acc caa gag cag cgc gat gca ggt atc gtt gcc gca tct gca ggt 288
Leu Thr Gln Glu Gln Arg Asp Ala Gly Ile Val Ala Ala Ser Ala Gly
85 90 95

aac cat gcc cag ggc gtg gcc tat gtg tgc aag tcc ttg ggc gtt cag 336
Asn His Ala Gln Gly Val Ala Tyr Val Cys Lys Ser Leu Gly Val Gln
100 105 110

gga cgc atc tat gtt cct gtg cag act cca aag caa aag cgt gac cgc 384
Gly Arg Ile Tyr Val Pro Val Gln Thr Pro Lys Gln Lys Arg Asp Arg
115 120 125

atc atg gtt cac ggc gga gag ttt gtc tcc ttg gtg gtc act ggc aat 432
Ile Met Val His Gly Gly Glu Phe Val Ser Leu Val Val Thr Gly Asn
130 135 140

aac ttc gac gaa gca tcg gct gca gcg cat gaa gat gca gag cgc acc 480
Asn Phe Asp Glu Ala Ser Ala Ala Ala His Glu Asp Ala Glu Arg Thr
145 150 155 160

ggc gca acg ctg atc gag cct ttc gat gct cgc aac acc gtc atc ggt 528
Gly Ala Thr Leu Ile Glu Pro Phe Asp Ala Arg Asn Thr Val Ile Gly
165 170 175

cag ggc acc gtg gct gct gag atc ttg tcg cag ctg act tcc atg ggc 576
Gln Gly Thr Val Ala Ala Glu Ile Leu Ser Gln Leu Thr Ser Met Gly
180 185 190

aag agt gca gat cac gtg atg gtt cca gtc ggc ggt ggc gga ctt ctt 624
Lys Ser Ala Asp His Val Met Val Pro Val Gly Gly Gly Gly Leu Leu
195 200 205

gca ggt gtg gtc agc tac atg gct gat atg gca cct cgc act gcg atc 672
Ala Gly Val Val Ser Tyr Met Ala Asp Met Ala Pro Arg Thr Ala Ile
210 215 220

gtt ggt atc gaa cca gcg gga gca gca tcc atg cag gct gca ttg cac 720
Val Gly Ile Glu Pro Ala Gly Ala Ala Ser Met Gln Ala Ala Leu His
225 230 235 240

aat ggt gga cca atc act ttg gag act gtt gat ccc ttt gtg gac ggc 768
Asn Gly Gly Pro Ile Thr Leu Glu Thr Val Asp Pro Phe Val Asp Gly
245 250 255

gca gca gtc aaa cgt gtc gga gat ctc aac tac acc atc gtg gag aag 816
Ala Ala Val Lys Arg Val Gly Asp Leu Asn Tyr Thr Ile Val Glu Lys
260 265 270

```
aac cag ggt cgc gtg cac atg atg agc gcg acc gag ggc gct gtg tgt      864
Asn Gln Gly Arg Val His Met Met Ser Ala Thr Glu Gly Ala Val Cys
        275                 280                 285

act gag atg ctc gat ctt tac caa aac gaa ggc atc atc gcg gag cct      912
Thr Glu Met Leu Asp Leu Tyr Gln Asn Glu Gly Ile Ile Ala Glu Pro
    290                 295                 300

gct ggc gcg ctg tct atc gct ggg ttg aag gaa atg tcc ttt gca cct      960
Ala Gly Ala Leu Ser Ile Ala Gly Leu Lys Glu Met Ser Phe Ala Pro
305                 310                 315                 320

ggt tct gtc gtg gtg tgc atc atc tct ggt ggc aac aac gat gtg ctg     1008
Gly Ser Val Val Val Cys Ile Ile Ser Gly Gly Asn Asn Asp Val Leu
                325                 330                 335

cgt tat gcg gaa atc gct gag cgc tcc ttg gtg cac cgc ggt ttg aag     1056
Arg Tyr Ala Glu Ile Ala Glu Arg Ser Leu Val His Arg Gly Leu Lys
            340                 345                 350

cac tac ttc ttg gtg aac ttc ccg caa aag cct ggt cag ttg cgt cac     1104
His Tyr Phe Leu Val Asn Phe Pro Gln Lys Pro Gly Gln Leu Arg His
        355                 360                 365

ttc ctg gaa gat atc ctg gga ccg gat gat gac atc acg ctg ttt gag     1152
Phe Leu Glu Asp Ile Leu Gly Pro Asp Asp Asp Ile Thr Leu Phe Glu
    370                 375                 380

tac ctc aag cgc aac aac cgt gag acc ggt act gcg ttg gtg ggt att     1200
Tyr Leu Lys Arg Asn Asn Arg Glu Thr Gly Thr Ala Leu Val Gly Ile
385                 390                 395                 400

cac ttg agt gaa gca tca gga ttg gat tct ttg ctg gaa cgt atg gag     1248
His Leu Ser Glu Ala Ser Gly Leu Asp Ser Leu Leu Glu Arg Met Glu
                405                 410                 415

gaa tcg gca att gat tcc cgt cgc ctc gag ccg ggc acc cct gag tac     1296
Glu Ser Ala Ile Asp Ser Arg Arg Leu Glu Pro Gly Thr Pro Glu Tyr
            420                 425                 430

gaa tac ttg acc taa                                                 1311
Glu Tyr Leu Thr
        435
```

<210> 16
<211> 436
<212> PRT
<213> Corynebacterium glutamicum

<400> 16

```
Met Ser Glu Thr Tyr Val Ser Glu Lys Ser Pro Gly Val Met Ala Ser
1               5                   10                  15

Gly Ala Glu Leu Ile Arg Ala Ala Asp Ile Gln Thr Ala Gln Ala Arg
            20                  25                  30

Ile Ser Ser Val Ile Ala Pro Thr Pro Leu Gln Tyr Cys Pro Arg Leu
        35                  40                  45

Ser Glu Glu Thr Gly Ala Glu Ile Tyr Leu Lys Arg Glu Asp Leu Gln
    50                  55                  60
```

Asp Val Arg Ser Tyr Lys Ile Arg Gly Ala Leu Asn Ser Gly Ala Gln
65                  70                  75                  80

Leu Thr Gln Glu Gln Arg Asp Ala Gly Ile Val Ala Ala Ser Ala Gly
                85                  90                  95

Asn His Ala Gln Gly Val Ala Tyr Val Cys Lys Ser Leu Gly Val Gln
            100                 105                 110

Gly Arg Ile Tyr Val Pro Val Gln Thr Pro Lys Gln Lys Arg Asp Arg
        115                 120                 125

Ile Met Val His Gly Gly Glu Phe Val Ser Leu Val Val Thr Gly Asn
    130                 135                 140

Asn Phe Asp Glu Ala Ser Ala Ala Ala His Glu Asp Ala Glu Arg Thr
145                 150                 155                 160

Gly Ala Thr Leu Ile Glu Pro Phe Asp Ala Arg Asn Thr Val Ile Gly
                165                 170                 175

Gln Gly Thr Val Ala Ala Glu Ile Leu Ser Gln Leu Thr Ser Met Gly
            180                 185                 190

Lys Ser Ala Asp His Val Met Val Pro Val Gly Gly Gly Gly Leu Leu
        195                 200                 205

Ala Gly Val Val Ser Tyr Met Ala Asp Met Ala Pro Arg Thr Ala Ile
    210                 215                 220

Val Gly Ile Glu Pro Ala Gly Ala Ala Ser Met Gln Ala Ala Leu His
225                 230                 235                 240

Asn Gly Gly Pro Ile Thr Leu Glu Thr Val Asp Pro Phe Val Asp Gly
                245                 250                 255

Ala Ala Val Lys Arg Val Gly Asp Leu Asn Tyr Thr Ile Val Glu Lys
            260                 265                 270

Asn Gln Gly Arg Val His Met Met Ser Ala Thr Glu Gly Ala Val Cys
        275                 280                 285

Thr Glu Met Leu Asp Leu Tyr Gln Asn Glu Gly Ile Ile Ala Glu Pro
    290                 295                 300

Ala Gly Ala Leu Ser Ile Ala Gly Leu Lys Glu Met Ser Phe Ala Pro
305                 310                 315                 320

Gly Ser Val Val Val Cys Ile Ile Ser Gly Gly Asn Asn Asp Val Leu
                325                 330                 335

Arg Tyr Ala Glu Ile Ala Glu Arg Ser Leu Val His Arg Gly Leu Lys

```
                340                 345                 350

His Tyr Phe Leu Val Asn Phe Pro Gln Lys Pro Gly Gln Leu Arg His
        355                 360                 365

Phe Leu Glu Asp Ile Leu Gly Pro Asp Asp Asp Ile Thr Leu Phe Glu
    370                 375                 380

Tyr Leu Lys Arg Asn Asn Arg Glu Thr Gly Thr Ala Leu Val Gly Ile
385                 390                 395                 400

His Leu Ser Glu Ala Ser Gly Leu Asp Ser Leu Leu Glu Arg Met Glu
                405                 410                 415

Glu Ser Ala Ile Asp Ser Arg Arg Leu Glu Pro Gly Thr Pro Glu Tyr
            420                 425                 430

Glu Tyr Leu Thr
        435
```

<210> 17
<211> 120
<212> DNA
<213> Corynebacterium glutamicum

<220>
<221> CDS
<222> (1)..(120)
<223> Teil der Kodierregion des prpD1-Allels

<220>
<221> misc_feature
<222> (61)..(63)
<223> n is a, c, g, or t

<400> 17

```
gcg tgg ctg tta tcg ggc aaa gat cat gtt tat cat gtg cca ttg ccg      48
Ala Trp Leu Leu Ser Gly Lys Asp His Val Tyr His Val Pro Leu Pro
1               5                   10                  15

gaa cac ggc gag nnn aag ctg ggg att cta gag act tac aca aag gaa      96
Glu His Gly Glu Xaa Lys Leu Gly Ile Leu Glu Thr Tyr Thr Lys Glu
            20                  25                  30

cat tca gcg gaa tat caa tcg cag                                      120
His Ser Ala Glu Tyr Gln Ser Gln
        35                  40
```

<210> 18
<211> 40
<212> PRT
<213> Corynebacterium glutamicum

<220>
<221> misc_feature

<222> (21)..(21)
<223> The 'Xaa' at location 21 stands for Lys, Asn, Arg, Ser, Thr, Ile, Met, Glu, Asp, Gly, Ala, Val, Gln, His, Leu, Tyr, Trp, Cys, or Phe.

<400> 18

```
Ala Trp Leu Leu Ser Gly Lys Asp His Val Tyr His Val Pro Leu Pro
1               5                   10                  15

Glu His Gly Glu Xaa Lys Leu Gly Ile Leu Glu Thr Tyr Thr Lys Glu
            20                  25                  30

His Ser Ala Glu Tyr Gln Ser Gln
        35                  40
```

<210> 19
<211> 120
<212> DNA
<213> Corynebacterium glutamicum

<220>
<221> CDS
<222> (1)..(120)
<223> Teil der Kodierregion des prpD1-Allels

<400> 19

```
gcg tgg ctg tta tcg ggc aaa gat cat gtt tat cat gtg cca ttg ccg      48
Ala Trp Leu Leu Ser Gly Lys Asp His Val Tyr His Val Pro Leu Pro
1               5                   10                  15

gaa cac ggc gag ctc aag ctg ggg att cta gag act tac aca aag gaa      96
Glu His Gly Glu Leu Lys Leu Gly Ile Leu Glu Thr Tyr Thr Lys Glu
            20                  25                  30

cat tca gcg gaa tat caa tcg cag                                      120
His Ser Ala Glu Tyr Gln Ser Gln
        35                  40
```

<210> 20
<211> 40
<212> PRT
<213> Corynebacterium glutamicum

<400> 20

```
Ala Trp Leu Leu Ser Gly Lys Asp His Val Tyr His Val Pro Leu Pro
1               5                   10                  15

Glu His Gly Glu Leu Lys Leu Gly Ile Leu Glu Thr Tyr Thr Lys Glu
            20                  25                  30

His Ser Ala Glu Tyr Gln Ser Gln
        35                  40
```